# EUROPEAN PATENT APPLICATION

(11) **EP 3 459 938 A1**
(43) Date of publication of application: **27.03.2019**
(21) Application number: 17799286.4
(22) Date of filing: 11.05.2017
(51) Int. Cl.: C07D 277/82, C07C 69/63, C07C 69/75

(54) **POLYMERIZABLE COMPOUND MANUFACTURING METHOD, HALOGENATED SUBSTANCE, AND MIXTURE**

(30) Priority: 18.05.2016 JP 2016100010
(71) Applicant: Zeon Corporation, Tokyo 100-8246 (JP)
(72) Inventor: SAKAMOTO Kei, Tokyo 100-8246 (JP); OKUYAMA Kumi, Tokyo 100-8246 (JP); SANUKI Kanako, Tokyo 100-8246 (JP)
(74) Representative: Beckmann, Claus
(86) International application number: PCT/JP2017/017944
(87) International publication number: WO 2017/199862

(57) **Abstract**

Provided is a method of producing a high-purity polymerizable compound in an industrially advantageous manner. The production method is a method of producing a polymerizable compound indicated by the following formula (I). The method includes subjecting a composition containing a halogenated compound indicated by the following formula (II) to a dehydrohalogenation reaction in an organic solvent in the presence of an aqueous layer containing a basic compound.

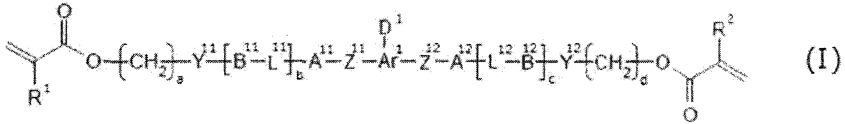

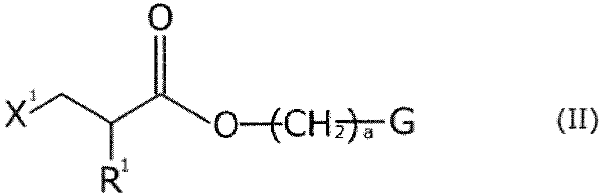

## Description

### TECHNICAL FIELD

The present disclosure relates to a method of producing a polymerizable compound that can be used in production of an optical film that enables uniform polarized light conversion over a wide wavelength region, and also to a halogenated compound and a mixture that can be used in this production method.

### BACKGROUND

Examples of retardation plates used in various devices such as flat panel displays include quarter-wave plates that convert linearly polarized light to circularly polarized light and half-wave plates that perform 90° conversion of the plane of vibration of linearly polarized light. Such retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. Specifically, since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment to polarized light with a retardation of 1/4λ or 1/2λ over the entire wavelength region of input light.

In order to solve this problem, various retardation plates having a property referred to as "reverse wavelength dispersion" have been studied. These retardation plates are wideband retardation plates that can achieve uniform retardation with respect to light over a wide wavelength region.

On the other hand, enhanced performance and widespread use of mobile information terminals such as mobile personal computers and mobile phones has been accompanied by demand for thickness-reduction of flat panel displays to as great an extent as possible. Consequently, there has also been demand for thickness-reduction of retardation plates used as components thereof.

In terms of methods of achieving thickness-reduction, a method in which a retardation plate is produced by applying a polymerizable composition containing a low-molecular weight polymerizable compound onto a film substrate to form an optical film has been regarded as the most effective method in recent years. For this reason, there has been much development of polymerizable compounds that are capable of forming optical films that excel in terms of reverse wavelength dispersion, and also polymerizable compositions in which these compounds are used.

In one example, PTL 1 proposes a polymerizable compound and a polymerizable composition that can form an optical film excelling in terms of reverse wavelength dispersion, have a low melting point suitable for processing, are easy to apply onto a substrate, have a wide temperature range over which liquid-crystallinity is displayed, and can be cheaply synthesized.

### CITATION LIST

### Patent Literature

PTL 1: WO 2014/010325 A1

### SUMMARY

### (Technical Problem)

The inventors focused on a polymerizable compound indicated by the following formula (I) ("polymerizable compound (I)") 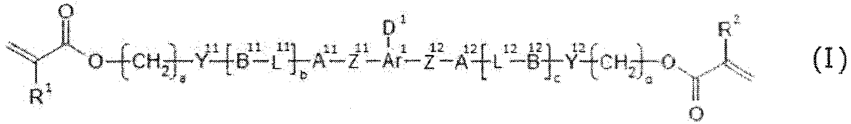 [the meaning of signs and subscript/superscript indicating chemical structure in formula (I) are described further below] as a compound that can provide an optical film having excellent performance in terms of reverse wavelength dispersion and the like. However, as a result of their investigation, the inventors discovered that there are cases in which it is difficult to produce the polymerizable compound with a sufficiently high yield by a conventional production method. For example, studies carried out by the inventors demonstrated that when the desired polymerizable compound is produced by a technique described in PTL 1, a halogenated compound of the polymerizable compound may be produced. This is presumed to be a result of the presence of an impurity in a halogen-containing compound used in synthesis of the polymerizable compound or a halogen-containing compound mixed into another raw material compound as an impurity, or under the influence of a by-product such as a salt produced in accompaniment to a reaction.

The present disclosure was completed in view of the circumstances set forth above and has an objective of providing a method of producing a high-purity polymerizable compound in an industrially advantageous manner.

Another objective of the present disclosure is to provide a halogenated compound and a mixture containing the halogenated compound that are useful in the method of producing a polymerizable compound.

### (Solution to Problem)

The inventors conducted diligent investigation in order to solve the problems set forth above. Through this investigation, the inventors conceived an idea that the yield of the aforementioned polymerizable compound (1) can be increased by subjecting a halogenated compound that is produced as a by-product to a dehydrohalogenation reaction at any stage during a process of synthesizing the polymerizable compound (I). Moreover, as a result of further investigation, the inventors discovered that by intentionally selecting a specific halogenated compound as a raw material compound for the polymerizable compound (I) and subjecting the halogenated compound to a dehydrohalogenation reaction, it is possible to produce the polymerizable compound (I) with a small mixing ratio of halogenated compound (i.e., high purity). The inventors completed the present disclosure through these investigations.

Accordingly, the present disclosure provides the following methods of producing a polymerizable compound, halogenated compounds, and mixtures.
[1] A method of producing a polymerizable compound indicated by formula (I), shown below, 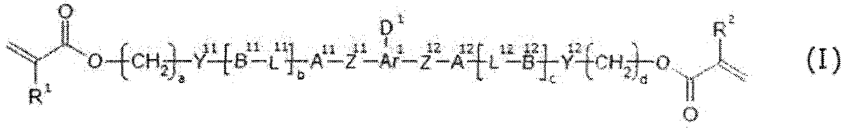 where, in formula (I):
   Ar¹ represents a divalent aromatic hydrocarbon cyclic group having D¹ as a substituent or a divalent aromatic heterocyclic group having D¹ as a substituent;
   D¹ represents an organic group having a carbon number of 1 to 20 and including at least one aromatic ring selected from the group consisting of an aromatic hydrocarbon ring and an aromatic heterocyclic ring;
   Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
   A¹¹, A¹², B¹¹, and B¹² each represent, independently of one another, an optionally substituted alicyclic group or an optionally substituted aromatic group;
   Y¹¹, Y¹², L¹¹, and L¹² each represent, independently of one another, a single bond, -O-, -CO-, -CO-O-, -O-CO-, -NR²¹-CO-, -CO-NR²²-, -O-CO-O-, -NR²³-CO-O-, -O-CO-NR²⁴-, or -NR²⁵-CO-NR²⁶-, where R²¹ to R²⁶ each represent, independently of one another, a hydrogen atom or an alkyl group having a carbon number of 1 to 6;
   R¹ and R² each represent, independently of one another, a hydrogen atom or a methyl group;
   a and d each represent, independently of one another, an integer of 1 to 20; and
   b and c are each, independently of one another, 0 or 1,
   the method comprising subjecting a composition containing a halogenated compound indicated by formula (II), shown below, 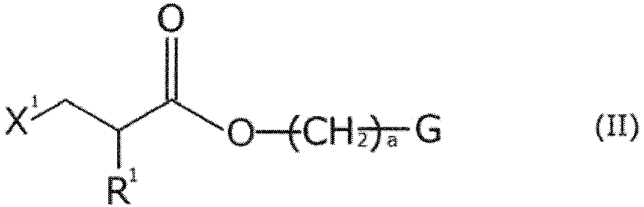
   where X¹ represents a halogen atom, G represents an organic group, and R¹ and a have the same meaning as in formula (I), to a dehydrohalogenation reaction in an organic solvent in the presence of an aqueous layer containing a basic compound.
[2] The method according to the foregoing [1], wherein
   the halogenated compound indicated by formula (II) is a halogenated compound indicated by formula (III), shown below, 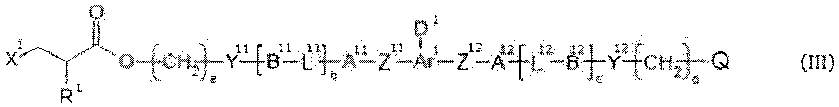 where, in formula (III):
   Q indicates a group represented by formula (III-1), shown below, 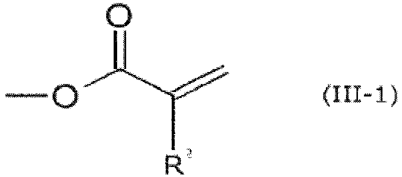 where R² has the same meaning as in formula (I), or represented by formula (III-2), shown below, 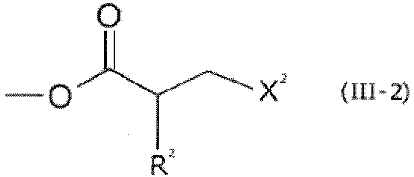 where X² represents a halogen atom and R² has the same meaning as in formula (I);
   X¹ has the same meaning as in formula (II); and
   Ar¹, D¹, Z¹¹, Z¹², A¹¹, A¹², B¹¹, B¹², Y¹¹, Y¹² , L¹¹, L¹², R¹, a, b, c, and d have the same meaning as in formula (I).
[3] The method according to the foregoing [2], wherein
   X¹ and X² are each a chlorine atom.
[4] The method according to the foregoing [1], wherein
   the halogenated compound indicated by formula (II) is a halogenated compound indicated by formula (IV), shown below, 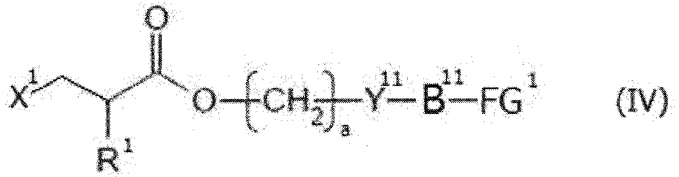 where, in formula (IV):
   FG¹ represents a hydroxy group, a carboxyl group, or an amino group;
   R¹, Y¹¹, B¹¹, and a have the same meaning as in formula (I); and
   X¹ has the same meaning as in formula (II).
[5] The method according to the foregoing [4], wherein
   X¹ is a chlorine atom.
[6] The method according to the foregoing [4] or [5], wherein
   FG¹ is a hydroxy group.
[7] The method according to any one of the foregoing [4] to [6], wherein
   the composition is a mixture containing the halogenated compound indicated by formula (IV) and a compound indicated by formula (V), shown below, 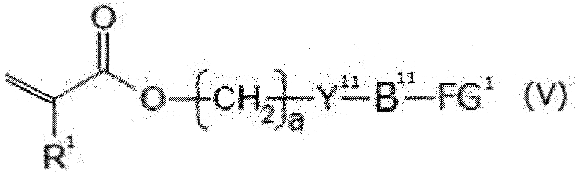 where R¹, Y¹¹, B¹¹, FG¹, and a have the same meaning as in formula (IV).
[8] The method according to the foregoing [7], wherein
   the halogenated compound indicated by formula (IV) constitutes a proportion of at least 0.01 mass% and not more than 5 mass% among a total of the halogenated compound indicated by formula (IV) and the compound indicated by formula (V).
[9] The method according to the foregoing [1], wherein
   the halogenated compound indicated by formula (II) is a halogenated compound indicated by formula (VI), shown below, 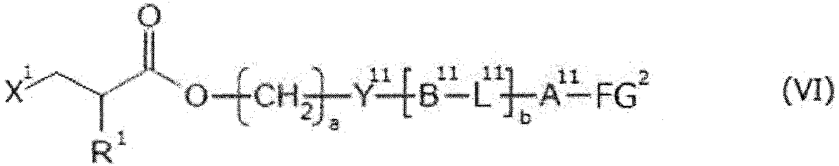 where, in formula (VI):
   FG² represents a hydroxy group, a carboxyl group, or an amino group;
   R¹, Y¹¹, B¹¹, L¹¹, A¹¹, a, and b have the same meaning as in formula (I); and
   X¹ has the same meaning as in formula (II).
[10] The method according to the foregoing [9], wherein
   X¹ is a chlorine atom.
[11] The method according to the foregoing [9] or [10], wherein
   FG² is a carboxyl group, and
   b is 1.
[12] The method according to any one of the foregoing [9] to [11], wherein
   the composition is a mixture containing the halogenated compound indicated by formula (VI) and a compound indicated by formula (VII), shown below, 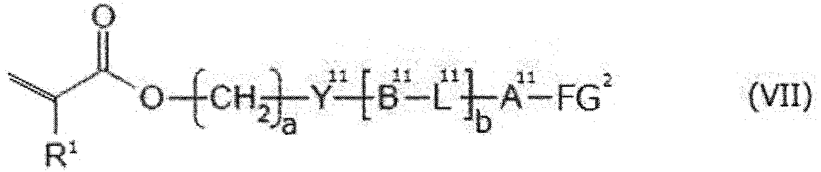 where R¹, Y¹¹, B¹¹, L¹¹, A¹¹, FG², a, and b have the same meaning as in formula (VI).
[13] The method according to the foregoing [12], wherein
   the halogenated compound indicated by formula (VI) constitutes a proportion of at least 0.01 mass% and not more than 5 mass% among a total of the halogenated compound indicated by formula (VI) and the compound indicated by formula (VII).
[14] The method according to any one of the foregoing [1] to [13], wherein
   Ar¹-D¹ is a divalent group represented by formula (VIII), shown below, 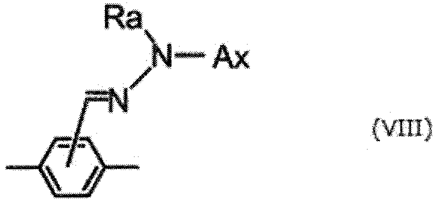 where, in formula (VIII):
   Ax represents an organic group having a carbon number of 2 to 20 and including at least one aromatic ring selected from the group consisting of an aromatic hydrocarbon ring and an aromatic heterocyclic ring; and
   Ra represents a hydrogen atom or an optionally substituted organic group having a carbon number of 1 to 20.
[15] The method according to the foregoing [14], wherein
   Ax is a group represented by formula (IX), shown below, 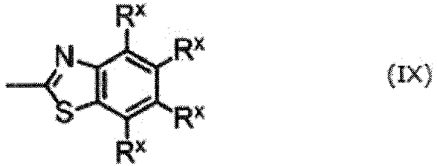 where R^{X} represents a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, or -C(=O)-O-R^{b}, where R^{b} represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 12, each R^{X} may be the same or different, and one or more ring constituent C-R^{X} may be replaced by a nitrogen atom.
[16] A halogenated compound indicated by formula (IV), shown below, 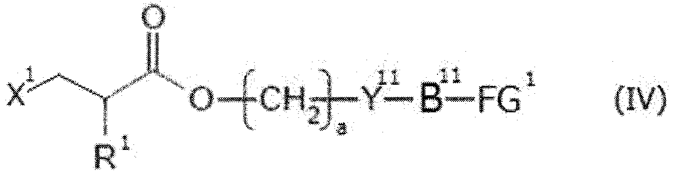 where, in formula (IV):
   X¹ represents a halogen atom;
   R¹ represents a hydrogen atom or a methyl group;
   Y¹¹ represents a single bond, -O-, -CO-, -CO-O-, -O-CO-, -NR¹¹-CO-, -CO-NR¹²-, -O-CO-O-, -NR¹³-CO-O-, -O-CO-NR¹⁴-, or -NR¹⁵-CO-NR¹⁶-, where R¹¹ to R¹⁶ each represent, independently of one another, a hydrogen atom or an alkyl group having a carbon number of 1 to 6;
   B¹¹ represents an optionally substituted alicyclic group or an optionally substituted aromatic group;
   FG¹ represents a hydroxy group, a carboxyl group, or an amino group; and
   a represents an integer of 1 to 20.
[17] The halogenated compound according to the foregoing [16], wherein
   X¹ is a chlorine atom.
[18] The halogenated compound according to the foregoing [16] or [17], wherein
   FG¹ is a hydroxy group.
[19] A mixture comprising:
   the halogenated compound according to any one of the foregoing [16] to [18]; and
   a compound indicated by formula (V), shown below, 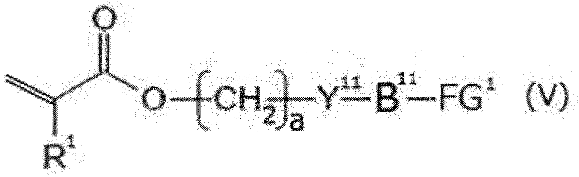
   where R¹, Y¹¹, B¹¹, FG¹, and a have the same meaning as in formula (IV).
[20] The mixture according to the foregoing [19], wherein
   the halogenated compound indicated by formula (IV) constitutes a proportion of at least 0.01 mass% and not more than 5 mass% among a total of the halogenated compound indicated by formula (IV) and the compound indicated by formula (V).
[21] A halogenated compound indicated by formula (VI), shown below, 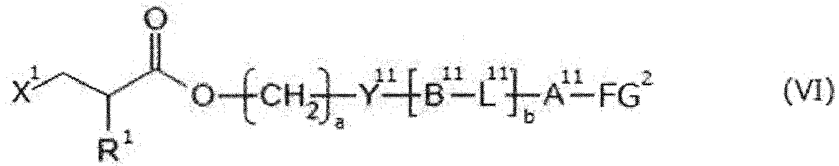 where, in formula (VI):
   X¹ represents a halogen atom;
   R¹ represents a hydrogen atom or a methyl group;
   Y¹¹ and L¹¹ each represent, independently of one another, a single bond, -O-, -CO-, -CO-O-, -O-CO-, -NR¹¹-CO-, -CO-NR¹²-. -O-CO-O-, -NR¹³-CO-O-, -O-CO-NR¹⁴-, or -NR¹⁵-CO-NR¹⁶-, where R¹¹ to R¹⁶ each represent, independently of one another, a hydrogen atom or an alkyl group having a carbon number of 1 to 6;
   A¹¹ and B¹¹ each represent, independently of one another, an optionally substituted alicyclic group or an optionally substituted aromatic group;
   FG² represents a hydroxy group, a carboxyl group, or an amino group;
   a represents an integer of 1 to 20; and
   b represents 0 or 1.
[22] The halogenated compound according to the foregoing [21], wherein
   X¹ is a chlorine atom.
[23] The halogenated compound according to the foregoing [21] or [22], wherein
   FG² is a carboxyl group, and
   b is 1.
[24] A mixture comprising:
   the halogenated compound according to any one of the foregoing [21] to [23]; and
   a compound indicated by formula (VII), shown below, 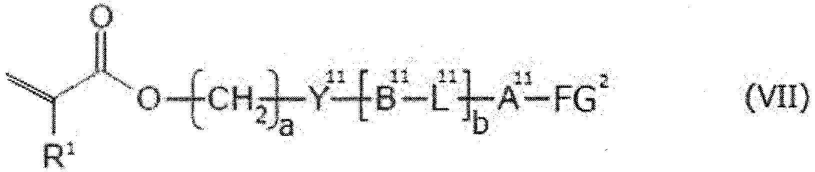 where R¹, Y¹¹, B¹¹, L¹¹, A¹¹, FG², a, and b have the same meaning as in formula (VI).
[25] The mixture according to the foregoing [24], wherein
   the halogenated compound indicated by formula (VI) constitutes a proportion of at least 0.01 mass% and not more than 5 mass% among a total of the halogenated compound indicated by formula (VI) and the compound indicated by formula (VII).
[26] A halogenated compound indicated by formula (III), shown below, 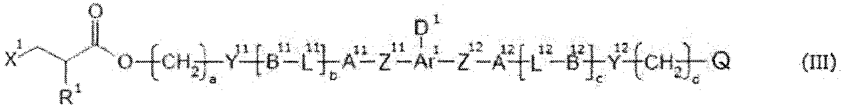 where, in formula (III):
   Q indicates a group represented by formula (III-1), shown below, 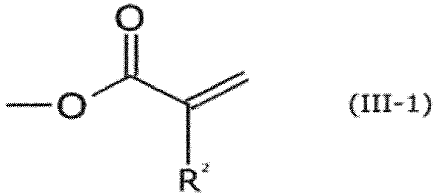 where R² represents a hydrogen atom or a methyl group, or represented by formula (III-2), shown below, 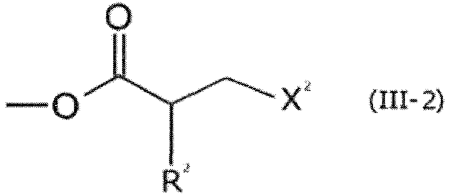 where X² represents a halogen atom and R² represents a hydrogen atom or a methyl group;
   X¹ represents a halogen atom;
   Ar¹ represents a divalent aromatic hydrocarbon cyclic group having D¹ as a substituent or a divalent aromatic heterocyclic group having D¹ as a substituent;
   D¹ represents an organic group having a carbon number of 1 to 20 and including at least one aromatic ring selected from the group consisting of an aromatic hydrocarbon ring and an aromatic heterocyclic ring;
   Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
   A¹¹, A¹², B¹¹, and B¹² each represent, independently of one another, an optionally substituted alicyclic group or an optionally substituted aromatic group;
   Y¹¹, Y¹², L¹¹, and L¹² each represent, independently of one another, a single bond, -O-, -CO-, -CO-O-, -O-CO-, -NR²¹-CO-, -CO-NR²²-, -O-CO-O-, -NR²³-CO-O-, -O-CO-NR²⁴-, or -NR²⁵-CO-NR²⁶-, where R²¹ to R²⁶ each represent, independently of one another, a hydrogen atom or an alkyl group having a carbon number of 1 to 6;
   R¹ represents a hydrogen atom or a methyl group;
   a and d each represent, independently of one another, an integer of 1 to 20; and
   b and c are each, independently of one another, 0 or 1.
[27] A mixture comprising:
   the halogenated compound according to the foregoing [26]; and
   a polymerizable compound indicated by formula (I), shown below, 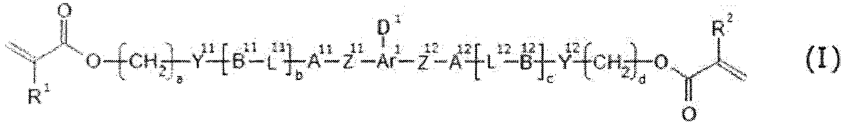 where, in formula (I):
      Ar¹ represents a divalent aromatic hydrocarbon cyclic group having D¹ as a substituent or a divalent aromatic heterocyclic group having D¹ as a substituent;
      D¹ represents an organic group having a carbon number of 1 to 20 and including at least one aromatic ring selected from the group consisting of an aromatic hydrocarbon ring and an aromatic heterocyclic ring;
      Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
      A¹¹, A¹², B¹¹, and B¹² each represent, independently of one another, an optionally substituted alicyclic group or an optionally substituted aromatic group;
      Y¹¹, Y¹², L¹¹, and L¹² each represent, independently of one another, a single bond, -O-, -CO-, -CO-O-, -O-CO-, -NR²¹-CO-, -CO-NR²²-, -O-CO-O-, -NR²¹-CO-O-, -O-CO-NR²⁴-, or -NR²⁵-CO-NR²⁶-, where R²¹ to R²⁶ each represent, independently of one another, a hydrogen atom or an alkyl group having a carbon number of 1 to 6;
      R¹ and R² each represent, independently of one another, a hydrogen atom or a methyl group;
      a and d each represent, independently of one another, an integer of 1 to 20; and
      b and c are each, independently of one another, 0 or 1.

### (Advantageous Effect)

According to the present disclosure, it is possible to provide a method of producing a high-purity polymerizable compound in an industrially advantageous manner.

Moreover, according to the present disclosure, it is possible to provide a halogenated compound and a mixture containing the halogenated compound that are useful in the method of producing a polymerizable compound.

### DETAILED DESCRIPTION

The following provides a detailed description of the present disclosure. Note that the phrase "optionally substituted" as used in the present disclosure means "unsubstituted or having one or more substituents". Also note that in a case in which an organic group, such as an alkyl group or aromatic hydrocarbon cyclic group, included in a general formula has a substituent, the carbon number of the substituted organic group is taken to be exclusive of the carbon number of the substituent. For example, in a case in which an aromatic hydrocarbon cyclic group having a carbon number of 6 to 20 has a substituent, the carbon number of the aromatic hydrocarbon cyclic group having a carbon number of 6 to 20 is taken to be exclusive of the carbon number of the substituent.

A presently disclosed method of producing a polymerizable compound is used for producing the aforementioned polymerizable compound (1). Moreover, a presently disclosed halogenated compound and a presently disclosed mixture can be used in the presently disclosed method of producing a polymerizable compound.

The presently disclosed method of producing a polymerizable compound is a method of producing the polymerizable compound (I) that includes subjecting a composition containing a halogenated compound indicated by formula (II) ("halogenated compound (II)") that is dissolved in an organic solvent to a dehydrohalogenation reaction in the presence of an aqueous layer containing at least one basic compound.

Through the presently disclosed method of producing a polymerizable compound, the halogenated compound (II) can be caused to undergo a dehydrohalogenation reaction to thereby reduce the proportion of halogenated compound among a finally obtained product and increase the yield of the polymerizable compound (I).

Consequently, the presently disclosed production method enables production of a high-purity polymerizable compound (I) in an industrially advantageous manner.

### (1) Polymerizable compound (I)

The polymerizable compound (I), which is a target product of the presently disclosed production method, is a compound that can be used in production of an optical film. By using this polymerizable compound (I), it is possible to produce an optical film that excels in terms of various properties such as reverse wavelength dispersion. The polymerizable compound (I) is a compound indicated by the following formula (I).

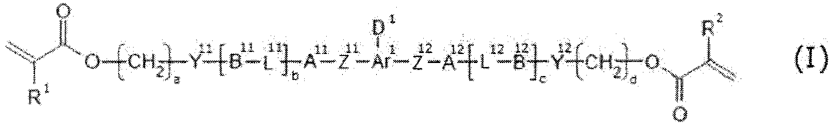

In formula (I), a and d are each, independently of one another, an integer of 1 to 20, preferably an integer of 2 to 12, and more preferably an integer of 4 to 8, and b and c are each, independently of one another, 0 or 1, and preferably 1.

Ar¹ is a divalent aromatic hydrocarbon cyclic group having D¹ as a substituent or a divalent aromatic heterocyclic group having D¹ as a substituent. D¹ is an organic group having a carbon number of 1 to 20 and including at least one aromatic ring selected from the group consisting of an aromatic hydrocarbon ring and an aromatic heterocyclic ring.

The divalent aromatic hydrocarbon cyclic group having D¹ as a substituent or the divalent aromatic heterocyclic group having D¹ as a substituent is a group resulting from removal of two hydrogen atoms from the cyclic portion of the aromatic hydrocarbon ring to which D¹ is bonded or the aromatic heterocyclic ring to which D¹ is bonded. Note that the removed hydrogen atoms are hydrogen atoms bonded to carbon other than the carbon to which D¹ is bonded.

Examples of the divalent aromatic hydrocarbon cyclic group of Ar¹ include a 1,4-phenylene group, a 1,3-phenylene group, a 1,4-naphthylene group, a 2,6-naphthylene group, a 1,5-naphthylene group, an anthracenyl-9,10-diyl group, an anthracenyl-1,4-diyl group, and an anthracenyl-2,6-diyl group.

Of these divalent aromatic hydrocarbon cyclic groups, a 1,4-phenylene group, a 1,4-naphthylene group, or a 2,6-naphthylene group is preferable.

Examples of the divalent aromatic heterocyclic group of Ar¹ include a benzothiazole-4,7-diyl group, 1,2-benzisothiazole-4,7-diyl group, a benzoxazole-4,7-diyl group, an indonyl-4,7-diyl group, a benzimidazole-4,7-diyl group, a benzopyrazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, a benzo[1,2-b:4,5-b']difuranyl-4,8-diyl group, a benzo[2,1-b:4,5-b']dipyrrole-4,8-diyl group, a benzo[1,2-b:5,4-b']dipyrrole-4,8-diyl group, and a benzo[1,2-d:4,5-d']diimidazole-4,8-diyl group.

Of these divalent aromatic heterocyclic groups, a benzothiazole-4,7-diyl group, a benzoxazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, or a benzo[1,2-b:4,5-b']difuranyl-4,8-diyl group is preferable.

The divalent aromatic hydrocarbon cyclic group or divalent aromatic heterocyclic group of Ar¹ may, besides D¹, have one or more substituents selected from alkyl groups having a carbon number of 1 to 6 such as a methyl group, an ethyl group, a propyl group, an isopropyl group, a butyl group, a sec-butyl group, and a tertiary butyl group. In a case in which the group has a plurality of substituents, these substituents may be the same or different. The divalent aromatic hydrocarbon cyclic group or divalent aromatic heterocyclic group preferably has one or more substituents selected from a methyl group, an ethyl group, a propyl group, a sec-butyl group, and a tertiary butyl group as a substituent other than D¹.

In the "organic group having a carbon number of 1 to 20 and including at least one aromatic ring selected from the group consisting of an aromatic hydrocarbon ring and an aromatic heterocyclic ring" of D¹, the "aromatic ring" is a cyclic structure that displays aromaticity in the broad sense according to Huckel's law. In other words, "aromatic ring" refers to cyclic conjugated structures including 4n + 2 π-electrons and cyclic structures that display aromaticity through the contribution of a lone pair of electrons of a heteroatom such as sulfur, oxygen, or nitrogen to the π-electron system, representative examples of which include thiophenes, furans, and benzothiazoles.

The aromatic ring included in D¹ may have one or a plurality of substituents.

The total number of π-electrons included in Ar¹ and D¹ is normally 12 or more, preferably at least 12 and not more than 22, and more preferably at least 12 and not more than 20.

Examples of the aromatic hydrocarbon ring of D¹ include a benzene ring, a naphthalene ring, an anthracene ring, a phenanthrene ring, a pyrene ring, and a fluorene ring.

Of these aromatic hydrocarbon rings, a benzene ring or a naphthalene ring is preferable.

Examples of the aromatic heterocyclic ring of D¹ include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a benzothiazole ring, a benzoxazole ring, a 1-benzofuran ring, a 2-benzofuran ring, a benzothiophene ring, a 1H-isoindole-1,3(2H)-dione ring, a thiophene ring, a furan ring, a benzo[c]thiophene ring, an oxazole ring, a thiazole ring, an oxadiazole ring, a pyran ring, a benzisoxazole ring, a thiadiazole ring, a benzoxadiazole ring, or a benzothiadiazole ring is preferable.

The organic group represented by D¹ that has a carbon number of 1 to 20 and includes at least one aromatic ring selected from the group consisting of an aromatic hydrocarbon ring and an aromatic heterocyclic ring may be, but is not specifically limited to, an optionally substituted aromatic hydrocarbon cyclic group, an optionally substituted aromatic heterocyclic group, or a group represented by a formula: -R^{f}C(=N-NR^{g}R^{h}).

In the preceding formula, R^{f} represents a hydrogen atom or an alkyl group having a carbon number of 1 to 6 such as a methyl group, an ethyl group, a propyl group, or an isopropyl group.

Moreover, R^{g} in the preceding formula represents a hydrogen atom or an optionally substituted organic group having a carbon number of 1 to 20. Examples of the organic group having a carbon number of 1 to 20 and substituents thereof include the same specific examples as listed for an organic group having a carbon number of 1 to 20 and substituents thereof described further below for Ra.

Furthermore, R^{h} in the preceding formula represents an organic group having a carbon number of 2 to 20 and including at least one aromatic ring selected from the group consisting of an aromatic hydrocarbon ring and an aromatic heterocyclic ring. Specific examples of the organic group having a carbon number of 2 to 20 and substituents thereof include the same specific examples as listed for an organic group having a carbon number of 2 to 20 and substituents thereof described further below for Ax.

Specific examples of aromatic hydrocarbon cyclic groups that may constitute D¹ include a phenyl group, a naphthyl group, an anthracenyl group, a phenanthrenyl group, a pyrenyl group, and a fluorenyl group.

Of these aromatic hydrocarbon cyclic groups, a phenyl group or a naphthyl group is preferable.

Examples of aromatic heterocyclic groups that may constitute D¹ include a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, a benzothiazolyl group, a benzothienyl group, a benzotriazinyl group, a benzotriazolyl group, a benzopyrazolyl group, a benzopyranonyl group, a dihydropyranyl group, a tetrahydropyranyl group, a dihydrofuranyl group, and a tetrahydrofuranyl group.

Of these aromatic heterocyclic groups, a furanyl group, a thienyl group, an oxazolyl group, a thiazolyl group, a benzothiazolyl group, a benzoxazolyl group, a 1-benzofuranyl group, a 2-benzofuranyl group, a benzothienyl group, or a thiazolopyridyl group is preferable.

The aromatic hydrocarbon cyclic group or aromatic heterocyclic group that may constitute D¹ may have one or more substituents selected from aliphatic hydrocarbon groups having a carbon number of 1 to 20 such as a methyl group, an ethyl group, a propyl group, an isopropyl group, a butyl group, and a sec-butyl group; halogen atoms such as a fluorine atom and a chlorine atom; a cyano group; substituted amino groups such as a dimethylamino group; alkoxy groups having a carbon number of 1 to 6 such as a methoxy group, an ethoxy group, and an isopropoxy group; a nitro group; cycloalkyl groups having a carbon number of 3 to 8 such as a cyclopentyl group and a cyclohexyl group; haloalkyl groups having a carbon number of 1 to 6 such as a trifluoromethyl group; -C(=O)-R^{b'}; -C(=O)-OR^{b'}; -SR^{b'}; -SO₂R^{d'}; a hydroxy group; and the like. R^{b'} represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 12, and R^{d'} represents an alkyl group having a carbon number of 1 to 6 such as a methyl group or an ethyl group; or an optionally substituted aromatic hydrocarbon cyclic group having a carbon number of 6 to 20 such as a phenyl group, a 4-methylphenyl group, or a 4-methoxyphenyl group. In a case in which the aromatic hydrocarbon cyclic group or aromatic heterocyclic group has a plurality of substituents, these substituents may be the same or different.

Examples of possible substituents for the optionally substituted alkyl group having a carbon number of 1 to 20, the optionally substituted alkenyl group having a carbon number of 2 to 20, or the optionally substituted aromatic hydrocarbon cyclic group having a carbon number of 5 to 12 of R^{b'} include halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; and fluoroalkyl groups having a carbon number of 1 to 12 in which one or more hydrogen atoms are substituted with fluorine atoms such as a trifluoromethyl group, a pentafluoroethyl group, and -CH₂CF₃. The alkyl group having a carbon number of 1 to 20, the alkenyl group having a carbon number of 2 to 20, or the aromatic hydrocarbon cyclic group having a carbon number of 5 to 12 of R^{b'} may have one or a plurality of substituents selected from the substituents listed above, and in a case in which the group has a plurality of substituents, these substituents may be the same or different.

Examples of possible substituents of the cycloalkyl group having a carbon number of 3 to 12 of R^{b'} include halogen atoms such as a fluorine atom and a chlorine atom; a cyano group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon groups having a carbon number of 6 to 20 such as a phenyl group and a naphthyl group. The cycloalkyl group having a carbon number of 3 to 12 of R^{b'} may have one or a plurality of substituents selected from the substituents listed above, and in a case in which the group has a plurality of substituents, these substituents may be the same or different.

Examples of combinations of Ar¹ and D¹ (Ar¹-D¹) set forth above include a phenylene group substituted with a group represented by -R^{f}C(=N-NR^{g}R^{h}), a benzothiazole-4,7-diyl group substituted with a 1-benzofuran-2-yl group, a benzothiazole-4,7-diyl group substituted with a 5-(2-butyl)-1-benzofuran-2-yl group, a benzothiazole-4,7-diyl group substituted with a 4,6-dimethyl-1-benzofuran-2-yl group, a benzothiazole-4,7-diyl group substituted with a 6-methyl-1-benzofuran-2-yl group, a benzothiazole-4,7-diyl group substituted with a 4,6,7-trimethyl-1-benzofuran-2-yl group, a benzothiazole-4,7-diyl group substituted with a 4,5,6-trimethyl-1-benzofuran-2-yl group, a benzothiazole-4,7-diyl group substituted with a 5-methyl-1-benzofuran-2-yl group, a benzothiazole-4,7-diyl group substituted with a 5-propyl-1-benzofuran-2-yl group, a benzothiazole-4,7-diyl group substituted with a 7-propyl-1-benzofuran-2-yl group, a benzothiazole-4,7-diyl group substituted with a 5-fluoro-1-benzofuran-2-yl group, a benzothiazole-4,7-diyl group substituted with a phenyl group, a benzothiazole-4,7-diyl group substituted with a 4-fluorophenyl group, a benzothiazole-4,7-diyl group substituted with a 4-nitrophenyl group, a benzothiazole-4,7-diyl group substituted with a 4-trifluoromethylphenyl group, a benzothiazole-4,7-diyl group substituted with a 4-cyanophenyl group, a benzothiazole-4,7-diyl group substituted with a 4-methanesulfonyl-phenyl group, a benzothiazole-4,7-diyl group substituted with a thiophene-2-yl group, a benzothiazole-4,7-diyl group substituted with a thiophene-3-yl group, a benzothiazole-4,7-diyl group substituted with a 5-methylthiophene-2-yl group, a benzothiazole-4,7-diyl group substituted with a 5-chlorothiophene-2-yl group, a benzothiazole-4,7-diyl group substituted with a thieno[3,2-b]thiophene-2-yl group, a benzothiazole-4,7-diyl group substituted with a 2-benzothiazolyl group, a benzothiazole-4,7-diyl group substituted with a 4-biphenyl group, a benzothiazole-4,7-diyl group substituted with a 4-propylbiphenyl group, a benzothiazole-4,7-diyl group substituted with a 4-thiazolyl group, a benzothiazole-4,7-diyl group substituted with a 1-phenylethylene-2-yl, a benzothiazole-4,7-diyl group substituted with a 4-pyridyl group, a benzothiazole-4,7-diyl group substituted with a 2-furyl group, a benzothiazole-4,7-diyl group substituted with a naphtho[1,2-b]furan-2-yl group, a 1H-isoindole-1,3(2H)-dione-4,7-diyl group substituted with a 5-methoxy-2-benzothiazolyl group, a 1H-isoindole-1,3(2H)-dione-4,7-diyl group substituted with a phenyl group, a 1H-isoindole-1,3(2H)-dione-4,7-diyl group substituted with a 4-nitrophenyl group, and a 1H-isoindole-1,3(2H)-dione-4,7-diyl group substituted with a 2-thiazolyl group. R^{f}, R^{g}, and R^{h} have the same meaning here as previously described.

Ar¹-D¹ is preferably a divalent group represented by the following formula (VIII). 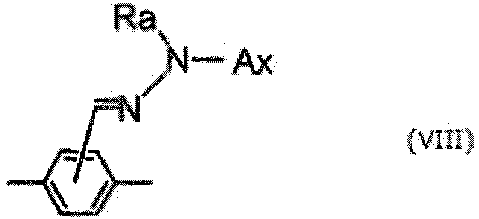 [In formula (VIII), Ax represents an organic group having a carbon number of 2 to 20 and including at least one aromatic ring selected from the group consisting of an aromatic hydrocarbon ring and an aromatic heterocyclic ring, and Ra represents a hydrogen atom or an optionally substituted organic group having a carbon number of 1 to 20.]

In the present specification, the partial structure indicated by formula (i), shown below, means the partial structure indicated by formula (ia) and/or (ib), shown below.

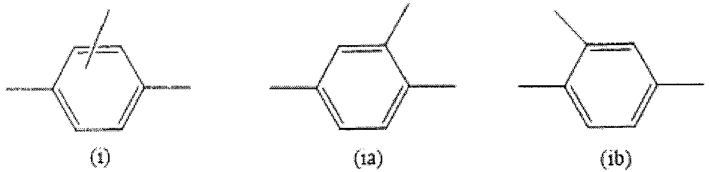

In the "organic group having a carbon number of 2 to 20 and including at least one aromatic ring selected from the group consisting of an aromatic hydrocarbon ring and an aromatic heterocyclic ring" of Ax, the "aromatic ring" is a cyclic structure that displays aromaticity in the broad sense according to Huckel's law. In other words, "aromatic ring" refers to cyclic conjugated structures including 4n + 2 π-electrons and cyclic structures that display aromaticity through the contribution of a lone pair of electrons of a heteroatom such as sulfur, oxygen, or nitrogen to the π-electron system, representative examples of which include thiophenes, furans, and benzothiazoles.

The organic group of Ax that has a carbon number of 2 to 20 and includes at least one aromatic ring selected from the group consisting of an aromatic hydrocarbon ring and an aromatic heterocyclic ring may include a plurality of aromatic rings, and may include both an aromatic hydrocarbon ring and an aromatic heterocyclic ring.

Examples of the aromatic hydrocarbon ring of Ax include a benzene ring, a naphthalene ring, an anthracene ring, a phenanthrene ring, a pyrene ring, and fluorene ring.

Of these aromatic hydrocarbon rings, a benzene ring or a naphthalene ring is preferable.

Examples of the aromatic heterocyclic ring of Ax include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, thiazolopyrazine ring, a thiazolopyridine ring, a thiazolopyridazine ring, a thiazolopyrimidine ring, a thiophene ring, a triazine ring, triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a monocyclic aromatic heterocyclic ring such as a furan ring, a thiophene ring, an oxazole ring, or a thiazole ring; or a fused ring aromatic heterocyclic ring such as a benzothiazole ring, a benzoxazole ring, a quinoline ring, a 1-benzofuran ring, a 2-benzofuran ring, a benzothiophene ring, a thiazolopyridine ring, or a thiazolopyrazine ring is preferable.

The aromatic ring included in Ax is optionally substituted. Examples of possible substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; substituted amino groups such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; -C(=O)-R^{b}; -C(=O)-OR^{b}; and -SO₂R^{d}. R^{b} represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 12. Moreover, R^{d} represents an alkyl group having a carbon number of 1 to 6 such as a methyl group or an ethyl group; or an optionally substituted aromatic hydrocarbon cyclic group having a carbon number of 6 to 20 such as a phenyl group, a 4-methylphenyl group, or a 4-methoxyphenyl group. Of these examples, halogen atoms, a cyano group, alkyl groups having a carbon number of 1 to 6, and alkoxy groups having a carbon number of 1 to 6 are preferable as substituents of the aromatic ring included in Ax.

Also note that Ax may have a plurality of substituents selected from the substituents listed above. In a case in which Ax has a plurality of substituents, these substituents may be the same or different.

Examples of the alkyl group having a carbon number of 1 to 20 in the optionally substituted alkyl group having a carbon number of 1 to 20 of R^{b} include a methyl group, an ethyl group, an n-propyl group, an isopropyl group, an n-butyl group, an isobutyl group, a 1-methylpentyl group, a 1-ethylpentyl group, a sec-butyl group, a t-butyl group, an n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group. The carbon number of the optionally substituted alkyl group having a carbon number of 1 to 20 is preferably 1 to 12, and more preferably 4 to 10.

Examples of the alkenyl group having a carbon number of 2 to 20 in the optionally substituted alkenyl group having a carbon number of 2 to 20 of R^{b} include a vinyl group, a propenyl group, an isopropenyl group, a butenyl group, an isobutenyl group, a pentenyl group, a hexenyl group, a heptenyl group, an octenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, and an icosenyl group.

The carbon number of the optionally substituted alkenyl group having a carbon number of 2 to 20 is preferably 2 to 12.

Examples of possible substituents of the alkyl group having a carbon number of 1 to 20 or alkenyl group having a carbon number of 2 to 20 of R^{b} include halogen atoms such as a fluorine atom and a chlorine atom; a cyano group; substituted amino groups such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are substituted with fluorine atoms such as a trifluoromethyl group, a pentafluoroethyl group, and -CH₂CF₃; a benzofuryl group; a benzopyranyl group; a benzodioxolyl group; and a benzodioxanyl group. Of these examples, halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; and fluoroalkyl groups having a carbon number of 1 to 12 in which one or more hydrogen atoms are substituted with fluorine atoms such as a trifluoromethyl group, a pentafluoroethyl group, and -CH₂CF₃ are preferable as substituents of the alkyl group having a carbon number of 1 to 20 or alkenyl group having a carbon number of 2 to 20 of R^{b}.

The alkyl group having a carbon number of 1 to 20 or alkenyl group having a carbon number of 2 to 20 of R^{b} may have a plurality of substituents selected from the substituents listed above. In a case in which the alkyl group having a carbon number of 1 to 20 or alkenyl group having a carbon number of 2 to 20 of R^{b} has a plurality of substituents, these substituents may be the same or different.

Examples of the cycloalkyl group having a carbon number of 3 to 12 in the optionally substituted cycloalkyl group having a carbon number of 3 to 12 of R^{b} include a cyclopropyl group, a cyclobutyl group, a cyclopentyl group, a cyclohexyl group, and a cyclooctyl group. Of these examples, a cyclopentyl group or a cyclohexyl group is preferable.

Examples of possible substituents of the cycloalkyl group having a carbon number of 3 to 12 of R^{b} include halogen atoms such as a fluorine atom and a chlorine atom; a cyano group; substituted amino groups such as a dimethylamino group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon groups having a carbon number of 6 to 20 such as a phenyl group and a naphthyl group. Of these examples, halogen atoms such as a fluorine atom and a chlorine atom; a cyano group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon groups having a carbon number of 6 to 20 such as a phenyl group and a naphthyl group are preferable as substituents of the cycloalkyl group having a carbon number of 3 to 12 of R^{b}.

The cycloalkyl group having a carbon number of 3 to 12 of R^{b} may have a plurality of substituents. In a case in which the cycloalkyl group having a carbon number of 3 to 12 of R^{b} has a plurality of substituents, these substituents may be the same or different.

Examples of the aromatic hydrocarbon cyclic group having a carbon number of 5 to 12 in the optionally substituted aromatic hydrocarbon cyclic group having a carbon number of 5 to 12 of R^{b} include a phenyl group, a 1-naphthyl group, and a 2-naphthyl group. Of these examples, a phenyl group is preferable.

Examples of possible substituents of the optionally substituted aromatic hydrocarbon cyclic group having a carbon number of 5 to 12 include halogen atoms such as a fluorine atom and a chlorine atom; a cyano group; substituted amino groups such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are substituted with fluorine atoms such as a trifluoromethyl group, a pentafluoroethyl group, and -CH₂CF₃; a benzofuryl group; a benzopyranyl group; a benzodioxolyl group; and a benzodioxanyl group. Of these examples, one or more substituents selected from halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; and fluoroalkyl groups having a carbon number of 1 to 12 in which one or more hydrogen atoms are substituted with fluorine atoms such as a trifluoromethyl group, a pentafluoroethyl group, and -CH₂CF₃ are preferable as substituents of the aromatic hydrocarbon cyclic group having a carbon number of 5 to 12.

The aromatic hydrocarbon cyclic group having a carbon number of 5 to 12 may have a plurality of substituents. In a case in which the aromatic hydrocarbon cyclic group having a carbon number of 5 to 12 has a plurality of substituents, these substituents may be the same or different.

The aromatic ring included in Ax may have a plurality of substituents that are the same or different, and two substituents that are adjacent to one another may be bonded to form a ring. The formed ring may be a monocycle or a fused polycycle, and may be an unsaturated ring or a saturated ring.

Note that the "carbon number" of the organic group having a carbon number of 2 to 20 of Ax refers to the total carbon number of the entire organic group exclusive of carbon atoms of substituents.

Examples of the organic group of Ax that has a carbon number of 2 to 20 and includes at least one aromatic ring selected from the group consisting of an aromatic hydrocarbon ring and an aromatic heterocyclic ring include aromatic hydrocarbon cyclic groups having a carbon number of 6 to 20 such as a phenyl group, a naphthyl group, an anthracenyl group, a phenanthrenyl group, a pyrenyl group, and a fluorenyl group; aromatic heterocyclic groups having a carbon number of 2 to 20 such as a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, a benzothiazolyl group, a benzothiophenyl group, a benzotriazinyl group, a benzotriazolyl group, a benzopyrazolyl group, a benzopyranonyl group, a dihydropyranyl group, a tetrahydropyranyl group, a dihydrofuranyl group, and a tetrahydrofuranyl group; hydrocarbon cyclic groups including at least one aromatic ring selected from the group consisting of an aromatic hydrocarbon ring and an aromatic heterocyclic ring; heterocyclic groups including at least one aromatic ring selected from the group consisting of an aromatic hydrocarbon ring and an aromatic heterocyclic ring; alkyl groups having a carbon number of 3 to 20 and including at least one aromatic ring selected from the group consisting of an aromatic hydrocarbon ring and an aromatic heterocyclic ring; alkenyl groups having a carbon number of 4 to 20 and including at least one aromatic ring selected from the group consisting of an aromatic hydrocarbon ring and an aromatic heterocyclic ring; and alkynyl groups having a carbon number of 4 to 20 and including at least one aromatic ring selected from the group consisting of an aromatic hydrocarbon ring and an aromatic heterocyclic ring.

For the hydrocarbon cyclic groups including at least one aromatic ring selected from the group consisting of an aromatic hydrocarbon ring and an aromatic heterocyclic ring; the heterocyclic groups including at least one aromatic ring selected from the group consisting of an aromatic hydrocarbon ring and an aromatic heterocyclic ring; the alkyl groups having a carbon number of 3 to 20 and including at least one aromatic ring selected from the group consisting of an aromatic hydrocarbon ring and an aromatic heterocyclic ring; the alkenyl groups having a carbon number of 4 to 20 and including at least one aromatic ring selected from the group consisting of an aromatic hydrocarbon ring and an aromatic heterocyclic ring; and the alkynyl groups having a carbon number of 4 to 20 and including at least one aromatic ring selected from the group consisting of an aromatic hydrocarbon ring and an aromatic heterocyclic ring, specific examples of the aromatic hydrocarbon ring and the aromatic heterocyclic ring include the same specific examples as listed for the aromatic hydrocarbon ring and aromatic heterocyclic ring of D¹.

Note that the aforementioned organic group may have one or a plurality of substituents. In a case in which the organic group has a plurality of substituents, these substituents may be the same or different.

Examples of possible substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; substituted amino groups such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; -C(=O)-R^{b}; -C(=O)-OR^{b}; and -SO₂R^{d}. R^{b} and R^{d} have the same meaning here as previously described.

Of these examples, one or more substituents selected from halogen atoms, a cyano group, alkyl groups having a carbon number of 1 to 6, and alkoxy groups having a carbon number of 1 to 6 are preferable as substituents of the organic group of Ax.

Specific examples that are preferable as the organic group of Ax that has a carbon number of 2 to 20 and includes at least one aromatic ring selected from the group consisting of an aromatic hydrocarbon ring and an aromatic heterocyclic ring are shown below. However, the following examples are not intended to be limiting. Note that "-" in the following formulae indicates a bond with a N atom that extends from any position in a ring (i.e., a N atom that is bonded to Ax in formula (VIII)).

### 1) Aromatic hydrocarbon cyclic groups

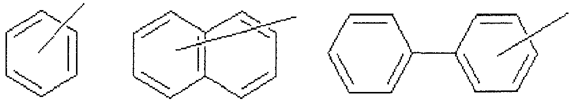

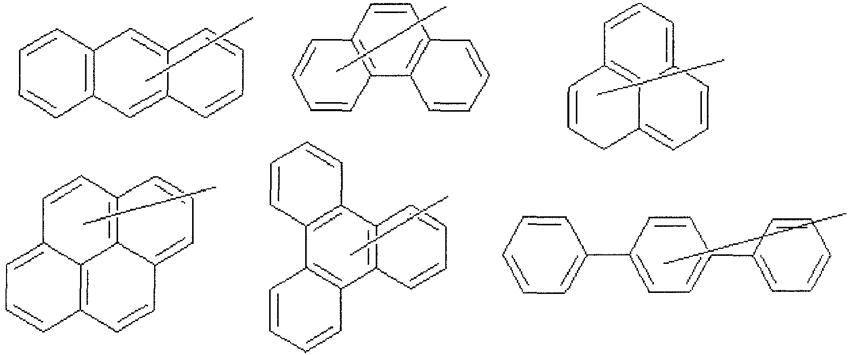

### 2) Aromatic heterocyclic groups

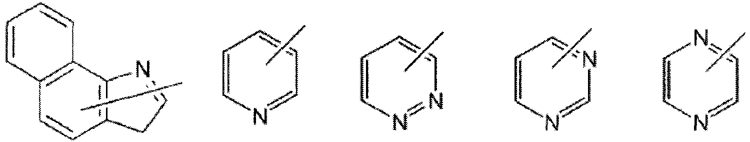

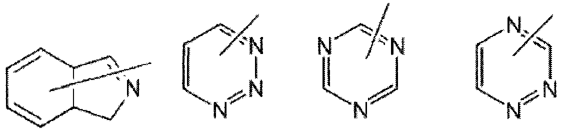

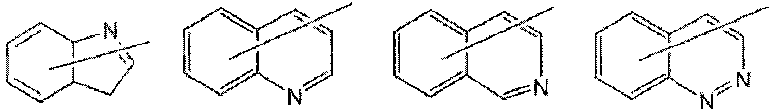

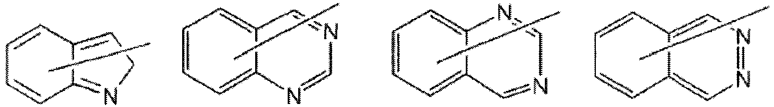

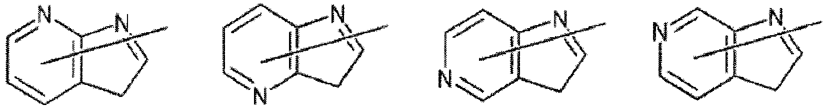

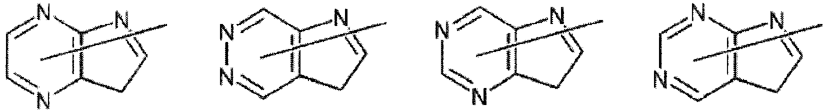

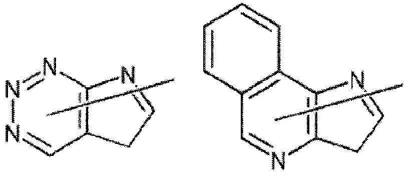

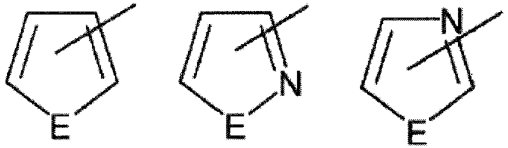

[In each formula, E represents -NR^{z}-, an oxygen atom, or a sulfur atom, where R^{z} represents a hydrogen atom or an alkyl group having a carbon number of 1 to 6 such as a methyl group, an ethyl group, or a propyl group.] 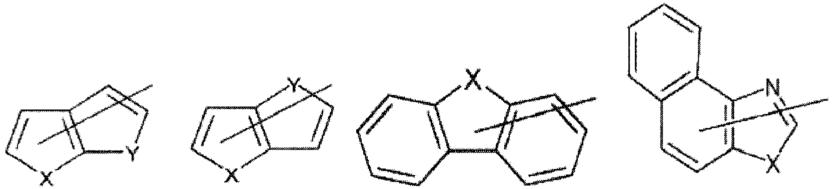 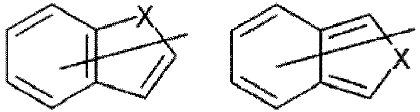 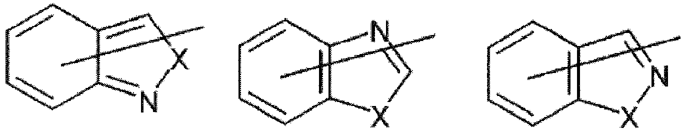 [In each formula, X and Y each represent, independently of one another, -NR^{z}-, an oxygen atom, a sulfur atom, -SO-, or -SO₂-, where R^{z} represents a hydrogen atom or an alkyl group having a carbon number of 1 to 6 such as a methyl group, an ethyl group, or a propyl group.] 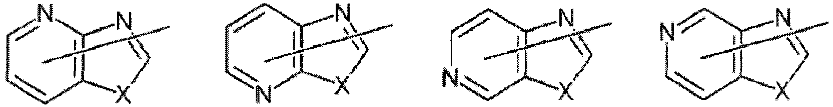 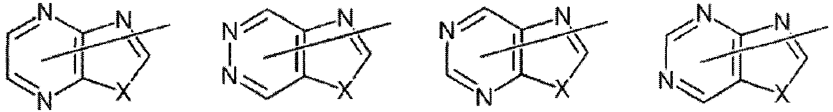 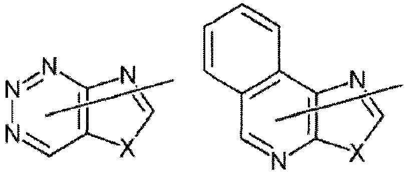 [In each formula, X represents the same as previously described.]

### 3) Hydrocarbon cyclic groups including at least one aromatic ring

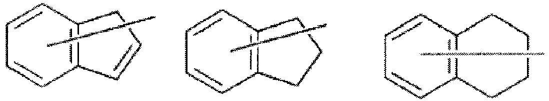

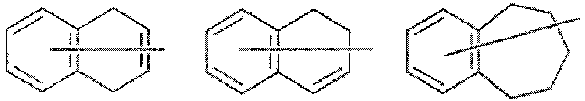

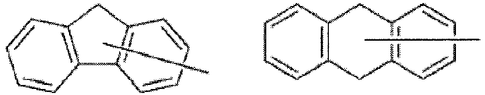

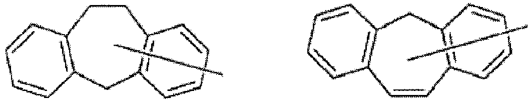

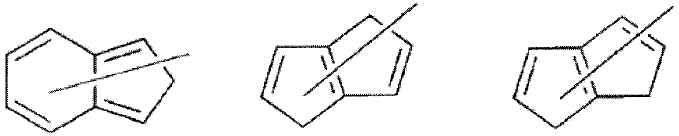

### 4) Heterocyclic groups including at least one aromatic ring

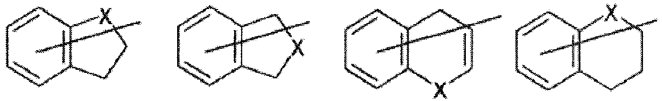

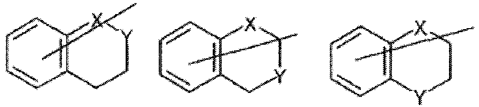

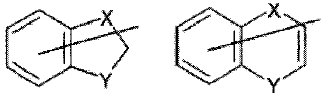

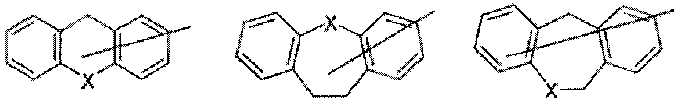

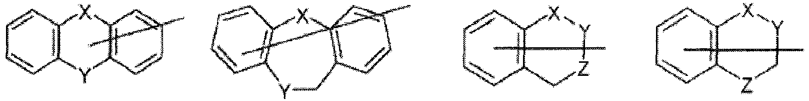

[In each formula, X and Y have the same meaning as previously described; and Z represents -NR^{z}-, an oxygen atom, or a sulfur atom, where R^{z} has the same meaning as previously described. However, cases in which oxygen atoms, sulfur atoms, -SO-, and -SO₂- are adjacent to one another are excluded.]

### 5) Alkyl groups including at least one aromatic ring selected from the group consisting of an aromatic hydrocarbon ring and an aromatic heterocyclic ring

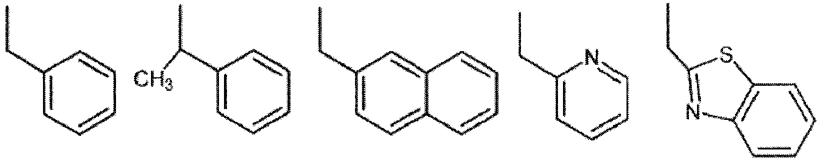

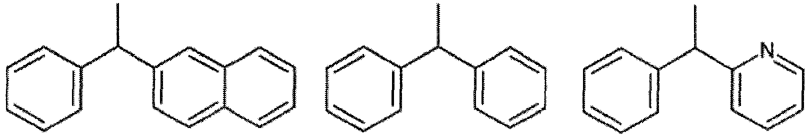

### 6) Alkenyl groups including at least one aromatic ring selected from the group consisting of an aromatic hydrocarbon ring and an aromatic heterocyclic ring

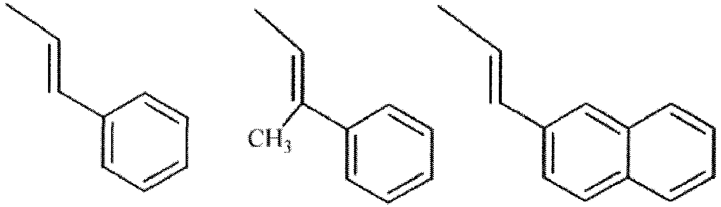

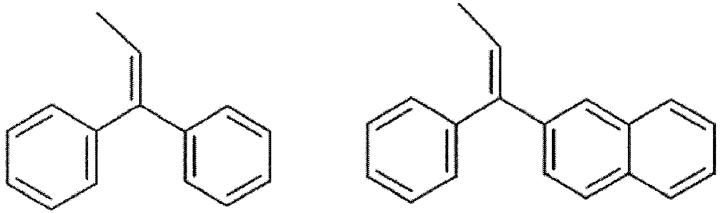

### 7) Alkynyl groups including at least one aromatic ring selected from the group consisting of an aromatic hydrocarbon ring and an aromatic heterocyclic ring

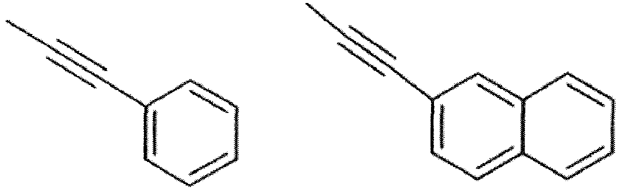

The ring(s) of the specific preferable examples of Ax shown above may have one or a plurality of substituents. In a case in which a ring has a plurality of substituents, these substituents may be the same or different. Examples of possible substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; substituted amino groups such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; -C(=O)-R^{b}; -C(=O)-OR^{b}; and -SO₂R^{d}.

R^{b} and R^{d} have the same meaning here as previously described. Of these examples, halogen atoms, a cyano group, alkyl groups having a carbon number of 1 to 6, and alkoxy groups having a carbon number of 1 to 6 are preferable as substituents of a ring included in Ax.

Specific examples of Ax that are more preferable are shown below. However, Ax is not limited to the following examples. 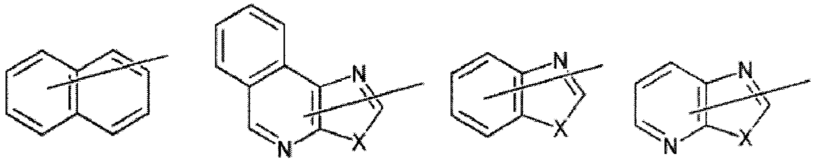 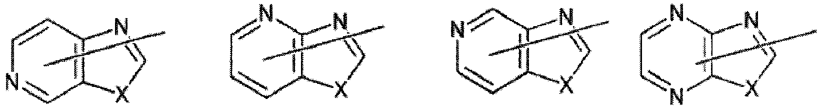 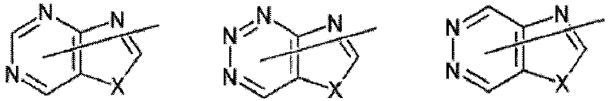 [In each formula, X has the same meaning as previously described.]

Note that the rings may have one or a plurality of substituents as previously explained. In a case in which a ring has a plurality of substituents, these substituents may be the same or different. Examples of possible substituents include halogen atoms such as a fluorine atom, a chlorine atom, and a bromine atom; alkyl groups having a carbon number of 1 to 6 such as a methyl group, an ethyl group, and a propyl group; a cyano group; alkenyl groups having a carbon number of 2 to 6 such as a vinyl group and an allyl group; haloalkyl groups having a carbon number of 1 to 6 such as a trifluoromethyl group and a pentafluoroethyl group; substituted amino groups such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; -C(=O)-R^{b}; -C(=O)-OR^{b}; and -SO₂R^{d}. R^{b} and R^{d} have the same meaning here as previously described.

Of these examples, halogen atoms, a cyano group, alkyl groups having a carbon number of 1 to 6, and alkoxy groups having a carbon number of 1 to 6 are preferable as substituents of the rings.

A group represented by the following formula (IX) is even more preferable as Ax.

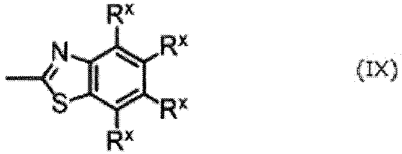

In formula (IX), R^{X} represents a hydrogen atom; a halogen atom such as a fluorine atom, a chlorine atom, or a bromine atom; an alkyl group having a carbon number of 1 to 6 such as a methyl group, an ethyl group, or a propyl group; a cyano group; a nitro group; a fluoroalkyl group having a carbon number of 1 to 6 such as a trifluoromethyl group or a pentafluoroethyl group; an alkoxy group having a carbon number of 1 to 6 such as a methoxy group, an ethoxy group, or an isopropoxy group; or -C(=O)-O-R^{b}, where R^{b} represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 12 as previously described.

Note that each R^{X} may be the same or different, and that any ring constituent C-R^{X} may be replaced by a nitrogen atom.

The following shows specific examples of groups resulting from one or more C-R^{X} in the group represented by formula (IX) being replaced by a nitrogen atom. However, examples of groups resulting from one or more C-R^{X} being replaced by a nitrogen atom are not limited to the following. 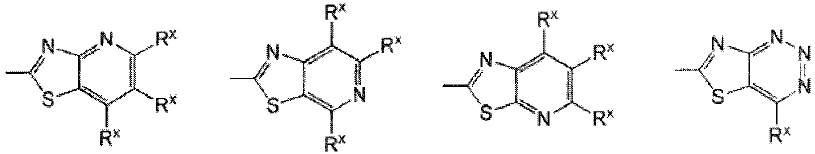 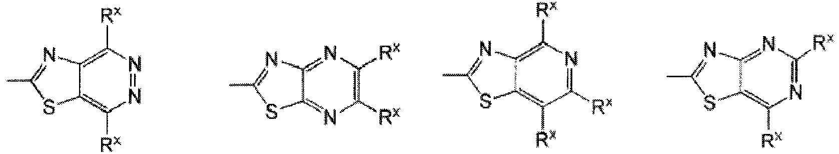 [In each formula, R^{X} has the same meaning as previously described.]

Of these examples, a group for which every R^{X} of the group represented by formula (IX) is a hydrogen atom is preferable as Ax.

Examples of the optionally substituted organic group having a carbon number of 1 to 20 of Ra in the divalent group represented by formula (VIII) include, but are not specifically limited to, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, -C(=O)-R^{b}, -SO₂-R^{d}, -C(=S)NH-Rⁱ, an optionally substituted aromatic hydrocarbon cyclic group having a carbon number of 6 to 20, and an aromatic heterocyclic group having a carbon number of 2 to 20.

R^{b} and R^{d} have the same meaning here as previously described, and Rⁱ represents an optionally substituted alkyl group having a carbon number of 1 to 20, an optionally substituted alkenyl group having a carbon number of 2 to 20, an optionally substituted cycloalkyl group having a carbon number of 3 to 12, an optionally substituted aromatic hydrocarbon cyclic group having a carbon number of 5 to 20, or an optionally substituted aromatic heterocyclic group having a carbon number of 5 to 20.

Examples of the alkyl group having a carbon number of 1 to 20 and substituents thereof in the optionally substituted alkyl group having a carbon number of 1 to 20, the alkenyl group having a carbon number of 2 to 20 and substituents thereof in the optionally substituted alkenyl group having a carbon number of 2 to 20, and the cycloalkyl group having a carbon number of 3 to 12 and substituents thereof in the optionally substituted cycloalkyl group having a carbon number of 3 to 12 include the same specific examples as listed for the alkyl group having a carbon number of 1 to 20 and substituents thereof, the alkenyl group having a carbon number of 2 to 20 and substituents thereof, and the cycloalkyl group having a carbon number of 3 to 12 and substituents thereof for R^{b}. Examples of the optionally substituted aromatic hydrocarbon cyclic group having a carbon number of 5 to 20 of Rⁱ include a phenyl group, a 1-naphthyl group, and a 2-naphthyl group. Examples of the optionally substituted aromatic heterocyclic group having a carbon number of 5 to 20 of Rⁱ include a pyridinyl group and a quinolyl group. Examples of possible substituents of the aromatic hydrocarbon cyclic group and the aromatic heterocyclic group include the same examples as listed for substituents of the organic group having a carbon number of 2 to 20 of Ax.

Examples of the alkyl group having a carbon number of 1 to 20 in the optionally substituted alkyl group having a carbon number of 1 to 20 of Ra include a methyl group, an ethyl group, an n-propyl group, an isopropyl group, an n-butyl group, an isobutyl group, a 1-methylpentyl group, a 1-ethylpentyl group, a sec-butyl group, a t-butyl group, an n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group. The carbon number of the optionally substituted alkyl group having a carbon number of 1 to 20 is preferably 1 to 12, and more preferably 1 to 10.

Examples of the alkenyl group having a carbon number of 2 to 20 in the optionally substituted alkenyl group having a carbon number of 2 to 20 of Ra include a vinyl group, a propenyl group, an isopropenyl group, a butenyl group, an isobutenyl group, a pentenyl group, a hexenyl group, a heptenyl group, an octenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, and an icosenyl group.

The carbon number of the optionally substituted alkenyl group having a carbon number of 2 to 20 is preferably 2 to 12.

Examples of the alkynyl group having a carbon number of 2 to 20 in the optionally substituted alkynyl group having a carbon number of 2 to 20 of Ra include an ethynyl group, a propynyl group, a 2-propynyl group (propargyl group), a butynyl group, a 2-butynyl group, a 3-butynyl group, a pentynyl group, a 2-pentynyl group, a hexynyl group, a 5-hexynyl group, a heptynyl group, an octynyl group, a 2-octynyl group, a nonanyl group, a decanyl group, and a 7-decanyl group.

Examples of the cycloalkyl group having a carbon number of 3 to 12 in the optionally substituted cycloalkyl group having a carbon number of 3 to 12 of Ra include a cyclopropyl group, a cyclobutyl group, a cyclopentyl group, a cyclohexyl group, and a cyclooctyl group.

Examples of possible substituents of the alkyl group having a carbon number of 1 to 20, alkenyl group having a carbon number of 2 to 20, or alkynyl group having a carbon number of 2 to 20 of Ra include halogen atoms such as a fluorine atom and a chlorine atom; a cyano group; substituted amino groups such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are substituted with fluorine atoms such as a trifluoromethyl group, a pentafluoroethyl group, and -CH₂CF₃; a benzofuryl group; a benzopyranyl group; a benzodioxolyl group; a benzodioxanyl group; -C(=O)-R^{b}; -C(=O)-OR^{b}; -SO₂R^{d}; -SR^{b}; alkoxy groups having a carbon number of 1 to 12 that are substituted with -SR^{b}; and a hydroxy group. R^{b} and R^{d} have the same meaning here as previously described.

The alkyl group having a carbon number of 1 to 20, alkenyl group having a carbon number of 2 to 20, or alkynyl group having a carbon number of 2 to 20 of Ra may have a plurality of the substituents listed above, and in such a case, these substituents may be the same or different.

Examples of possible substituents of the cycloalkyl group having a carbon number of 3 to 12 of Ra include halogen atoms such as a fluorine atom and a chlorine atom; a cyano group; substituted amino groups such as a dimethylamino group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; cycloalkyl groups having a carbon number of 3 to 8 such as a cyclopropyl group, a cyclopentyl group, and a cyclohexyl group; -C(=O)-R^{b}; -C(=O)-OR^{b}; -SO₂R^{d}; and a hydroxy group. R^{b} and R^{d} have the same meaning here as previously described.

The cycloalkyl group having a carbon number of 3 to 12 of Ra may have a plurality of the substituents listed above, and in such a case, these substituents may be the same or different.

Examples of the aromatic hydrocarbon cyclic group having a carbon number of 6 to 20, the aromatic heterocyclic group having a carbon number of 2 to 20, and substituents thereof for Ra include the same examples as listed for the aromatic hydrocarbon cyclic group, the aromatic heterocyclic group, and substituents thereof for Ax.

Of these examples, Ra is preferably a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 5 to 20, an optionally substituted aromatic hydrocarbon cyclic group having a carbon number of 6 to 18, or an optionally substituted aromatic heterocyclic group having a carbon number of 5 to 18, and is more preferably a hydrogen atom, an optionally substituted alkyl group having a carbon number of 1 to 10, an optionally substituted alkenyl group having a carbon number of 2 to 10, an optionally substituted alkynyl group having a carbon number of 2 to 10, an optionally substituted cycloalkyl group having a carbon number of 5 to 10, or an aromatic hydrocarbon cyclic group having a carbon number of 6 to 12.

Z¹¹ and Z¹² in the above-described formula (I) are each, independently of one another, -CO-O-, -O-CO-, -NR¹¹-CO-, or -CO-NR¹²-, where R¹¹ and R¹² are each, independently of one another, a hydrogen atom or an alkyl group having a carbon number of 1 to 6. In particular, Z¹¹ is preferably -CO-O-. Moreover, Z¹² is preferably -O-CO-.

A¹¹ and A¹² are each, independently of one another, an optionally substituted alicyclic group or an optionally substituted aromatic group. In particular, A¹¹ and A¹² are preferably each an optionally substituted alicyclic group.

The optionally substituted alicyclic group is an unsubstituted divalent alicyclic group or a substituted divalent alicyclic group. Moreover, the divalent alicyclic group is a divalent aliphatic group that has a cyclic structure and that normally has a carbon number of 5 to 20.

Specific examples of the divalent alicyclic group of A¹¹ and A¹² include cycloalkanediyl groups having a carbon number of 5 to 20 such as cyclopentane-1,3-diyl, cyclohexane-1,4-diyl, cycloheptane-1,4-diyl, and cyclooctane-1,5-diyl; and bicycloalkanediyl groups having a carbon number of 5 to 20 such as decahydronaphthalene-1,5-diyl and decahydronaphthalene-2,6-diyl.

The optionally substituted aromatic group is an unsubstituted divalent aromatic group or a substituted divalent aromatic group. Moreover, the divalent aromatic group is a divalent aromatic group that has an aromatic ring structure and that normally has a carbon number of 2 to 20.

Specific examples of the divalent aromatic group of A¹¹ and A¹² include divalent aromatic hydrocarbon cyclic groups having a carbon number of 6 to 20 such as a 1,4-phenylene group, a 1,4-naphthylene group, a 1,5-naphthylene group, a 2,6-naphthylene group, and a 4,4'-biphenylene group; and divalent aromatic heterocyclic groups having a carbon number of 2 to 20 such as furan-2,5-diyl, thiophene-2,5-diyl, pyridine-2,5-diyl, and pyrazine-2,5-diyl.

Examples of possible substituents of the divalent alicyclic group or divalent aromatic group of A¹¹ and A¹² include halogen atoms such as a fluorine atom, a chlorine atom, and a bromine atom; alkyl groups having a carbon number of 1 to 6 such as a methyl group and an ethyl group; alkoxy groups having a carbon number of 1 to 5 such as a methoxy group and an isopropoxy group; a nitro group; and a cyano group. The alicyclic group or aromatic group may have one or more substituents selected from the substituents listed above. In a case in which the group has a plurality of substituents, these substituents may be the same or different.

In a case in which b and/or c is 1, L¹¹ and L¹² are each, independently of one another, a single bond, -O-, -CO-, -CO-O-, -O-CO-, -NR²¹-CO-, -CO-NR²²-, -O-CO-O-, -NR²³-CO-O- , -O-CO-NR²⁴-, or -NR²⁵-CO-NR²⁶-, where R²¹ to R²⁶ are each, independently of one another, a hydrogen atom or an alkyl group having a carbon number of 1 to 6. In particular, L¹¹ and L¹² are preferably each, independently of one another, -O-, -CO-O-, or -O-CO-.

Examples of the alkyl group having a carbon number of 1 to 6 of R²¹ to R²⁶ include a methyl group, an ethyl group, a propyl group, and an isopropyl group.

Moreover, in a case in which b and/or c is 1, B¹¹ and B¹² are each, independently of one another, an optionally substituted alicyclic group or an optionally substituted aromatic group. In particular, B¹¹ and B¹² are preferably each an optionally substituted aromatic group.

The optionally substituted alicyclic group is an unsubstituted divalent alicyclic group or a substituted divalent alicyclic group. Moreover, the divalent alicyclic group is a divalent aliphatic group that has a cyclic structure and that normally has a carbon number of 5 to 20.

Specific examples of the divalent alicyclic group of B¹¹ and B¹² include the same examples as listed for the divalent alicyclic group of A¹¹ and A¹² in formula (I).

The optionally substituted aromatic group is an unsubstituted divalent aromatic group or a substituted divalent aromatic group. Moreover, the divalent aromatic group is a divalent aromatic group that has an aromatic ring structure and that normally has a carbon number of 2 to 20.

Specific examples of the divalent aromatic group of B¹¹ and B¹² include the same examples as listed for the divalent aromatic group of A¹¹ and A¹² in formula (I).

Moreover, examples of possible substituents of the divalent alicyclic group or divalent aromatic group of B¹¹ and B¹² include the same examples as listed for substituents of the divalent alicyclic group or divalent aromatic group of A¹¹ and A¹² in formula (I).

Y¹¹ and Y¹² are each, independently of one another, a single bond, -O-, -CO-, -CO-O-, -O-CO-, -NR²¹-CO-, -CO-NR²²-, -O-CO-O-, -NR²³-CO-O-, -O-CO-NR²⁴-, or -NR²⁵-CO-NR²⁶-, where R²¹ to R²⁶ are each, independently of one another, a hydrogen atom or an alkyl group having a carbon number of 1 to 6. In particular, Y¹¹ and Y¹² are preferably each, independently of one another, -O-, -CO-O-, or -O-CO-.

Examples of the alkyl group having a carbon number of 1 to 6 of R²¹ to R²⁶ include a methyl group, an ethyl group, a propyl group, and an isopropyl group.

R¹ and R² are each, independently of one another, a hydrogen atom or a methyl group. It is preferable that R¹ is the same as R² and more preferable that R¹ and R² are each a hydrogen atom.

From a viewpoint of obtaining an optical film or the like that excels in terms of reverse wavelength dispersion, it is preferable that the polymerizable compound (I) has a structure in which the left and right sides are roughly symmetrical with Ar¹-D¹ as a center. Specifically, it is preferable that R¹, a, and b are the same as R², d, and c, respectively, in the polymerizable compound (I), and that -Y¹¹-[B¹¹-L¹¹]_{b}-A¹¹-Z¹¹-(*) and (*)-Z¹²-A¹²-[L¹²-B¹²]_{c}-Y¹²- have symmetrical structures with the side (*) bonded to Ar¹ as the center of symmetry.

Note that "have symmetrical structures with ... (*) ... as the center of symmetry" refers to structures such as -CO-O-(*) and (*)-O-CO-, -O-(*) and (*)-O-, -O-CO-(*) and (*)-CO-O-, and the like.

Preferable examples of the polymerizable compound (I) for which b and c are 1 include, but are not specifically limited to, a compound indicated by the following formula (Ia). 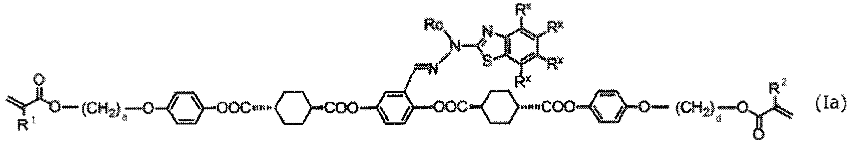 [In formula (Ia), R¹, R², Rc, R^{X}, a, and b have the same meaning as previously described, and every R^{X} is preferably a hydrogen atom].

### (2) Halogenated compound (II)

In the presently disclosed production method, the composition containing the halogenated compound (II) is subjected to a dehydrohalogenation reaction at any stage in synthesis of the polymerizable compound (I) set forth above so as to eliminate hydrogen halide from the halogenated compound (II).

The phrase "composition containing the halogenated compound (II)" as used in the present disclosure refers to the halogenated compound (II) itself or a mixture containing the halogenated compound (II) and a dehydrohalogenated product of the halogenated compound (II).

The halogenated compound (II) that is the subject of the dehydrohalogenation reaction is a compound represented by the following formula (II).

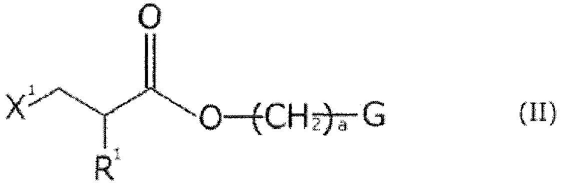

In formula (II), X¹ represents a halogen atom such as a fluorine atom, a chlorine atom, or a bromine atom, and is preferably a chlorine atom.

R¹ and a have the same meaning as in formula (I).

G is an organic group, and is preferably an organic group having a carbon number of 5 to 80 and including at least one aromatic ring. The aromatic ring may, for example, be an aromatic hydrocarbon ring or an aromatic heterocyclic ring, examples of which include the same examples as listed for the aromatic hydrocarbon ring and the aromatic heterocyclic ring of D¹ in formula (I).

The halogenated compound (II) is not specifically limited so long as it is a compound that can be used as a raw material compound for the polymerizable compound (I) and examples thereof include halogenated compounds indicated by the following formulae (III), (IV), and (VI) (referred to as "halogenated compound (III)", "halogenated compound (IV)", and "halogenated compound (VI)", respectively) that differ in terms of the structure of G.

### (2-1) Halogenated compound (III)

The halogenated compound (III) is a compound indicated by the following formula (III).

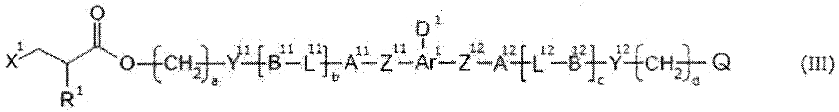

In formula (III), Q indicates a group represented by the following formula (III-1) 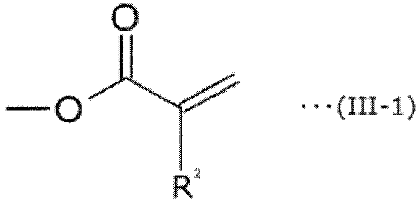 [in formula (III-1), R² represents the same as in formula (I)], or represented by the following formula (III-2) 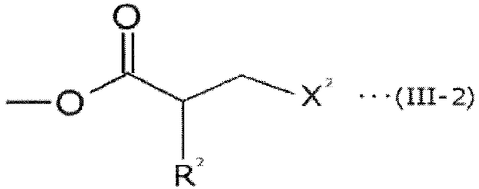 [in formula (III-2), X² represents a halogen atom such as a fluorine atom, a chlorine atom, or a bromine atom, and is preferably a chlorine atom; and R² has the same meaning as in formula (I)].

Also note that Ar¹, D¹, Z¹¹, Z¹², A¹¹, A¹², B¹¹, B¹², Y¹¹, Y¹², L¹¹, L¹², R¹, a, b, c, and d have the same meaning as in formula (I).

The halogenated compound (III) is a compound that only differs from the polymerizable compound (I) in terms of the structure of at least one end thereof. Therefore, the polymerizable compound (I) can be obtained as a dehydrohalogenated product of the halogenated compound (III) by subjecting the halogenated compound (III) to a dehydrohalogenation reaction so that a carbon-carbon double bond is formed at the end thereof.

More specific examples of the halogenated compound (III) include halogenated compounds indicated by the following formulae (IIIa), (IIIb), and (IIIc) (referred to as "halogenated compound (IIIa)", "halogenated compound (IIIb)", and "halogenated compound (IIIc)", respectively), and mixtures thereof. Note that no specific limitations are placed on the ratio in which the halogenated compound (IIIa), the halogenated compound (IIIb), and the halogenated compound (IIIc) are present in such a mixture.

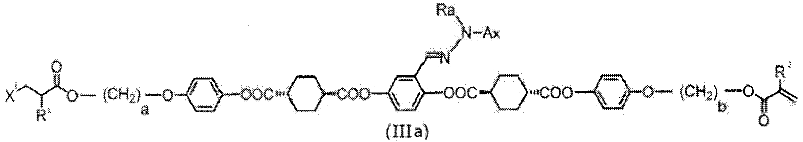

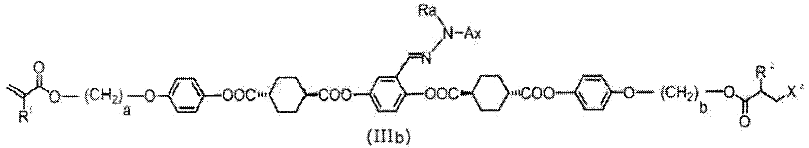

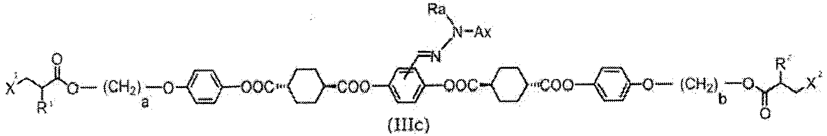

In formulae (IIIa) to (IIIc), a, b, Ra, Ax, R¹, R², X¹, and X² have the same meaning as previously described.

No specific limitations are placed on the method by which the halogenated compound (III) is obtained. For example, the halogenated compounds (IIIa) to (IIIc) can be obtained by the following production procedures 1 to 4.

### (Production procedure 1)

As previously explained, investigation carried out by the inventors has revealed that the halogenated compounds (IIIa) to (IIIc) may be produced as by-products when the desired polymerizable compound is produced by a procedure described in WO 2014/010325 A1. Specifically, at least one of the halogenated compounds (IIIa) to (IIIc) is obtained as a by-product in production of a polymerizable compound by the following procedure.

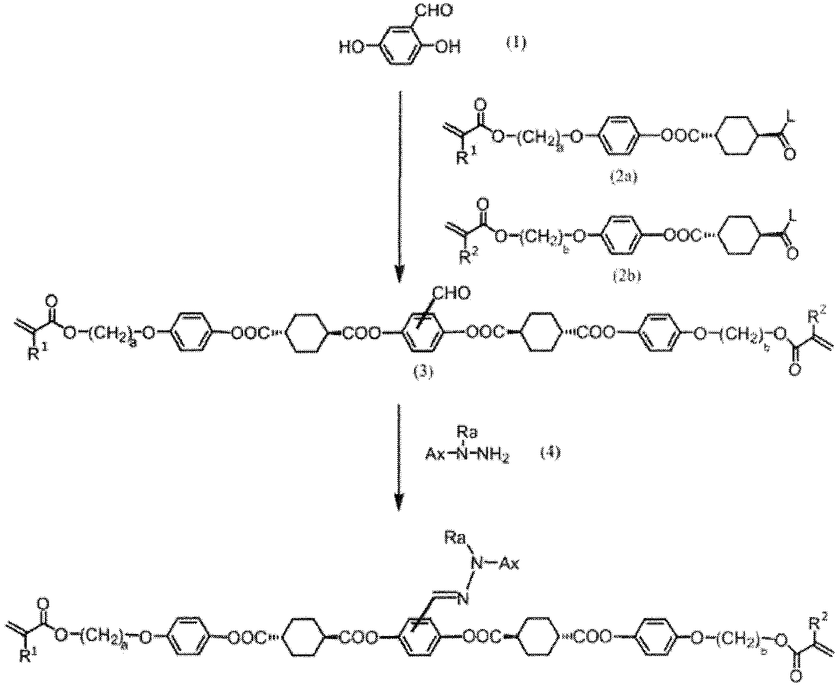

In the preceding formulae, L represents a leaving group such as a hydroxy group, a halogen atom, a methanesulfonyloxy group, or a p-toluenesulfonyloxy group. Moreover, R¹, R², Ra, Ax, a, and b have the same meaning as previously described.

Specifically, when the desired polymerizable compound is produced through a step of reacting a carboxylic acid derivative (2a) and subsequently a carboxylic acid derivative (2b) with a benzaldehyde compound (1) to obtain a compound (3) and a step of reacting the compound (3) and a hydrazine compound (4), at least one of the halogenated compounds (IIIa) to (IIIc) is produced as a by-product.

A mixture of the desired polymerizable compound and the halogenated compounds (IIIa) to (IIIc) that are produced as by-products can be used as a raw material in the presently disclosed production method without isolating the halogenated compounds (IIIa) to (IIIc). In other words, by subjecting the halogenated compounds (IIIa) to (IIIc) to a dehydrohalogenation reaction in the form of this mixture, these halogenated compounds can be efficiently converted to the polymerizable compound, and, as a result, the polymerizable compound can be obtained in a high yield.

### (Production procedure 2)

The halogenated compound (IIIa) can also be produced by the following procedure.

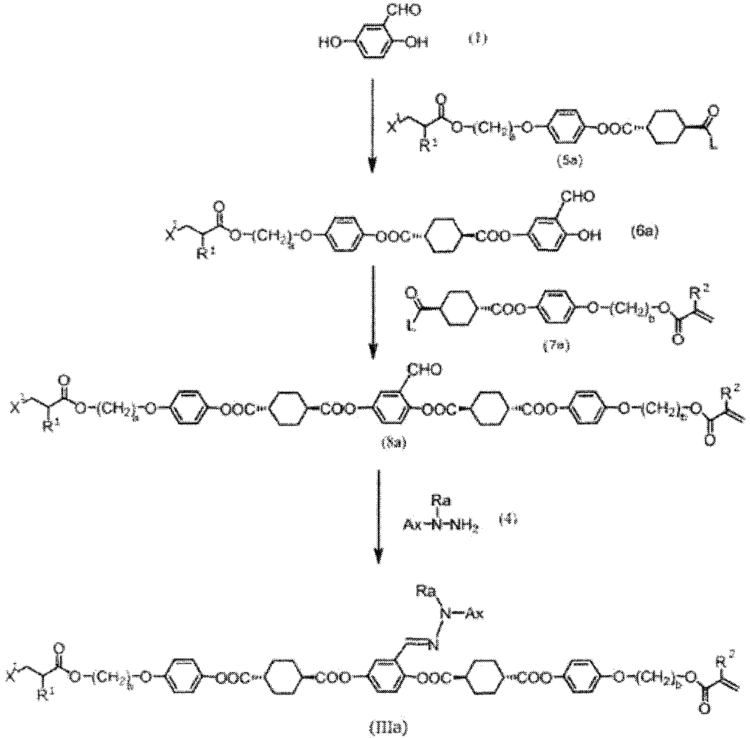

In the preceding formulae, L, X¹, R¹, R², Ra, Ax, a, and b have the same meaning as previously described.

Specifically, the halogenated compound (IIIa) can be obtained through a step of reacting a benzaldehyde compound (1) and a carboxylic acid derivative (5a) to obtain a hydroxy compound (6a), a step of reacting the hydroxy compound (6a) and a carboxylic acid derivative (7a) to obtain a compound (8a), and a step of reacting the compound (8a) and a hydrazine compound (4).

The carboxylic acid derivative (7a) and the hydrazine compound (4) may be any of those described in WO 2014/010325 A1.

The ratio in which the benzaldehyde compound (1) and the carboxylic acid derivative (5a) are used in the reaction thereof, in terms of a molar ratio "benzaldehyde compound (1):carboxylic acid derivative (5a)", is normally 1:1 to 10:1, and preferably 3:1 to 6:1.

In a situation in which the carboxylic acid derivative (5a) is a compound for which L in formula (5a) is a hydroxy group (hereinafter, this compound is referred to as "carboxylic acid compound (5')"), the hydroxy compound (6a) can be obtained by reacting the benzaldehyde compound (1) and the carboxylic acid compound (5') in the presence of a sulfonyl halide such as methanesulfonyl chloride or p-toluenesulfonyl chloride and a base such as triethylamine, diisopropylethylamine, pyridine, or 4-(dimethylamino)pyridine.

The amount of the sulfonyl halide that is used is normally 1 mol to 3 mol per 1 mol of the carboxylic acid compound (5').

The amount of the base that is used is normally 1 mol to 3 mol per 1 mol of the carboxylic acid compound (5').

Moreover, in a situation in which the carboxylic acid derivative (5a) is the carboxylic acid compound (5'), the hydroxy compound (6a) can alternatively be obtained by reacting the benzaldehyde compound (1) and the carboxylic acid compound (5') in the presence of a dehydration condensation agent such as 1-ethyl-3-(3-dimethylaminopropyl)carbodiimide hydrochloride or dicyclohexylcarbodiimide.

The amount of the dehydration condensation agent that is used is normally 1 mol to 3 mol per 1 mol of the carboxylic acid compound (5').

A carboxylic acid derivative (5a) in which L is a halogen atom can be obtained, for example, through the action of a halogenating agent such as phosphorus trichloride, phosphorus pentachloride, thionyl chloride, or oxalyl chloride on the carboxylic acid compound (5'). The hydroxy compound (6a) can then be obtained by reacting the carboxylic acid derivative (5a) in which L is a halogen atom and the benzaldehyde compound (1) in the presence of a base.

The base used in this reaction may, for example, be an organic base such as triethylamine or pyridine, or an inorganic base such as sodium hydroxide, sodium carbonate, or sodium hydrogen carbonate.

The amount of the base that is used is normally 1 mol to 3 mol per 1 mol of the carboxylic acid derivative (5a) in which L is a halogen atom.

Examples of solvents that may be used in the reaction of the benzaldehyde compound (1) and the carboxylic acid derivative (5a) described above include chlorine-containing solvents such as chloroform and methylene chloride; amide solvents such as N-methylpyrrolidone, N,N-dimethylformamide, N,N-dimethylacetamide, and hexamethylphosphoric triamide; ethers such as 1,4-dioxane, cyclopentyl methyl ether, tetrahydrofuran, tetrahydropyran, and 1,3-dioxolane; sulfur-containing solvents such as dimethyl sulfoxide and sulfolane; aromatic hydrocarbon solvents such as benzene, toluene, and xylene; aliphatic hydrocarbon solvents such as n-pentane, n-hexane, and n-octane; alicyclic hydrocarbon solvents such as cyclopentane and cyclohexane; and mixed solvents of two or more of the preceding solvents.

The amount of the solvent that is used can be set as appropriate in consideration of the types of compounds that are used, the reaction scale, and so forth, without any specific limitations, and is normally 1 g to 50 g per 1 g of the benzaldehyde compound (1).

The reaction of the hydroxy compound (6a) obtained as described above and the carboxylic acid derivative (7a) can be implemented in the same manner as the reaction of the benzaldehyde compound (1) and the carboxylic acid derivative (5a).

The ratio in which the hydroxy compound (6a) and the carboxylic acid derivative (7a) are used, in terms of a molar ratio "hydroxy compound (6a):carboxylic acid derivative (7a)", is normally 1:1 to 1:2, and preferably 1:1.1 to 1:1.5.

The reaction of the hydrazine compound (4) with the compound (8a) obtained through the reaction of the hydroxy compound (6a) and the carboxylic acid derivative (7a) can be carried out in the same manner as the reaction of the compound (3) and the hydrazine compound (4) in production procedure 1 set forth above, as described in WO 2014/010325 A1.

In this manner, the halogenated compound (IIIa) can be obtained.

Although no specific limitations are placed on the method by which the carboxylic acid compound (5') used in production procedure 2 is obtained, one example is the following method.

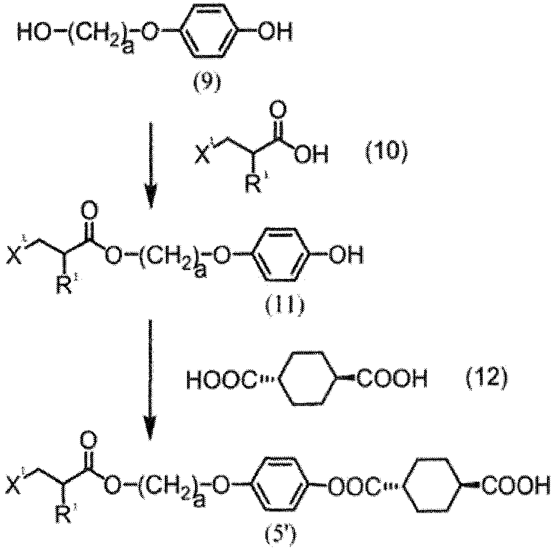

In the preceding formulae, X¹, R¹, and a have the same meaning as previously described.

Specifically, the carboxylic acid compound (5') can be obtained through a step of reacting a compound (9) and a compound (10) (3-halogeno(meth)acrylic acid) in the presence of an acid catalyst to obtain a compound (11) and a step of reacting the compound (11) and a compound (12) (trans- 1,4-cyclohexanedicarboxylic acid).

The compounds (9) and (10) can be produced by conventional and commonly known methods. A commercially available product may be used as the compound (10) without modification.

The ratio in which the compound (9) and the compound (10) are used in the reaction thereof, in terms of a molar ratio "compound (9):compound (10)", is normally 1:1 to 1:10, and preferably 1:2 to 1:4.

The acid catalyst that is used is not specifically limited and examples thereof include mineral acids such as hydrochloric acid, sulfuric acid, phosphoric acid, and nitric acid; heteropoly acids such as phosphotungstic acid; and organic acids such as methanesulfonic acid and p-toluenesulfonic acid.

Although no specific limitations are placed on the amount of the acid catalyst that is used, the amount is normally 0.01 mol to 1.0 mol, and preferably 0.05 mol to 0.4 mol per 1 mol of the compound (9).

Examples of solvents that may be used include the same solvents as listed as examples that may be used in the reaction of the benzaldehyde compound (1) and the carboxylic acid compound (5').

Although no specific limitations are placed on the amount of the solvent that is used, the amount is normally 0.2 parts by mass to 50 parts by mass, and preferably 1 part by mass to 20 parts by mass per 1 part by mass of the compound (10).

The reaction of the compound (9) and the compound (10) is preferably carried out while removing produced water from the reaction system from a viewpoint of obtaining the target in a good yield. The method by which the reaction is carried out while removing produced water from the system may, for example, be a method in which the reaction is carried out while removing water from the system using a water removal apparatus such as a Dean-Stark apparatus; a method in which the reaction is carried out while providing a dehydrating agent such as a molecular sieve in the reaction system to remove water produced in the reaction; a method in which the reaction is carried out while removing water from the system as an azeotrope with benzene or the like; or a method in which the reaction is carried out while chemically capturing water produced in the system using an orthoester, N,N-dicyclohexylcarbodiimide, or the like.

The dehydration condensation reaction of the compound (9) and the compound (10) may be carried out in the presence of a polymerization inhibitor in order to inhibit polymerization. Examples of polymerization inhibitors that may be used include 2,6-di(t-butyl)-4-methylphenol, 2,2'-methylenebis(6-t-butyl-p-cresol), triphenyl phosphite, and tris(nonylphenyl) phosphite.

Although no specific limitations are placed on the reaction temperature in the reaction of the compound (9) and the compound (10), the reaction temperature is normally 0°C to 150°C, and preferably 20°C to 120°C. The reaction time is dependent on the reaction temperature and so forth, but is normally 0.5 hours to 24 hours.

Next, the compound (11) obtained through the reaction of the compound (9) and the compound (10) is reacted with the compound (12).

The ratio in which the compound (11) and the compound (12) are used in this reaction, in terms of a molar ratio "compound (11):compound (12)", is normally 1:1 to 1:10, and preferably 1:1.5 to 1:4.

The carboxylic acid compound (5') can be obtained by reacting the compound (11) and the compound (12) in the presence of a sulfonyl halide such as methanesulfonyl chloride or p-toluenesulfonyl chloride and a base such as triethylamine, diisopropylethylamine, pyridine, or 4-(dimethylamino)pyridine.

The amount of the sulfonyl halide that is used is normally 1 mol to 1.5 mol per 1 mol of the compound (11).

The amount of the base that is used is normally 1 mol to 3 mol per 1 mol of the compound (11).

Examples of solvents that may be used include the same solvents as listed as examples that may be used in the previously described reaction of the benzaldehyde compound (1) and the carboxylic acid compound (5').

Although no specific limitations are placed on the amount of the solvent that is used, the amount is normally 0.2 parts by mass to 20 parts by mass, and preferably 1 part by mass to 10 parts by mass per 1 part by mass of the compound (11).

After the reaction has ended in any of the reactions described above, an after-treatment operation that is typically used in organic synthetic chemistry may be carried out and the target may be isolated as desired by commonly known separation/purification means such as column chromatography, recrystallization, or distillation.

The structure of the target compound can be identified through measurement of an NMR spectrum, IR spectrum, mass spectrum, or the like, elemental analysis, and so forth.

### (Production procedure 3)

The halogenated compound (IIIb) can be produced as follows.

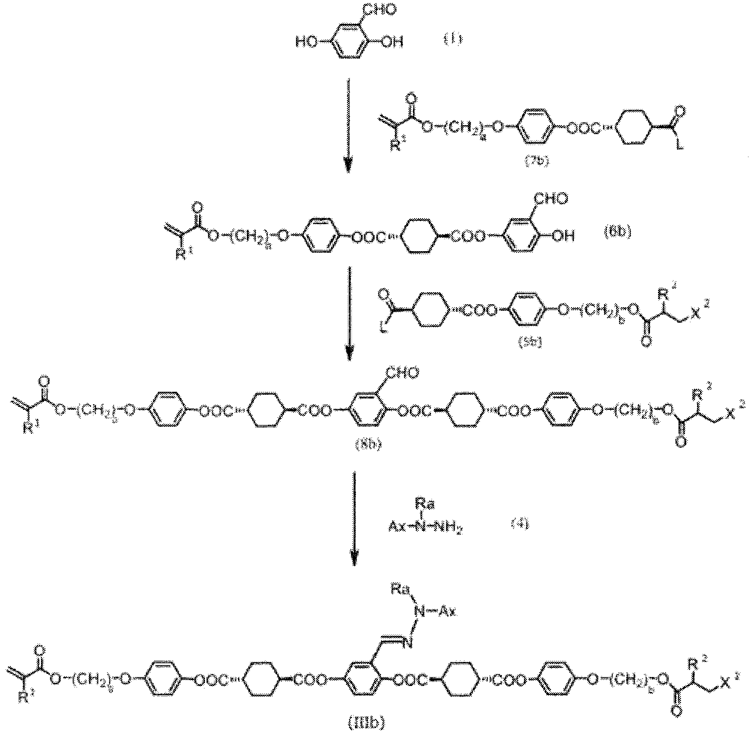

In the preceding formulae, L, X², R¹, R², Ra, Ax, a, and b have the same meaning as previously described.

Specifically, the halogenated compound (IIIb) can be obtained through a step of reacting a benzaldehyde compound (1) and a carboxylic acid derivative (7b) to obtain a compound (6b), a step of reacting the compound (6b) and a carboxylic acid derivative (5b) to obtain a compound (8b), and a step of reacting the compound (8b) and a hydrazine compound (4).

Production procedure 3 can be implemented in the same manner as production procedure 2 with the exception that the carboxylic acid derivative (7b) is used instead of the carboxylic acid derivative (5a) in production procedure 2 and the carboxylic acid derivative (5b) is used instead of the carboxylic acid derivative (7a) in production procedure 2.

### (Production procedure 4)

The halogenated compound (IIIc) can be produced by the following procedure.

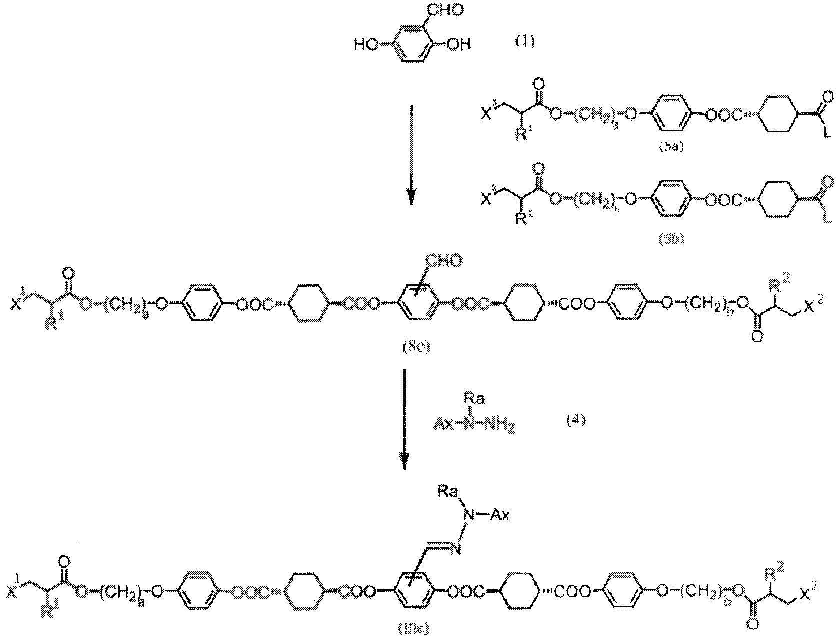

In the preceding formulae, L, X¹, X², R¹, R², Ra, Ax, a, and b have the same meaning as previously described.

Specifically, the halogenated compound (IIIc) can be obtained through a step of reacting a benzaldehyde compound (1) with a carboxylic acid derivative (5a) and a carboxylic acid derivative (5b) to obtain a compound (8c) and a step of reacting the compound (8c) and a hydrazine compound (4).

The reaction method, reaction conditions, and so forth can be set as appropriate in accordance with production procedure 2.

### (2-2) Halogenated compound (IV)

The halogenated compound (IV) is a compound indicated by the following formula (IV).

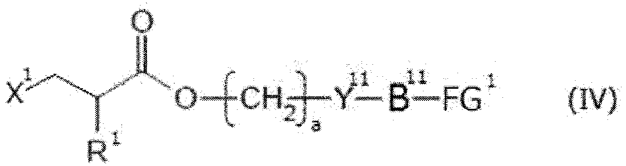

In formula (IV), FG¹ represents a hydroxy group, a carboxyl group, or an amino group, and is preferably a hydroxy group. Moreover, R¹, Y¹¹, B¹¹, and a have the same meaning as in formula (I), and X¹ has the same meaning as in formula (II).

When the halogenated compound (IV) is subjected to a dehydrohalogenation reaction, a compound indicated by the following formula (V) ("compound (V)") can be obtained as a dehydrohalogenated product of the halogenated compound (IV).

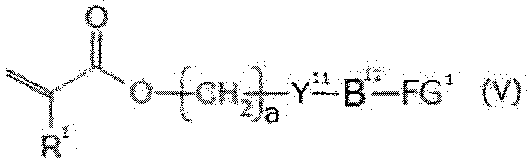

In formula (V), R¹, Y¹¹, B¹¹, FG¹, and a have the same meaning as in formula (IV).

Note that a mixture containing the halogenated compound (IV) and the compound (V) may be used as a composition containing the halogenated compound (IV) in the dehydrohalogenation reaction. By subjecting such a mixture to a dehydrohalogenation reaction, the halogenated compound (IV) in the mixture can be converted to the compound (V), and the compound (V) can be obtained in high purity. Although no specific limitations are placed on the ratio of the halogenated compound (IV) and the compound (V) in the mixture, the proportion constituted by the halogenated compound (IV) among the total of the halogenated compound (IV) and the compound (V) is preferably at least 0.01 mass% and not more than 5 mass%, more preferably at least 0.5 mass% and not more than 5 mass%, and even more preferably at least 2 mass% and not more than 5 mass%.

The resultant compound (V) can be used to obtain the desired polymerizable compound (I) through further synthesis carried out, for example, with reference to a method set forth above in "(2-1) Halogenated compound (II)".

### (2-3) Halogenated compound (VI)

The halogenated compound (VI) is a compound indicated by the following formula (VI).

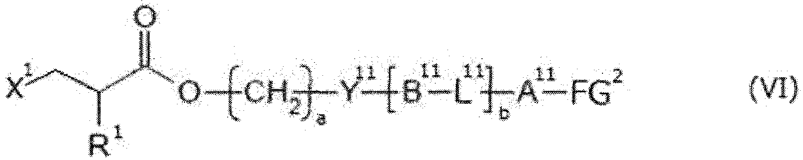

In formula (VI), FG² represents a hydroxy group, a carboxyl group, or an amino group, and is preferably a carboxyl group. Moreover, R¹, Y¹¹, B¹¹, L¹¹, A¹¹, a and b have the same meaning as in formula (I), and X¹ has the same meaning as in formula (II).

When the halogenated compound (VI) is subjected to a dehydrohalogenation reaction, a compound indicated by the following formula (VII) ("compound (VII)") can be obtained as a dehydrohalogenated product of the halogenated compound (VI).

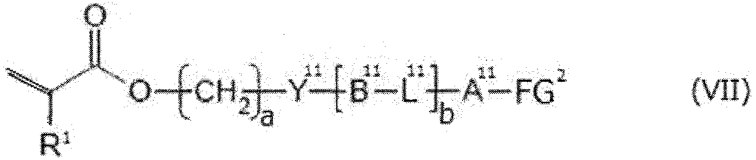

In formula (VII), R¹, Y¹¹, B¹¹, L¹¹, A¹¹, FG², a, and b have the same meaning as in formula (VI).

Note that a mixture containing the halogenated compound (VI) and the compound (VII) can be used as a composition containing the halogenated compound (VI) in the dehydrohalogenation reaction. By subjecting such a mixture to a dehydrohalogenation reaction, the halogenated compound (VI) in the mixture can be converted to the compound (VII), and the compound (VII) can be obtained in high purity. Although no specific limitations are placed on the ratio of the halogenated compound (VI) and the compound (VII) in the mixture, the proportion constituted by the halogenated compound (VI) among the total of the halogenated compound (VI) and the compound (VII) is preferably at least 0.01 mass% and not more than 5 mass%, more preferably at least 0.5 mass% and not more than 5 mass%, and even more preferably at least 1.5 mass% and not more than 5 mass%.

The resultant compound (VII) can be used to obtain the desired polymerizable compound (I) through further synthesis carried out, for example, with reference to a method set forth above in "(2-1) Halogenated compound (II)".

### (3) Dehydrohalogenation reaction

The dehydrohalogenation reaction is carried out in an organic solvent in the presence of an aqueous layer containing at least one basic compound.

### (3-1) Organic solvent

The organic solvent that is used may be any solvent without any specific limitations other than being a solvent in which the halogenated compound (II) can dissolve and that is inert in the reaction. Examples of solvents that may be used include ester solvents such as ethyl acetate, propyl acetate, and butyl acetate; ketone solvents such as cyclopentanone, methyl ethyl ketone, diethyl ketone, and methyl isobutyl ketone; halogenated hydrocarbon solvents such as dichloromethane, 1,2-dichloroethane, chloroform, carbon tetrachloride, chlorobenzene, and o-dichlorobenzene; ether solvents such as diethyl ether, diisopropyl ether, ethylene glycol dimethyl ether, cyclopentyl methyl ether, and tetrahydrofuran; aliphatic hydrocarbon solvents such as n-pentane, n-hexane, and n-heptane; aromatic hydrocarbon solvents such as benzene, toluene, and xylene; alicyclic hydrocarbon solvents such as cyclopentane and cyclohexane; and nitrogen-containing hydrocarbon solvents such as nitromethane, nitrobenzene, and acetonitrile.

One of these solvents may be used individually, or two or more of these solvents may be used in combination.

Of these solvents, a mixed solvent of an ester solvent and a nitrogen-containing hydrocarbon solvent or a mixed solvent of a ketone solvent and a nitrogen-containing hydrocarbon solvent is preferable, a mixed solvent of an ester solvent and a nitrogen-containing hydrocarbon solvent is more preferable, and a mixed solvent of ethyl acetate and acetonitrile is particularly preferable for reasons such as solubility of the halogenated compound (II) and acquisition of the target in a good yield.

In a case in which a mixed solvent of an ester solvent and a nitrogen-containing hydrocarbon solvent is used, the mixing ratio thereof, in terms of a volume ratio of the ester solvent and the nitrogen-containing hydrocarbon solvent, is normally 1:1 to 4:1, and preferably 2:1 to 3:1.

### (3-2) Basic compound-containing aqueous layer

Inorganic basic compounds and organic basic compounds can be used as the basic compound. From a viewpoint of causing the dehydrohalogenation reaction to proceed efficiently, it is preferable that at least an inorganic basic compound is used as the basic compound, and more preferable that an inorganic basic compound and an organic basic compound are used together as the basic compound.

Water that does not contain impurities such as distilled water is preferably used as water in the aqueous layer.

No specific limitations are placed on inorganic basic compounds that can be used. Examples include metal carbonates, metal hydrogen carbonates, and metal hydroxides.

Examples of metal carbonates include alkali metal carbonates such as lithium carbonate, sodium carbonate, and potassium carbonate; magnesium carbonate; and alkaline earth metal carbonates such as calcium carbonate and barium carbonate.

Examples of metal hydrogen carbonates include alkali metal hydrogen carbonates such as sodium hydrogen carbonate and potassium hydrogen carbonate; magnesium hydrogen carbonate; and alkaline earth metal hydrogen carbonates such as calcium hydrogen carbonate.

Examples of metal hydroxides include alkali metal hydroxides such as sodium hydroxide and potassium hydroxide; magnesium hydroxide; and alkaline earth metal hydroxides such as calcium hydroxide.

One of these inorganic basic compounds may be used individually, or two or more of these inorganic basic compounds may be used in combination.

Of these inorganic basic compounds, metal carbonates are preferable, alkali metal carbonates are more preferable, and sodium carbonate is even more preferable from a viewpoint of ease of acquisition and ease of handling.

Although no specific limitations are placed on the amount of the inorganic basic compound that is used, the amount is preferably 1 equivalent to 3 equivalents, and more preferably 1.5 equivalents to 2.5 equivalents per 1 equivalent of the halogenated compound (II) in order to increase the yield of dehydrohalogenated product and enable omission of a neutralization step after the reaction.

Moreover, although no specific limitations are placed on the concentration of inorganic basic compound in the aqueous layer, the concentration is preferably 0.5 mol/L to 2.5 mol/L, and more preferably 0.5 mol/L to 1.5 mol/L in order to increase the yield of dehydrohalogenated product and enable omission of a neutralization step after the reaction.

Examples of organic basic compounds that may be used include heterocyclic compounds such as pyridine, picoline, collidine, lutidine, and 4-(dimethylamino)pyridine; and tertiary amines such as triethylamine, N,N-diisopropylethylamine, and N,N-dimethylaniline.

Of these organic basic compounds, tertiary amines are preferable, and triethylamine is more preferable from a viewpoint of increasing the yield of dehydrohalogenated product.

Although no specific limitations are placed on the amount of the organic basic compound that is used, the amount is preferably 1 equivalent to 3 equivalents, and more preferably 1.2 equivalents to 2 equivalents per 1 equivalent of the halogenated compound (II).

### (3-3) Dehydrohalogenation reaction conditions

The reaction is preferably carried out in an inert atmosphere of argon, nitrogen, or the like.

The reaction temperature is normally -10°C to +80°C, preferably 10°C to 70°C, and more preferably 20°C to 60°C.

The reaction time may be a few minutes to 24 hours, and is preferably 0.5 hours to 10 hours, but is dependent on the reaction scale and so forth.

Progress of the reaction can be checked by commonly known analytical means (for example, thin-layer chromatography, high-performance liquid chromatography, or gas chromatography).

After the reaction has ended, an after-treatment operation that is typically used in organic synthetic chemistry may be carried out and the reaction product may be purified as desired by commonly known separation/purification means such as distillation, column chromatography, or recrystallization to isolate a dehydrohalogenated product (for example, the target polymerizable compound (I)).

Specifically, the target polymerizable compound (I) or the like can be efficiently isolated by removing the aqueous layer (aqueous phase) from the solution present after the reaction, washing the organic layer (organic phase) with water, and subsequently causing crystals to precipitate through addition of a poor solvent such as an alcohol solvent to the organic layer.

The structure of the target can be identified and confirmed by analytical means such as an NMR spectrum, an IR spectrum, or a mass spectrum.

### EXAMPLES

The following provides a more detailed description of the present disclosure through examples. However, the present disclosure is not in any way limited by the following examples.

### (Synthesis Example 1) Synthesis of compound 1

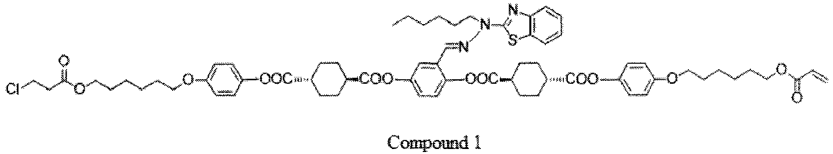

### Step 1: Synthesis of intermediate A

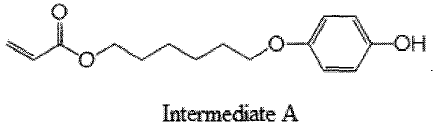

A three-necked reaction vessel equipped with a condenser and a thermometer was charged with 104.77 g (0.95 mol) of hydroquinone, 100 g (0.73 mol) of 6-chlorohexanol, 500 g of distilled water, and 100 g of o-xylene in a stream of nitrogen. The entire contents of the reaction vessel were stirred while further adding 35.15 g (0.88 mol) of sodium hydroxide gradually over 20 minutes such that the temperature of the contents did not exceed 40°C. After completion of addition of the sodium hydroxide, the contents were heated, and a reaction was carried out for 10 hours under reflux conditions (92°C).

The temperature of the reaction liquid was lowered to 80°C after the reaction ended, and 200 g of distilled water was added. Thereafter, the reaction liquid was cooled to 10°C to cause precipitation of crystals. Solid-liquid separation was carried out by filtration of the precipitated crystals. The resultant crystals were washed with 150 g of distilled water to yield 203.0 g of brown crystals. Through analysis of a portion of the brown crystals, the mass loss on drying was determined to be 36.3 mass%. Moreover, the ratio (molar ratio) of monoetherified product and dietherified product contained in the brown crystals (monoetherified product/dietherified product) was determined to be 92.0/8.0 through analysis by high-performance liquid chromatography. A three-necked reaction vessel equipped with a thermometer and a condenser including a Dean-Stark apparatus was charged with 157 g of the previously obtained brown crystals (crystals after solid-liquid separation and washing with distilled water), 500 g of toluene, and 1.05 g (4.76 mmol) of 2,6-di-t-butyl-p-cresol in a stream of nitrogen, and the entire contents of the reaction vessel were stirred to obtain a solution. The system was dehydrated by heating the resultant solution and removing water through the Dean-Stark apparatus under reflux conditions. Thereafter, the solution was cooled to 80°C, 4.57 g (47.6 mmol) of methanesulfonic acid was added thereto, and the solution was heated under reflux conditions (110°C) once again. Next, a dehydration reaction was carried out by adding 47.98 g (0.666 mol) of acrylic acid dropwise to the solution over 2 hours while removing produced water. Stirring was continued for 2 hours after the dropwise addition of acrylic acid. Next, the reaction liquid was cooled to 30°C, 500 g of distilled water was added, and the entire contents of the reaction vessel were stirred and subsequently left at rest. The organic layer was collected, 400 g of 5% saline water was added to the obtained organic layer, and liquid separation was carried out. The organic layer was collected and 10 g of activated carbon was added to the obtained organic layer. The entire contents were stirred for 30 minutes at 25°C and were subsequently filtered to remove the activated carbon. Next, 1.05 g (4.76 mmol) of 2,6-di-t-butyl-p-cresol was added to the resultant filtrate and then 350 g of toluene was evaporated under reduced pressure to concentrate the solution. Then, 300 g of n-heptane was added dropwise to the resultant concentrate over 30 minutes to cause precipitation of crystals, and cooling was performed to 5°C. The crystals were collected by filtration and the obtained crystals were washed with a mixture of 66.7 g of toluene and 133.3 g of n-heptane. Next, 144 g of toluene was added to the crystals and was heated to 40°C to dissolve the crystals. Then, 216 g of n-heptane was added dropwise to the resultant solution over 1 hour to cause precipitation of crystals, and cooling was performed to 5°C. The crystals were collected by filtration. The obtained crystals were then washed with a mixture of 72 g of toluene and 144 g of n-heptane, and were vacuum dried to yield 86.4 g (6-chlorohexanol basis yield: 58%) of intermediate A (4-(6-acryloyloxy-hex-1-yloxy)phenol) in the form of a white solid. The obtained white solid was purified by silica gel column chromatography (toluene:ethyl acetate = 95:5) to increase the purity to at least 99.5%.

The structure of the target was identified by ¹H-NMR.

¹H-NMR (500 MHz, DMSO-d₆, TMS, δ ppm): 8.87 (s, 1H), 6.72 (d, 2H, J = 9.0 Hz), 6.65 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.83 (t, 2H, J = 6.5 Hz), 1.56-1.72 (m, 4H), 1.31-1.47 (m, 4H)

### Step 2: Synthesis of intermediate B

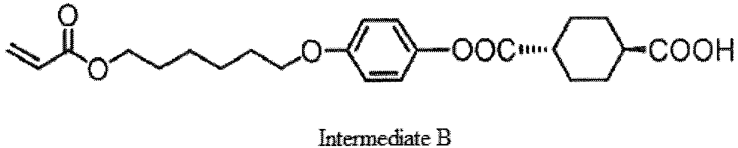

A three-necked reaction vessel equipped with a thermometer was charged with 17.98 g (104.42 mmol) of trans-1,4-cyclohexanedicarboxylic acid and 180 mL of tetrahydrofuran (THF) in a stream of nitrogen. In addition, 6.58 g (57.43 mmol) of methanesulfonyl chloride was added into the reaction vessel and the reaction vessel was immersed in a water bath to attain a reaction liquid internal temperature of 20°C. Next, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C.

Next, 638 mg (5.22 mmol) of 4-(dimethylamino)pyridine and 13.80 g (52.21 mmol) of the intermediate A synthesized in the preceding step 1 were added to the resultant reaction liquid, and the reaction vessel was immersed in a water bath once again to attain a reaction liquid internal temperature of 15°C. Thereafter, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C. Once the reaction ended, 1,000 mL of distilled water and 100 mL of saturated saline water were added, and extraction was performed twice with 400 mL of ethyl acetate. The organic layers were collected and were dried with anhydrous sodium sulfate, and then sodium sulfate was filtered off. Solvent was evaporated from the filtrate under reduced pressure using a rotary evaporator and then the obtained residue was purified by silica gel column chromatography (toluene:THF = 9:1 (volume ratio; same applies below)) to yield 14.11 g (yield: 65.0%) of intermediate B in the form of a white solid.

The structure of the target was identified by ¹H-NMR.

¹H-NMR (500 MHz, DMSO-d₆, TMS, δ ppm): 12.12 (s, 1H), 6.99 (d, 2H, J = 9.0 Hz), 6.92 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.94 (t, 2H, J = 6.5 Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H)

### Step 3: Synthesis of intermediate C

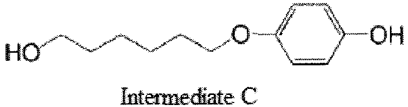

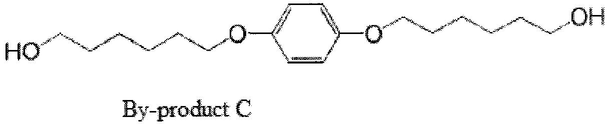

A three-necked reaction vessel equipped with a condenser and a thermometer was charged with 104.77 g (0.9515 mol) of hydroquinone, 100 g (0.7320 mol) of 6-chlorohexanol, 500 mL of distilled water, and 100 mL of o-xylene in a stream of nitrogen. The entire contents of the reaction vessel were stirred while gradually adding 35.15 g (0.8784 mol) of sodium hydroxide over 20 minutes such that the reaction liquid internal temperature did not exceed 40°C. After completion of addition of the sodium hydroxide, the contents were heated and a reaction was carried out for 12 hours under reflux conditions (96°C).

The reaction liquid internal temperature was lowered to 80°C after the reaction ended, and 200 mL of distilled water was added. Thereafter, the reaction liquid was cooled to 10°C to cause precipitation of crystals. Solid-liquid separation was carried out by filtration of the precipitated crystals. The resultant crystals were washed with 500 mL of distilled water and were vacuum dried to yield 123.3 g of brown crystals.

The content ratio (molar ratio) of compounds contained in the brown crystals (hydroquinone/intermediate C/by-product C) was determined to be 1.3/90.1/8.1 as a result of analysis of the brown crystals by high-performance liquid chromatography.

### Step 4: Synthesis of intermediate D

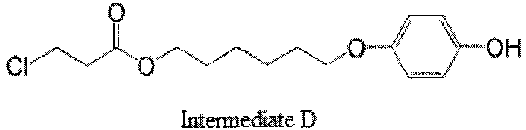

A three-necked reaction vessel equipped with a thermometer and a condenser including a Dean-Stark apparatus was charged with 15.3 g of the brown crystals synthesized in step 3, 70 mL of toluene, and 202 mg (0.921 mmol) of 2,6-di-t-butyl-4-methylphenol in a stream of nitrogen, and the entire contents of the reaction vessel were stirred. The entire contents were then heated to 80°C, 10.0 g (92.15 mmol) of 3-chloropropionic acid and 885 mg (9.21 mmol) of methanesulfonic acid were added, and a dehydration reaction was carried out for 2 hours under reflux conditions (110°C) while removing produced water. The reaction liquid internal temperature was lowered to 30°C after the reaction ended, 70 mL of distilled water was added, and the entire contents of the reaction vessel were stirred and subsequently left at rest. The organic layer was collected, 35 mL of distilled water was added to the obtained organic layer, and liquid separation was carried out. The organic layer was collected and 1.4 g of activated carbon was added to the obtained organic layer. The entire contents were stirred for 30 minutes at 25°C and were subsequently filtered to remove the activated carbon.

Next, 202 mg (0.921 mmol) of 2,6-di-t-butyl-4-methylphenol was added to the resultant filtrate, and solvent was evaporated from the filtrate under reduced pressure using a rotary evaporator. The obtained residue was purified by silica gel column chromatography (toluene:THF = 95:5) to yield 11.0 g (total yield of steps 2 and 3: 40.0%) of intermediate D in the form of a white solid.

The structure of the target was identified by ¹H-NMR.

¹H-NMR (500 MHz, CDCl₃, TMS, δ ppm): 6.78 (d, 2H, J = 9.0 Hz), 6.76 (d, 2H, J = 9.0 Hz), 4.91 (s, 1H), 4.14 (t, 2H, J = 6.5 Hz), 3.89 (t, 2H, J = 6.5 Hz), 3.76 (t, 2H, J = 6.5 Hz), 2.79 (t, 2H, J = 6.5 Hz), 1.65-1.79 (m, 4H), 1.41-1.50 (m, 4H)

### Step 5: Synthesis of intermediate E

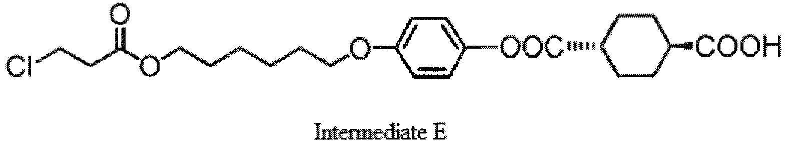

A three-necked reaction vessel equipped with a thermometer was charged with 12.50 g (72.60 mmol) of trans-1,4-cyclohexanedicarboxylic acid and 80 mL of THF in a stream of nitrogen. In addition, 4.35 g (37.97 mmol) of methanesulfonyl chloride was added into the reaction vessel and the reaction vessel was immersed in an ice water bath to attain a reaction liquid internal temperature of 5°C. Next, 4.03 g (39.83 mmol) of triethylamine was added dropwise over 5 minutes while maintaining the reaction liquid internal temperature at 5°C to 10°C. After completion of the dropwise addition, the entire contents of the reaction vessel were further stirred for 2 hours at 5°C to 10°C.

Next, 440 mg (3.60 mmol) of 4-(dimethylamino)pyridine and 10.9 g (36.24 mmol) of the intermediate D synthesized in step 4 were added to the resultant reaction liquid, and the reaction vessel was immersed in an ice water bath once again to attain a reaction liquid internal temperature of 5°C. Next, 4.03 g (39.83 mmol) of triethylamine was added dropwise over 5 minutes while maintaining the reaction liquid internal temperature at 5°C to 10°C. After completion of the dropwise addition, the ice water bath was removed and the entire contents of the reaction vessel were further stirred for 2 hours at 25°C. Once the reaction ended, 700 mL of distilled water and 70 mL of saturated saline water were added to the reaction liquid, and extraction was performed twice with 250 mL of ethyl acetate. The organic layers were collected and were dried with anhydrous sodium sulfate, and then sodium sulfate was filtered off. Solvent was evaporated from the filtrate under reduced pressure using a rotary evaporator and then the obtained residue was purified by silica gel column chromatography (chloroform:THF = 97:3) to yield 9.30 g (yield: 56.4%) of intermediate E in the form of a white solid.

The structure of the target was identified by ¹H-NMR.

¹H-NMR (500 MHz, DMSO-d₆, TMS, δ ppm): 12.12 (s, 1H), 6.99 (d, 2H, J = 9.0 Hz), 6.92 (d, 2H, J = 9.0 Hz), 4.07 (t, 2H, J = 6.5 Hz), 3.94 (t, 2H, J = 6.5 Hz), 3.79 (t, 2H, J = 6.5 Hz), 2.81 (t, 2H, J = 6.5 Hz), 2.47-2.56 (m, 1H), 2.18-2.27 (m, 1H), 2.01-2.11 (m, 2H), 1.93-2.01 (m, 2H), 1.65-1.74 (m, 2H), 1.57-1.65 (m, 2H), 1.34-1.52 (m, 8H)

### Step 6: Synthesis of intermediate F

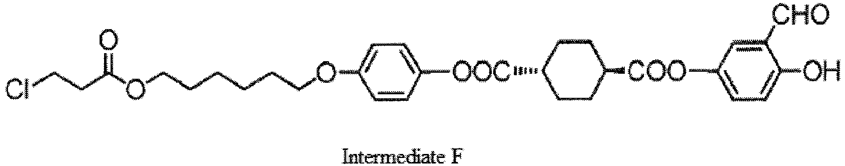

A three-necked reaction vessel equipped with a thermometer was charged with 4.90 g (10.77 mmol) of the intermediate E synthesized in step 5, 631 mg (8.63 mmol) of N,N-dimethylformamide, and 70 mL of toluene in a stream of nitrogen, a homogeneous solution was obtained, and the reaction vessel was immersed in an ice water bath to attain a reaction liquid internal temperature of 5°C. Next, 1.30 g (10.93 mmol) of thionyl chloride was added dropwise to the solution over 5 minutes while maintaining the reaction liquid internal temperature at 5°C to 10°C. After completion of the dropwise addition, the entire contents of the reaction vessel were further stirred for 2 hours at 5°C to 10°C. Once the reaction ended, the reaction liquid was concentrated using a rotary evaporator, 60 mL of THF was added to the resultant concentrate, and a homogeneous solution was obtained. Next, 7.50 g (54.30 mmol) of 2,5-dihydroxybenzaldehyde was added to the solution, the reaction vessel was immersed in an ice water bath to attain a reaction liquid internal temperature of 5°C, and 1.20 g (11.87 mmol) of triethylamine was added dropwise over 10 minutes. After completion of the dropwise addition, the entire contents of the reaction vessel were further stirred for 2 hours at 5°C to 10°C.

Once the reaction ended, 900 mL of distilled water and 150 mL of saturated saline water were added to the reaction liquid, and extraction was performed twice with 300 mL of ethyl acetate. The organic layers were dried with anhydrous sodium sulfate, and then sodium sulfate was filtered off. The filtrate was concentrated using a rotary evaporator. Thereafter, 300 mL of toluene was added to the concentrate and insoluble solid was removed by filtration. The filtrate was concentrated using a rotary evaporator to obtain a concentrate that was subsequently purified by silica gel column chromatography (toluene:THF = 95:5) to yield 2.77 g (yield: 44.6%) of intermediate F in the form of a white solid.

The structure of the target was identified by ¹H-NMR.

¹H-NMR (500 MHz, CDCl₃, TMS, δ ppm): 10.92 (s, 1H), 9.86 (s, 1H), 7.32 (d, 1H, J = 3.0 Hz), 7.22-7.28 (m, 1H), 7.01 (d, 1H, J = 9.0 Hz), 6.97 (d, 2H, J = 9.0 Hz), 6.88 (d, 2H, J = 9.0 Hz), 4.15 (t, 2H, J = 6.5 Hz), 3.94 (t, 2H, J = 6.5 Hz), 3.76 (t, 2H, J = 6.5 Hz), 2.79 (t, 2H, J = 6.5 Hz). 2.51-2.69 (m, 2H), 2.23-2.38 (m, 4H), 1.74-1.87 (m, 2H), 1.61-1.74 (m, 6H), 1.38-1.55 (m, 4H)

### Step 7: Synthesis of intermediate G

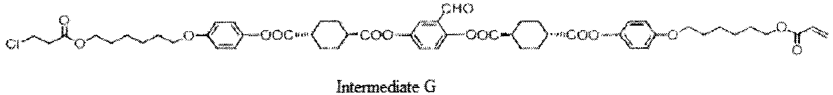

A three-necked reaction vessel equipped with a thermometer was charged with 2.80 g (6.75 mmol) of the intermediate B synthesized in step 2 and 40 mL of THF in a stream of nitrogen, and a homogeneous solution was obtained. Next, 773 mg (6.75 mmol) of methanesulfonyl chloride was added to the solution, and the reaction vessel was subsequently immersed in an ice water bath to attain a reaction liquid internal temperature of 5°C. In addition, 780 mg (7.71 mmol) of triethylamine was added dropwise over 5 minutes while maintaining the reaction liquid internal temperature at 5°C to 10°C. After completion of the dropwise addition, the ice water bath was removed, and the entire contents of the reaction vessel were caused to react for 1 hour at 25°C. Thereafter, 58.9 mg (0.482 mmol) of N,N-dimethylaminopyridine and 2.77 g (4.82 mmol) of the intermediate F synthesized in step 6 were added, and the reaction vessel was immersed in an ice water bath once again to attain a reaction liquid internal temperature of 5°C. Next, 585 mg (5.78 mmol) of triethylamine was added dropwise over 5 minutes while maintaining the reaction liquid internal temperature at 5°C to 10°C. After completion of the dropwise addition, the ice water bath was removed, and the entire contents of the reaction vessel were further stirred for 2 hours at 25°C.

Once the reaction ended, 500 mL of distilled water and 50 mL of saturated saline water were added to the reaction liquid, and extraction was performed twice with 300 mL of chloroform. The organic layers were collected and were dried with anhydrous sodium sulfate, and then sodium sulfate was filtered off. The filtrate was concentrated using a rotary evaporator, and the resultant solid was dissolved in 10 mL of THF. Crystals were caused to precipitate by adding 50 mL of methanol to the resultant solution and were subsequently filtered off. The obtained crystals were washed with methanol and were subsequently vacuum dried to yield 4.27 g (yield: 90.8%) of intermediate G in the form of a white solid.

The structure of the target was identified by ¹H-NMR.

¹H-NMR (500 MHz, CDCl₃, TMS, δ ppm): 10.08 (s, 1H), 7.61 (d, 1H, J = 3.0 Hz), 7.37 (dd, 1H, J = 3.0 Hz, 9.0 Hz), 7.20 (d, 1H, J = 9.0 Hz), 6.98 (d, 2H, J = 9.0 Hz), 6.97 (d, 2H, J = 9.0 Hz), 6.88 (d, 4H, J = 9.0 Hz), 6.40 (dd, 1H, J = 1.5 Hz, 17.5 Hz), 6.12 (dd, 1H, J = 10.5 Hz, 17.5 Hz), 5.82 (dd, 1H, J = 1.5 Hz, 10.5 Hz), 4.17 (t, 2H, J = 6.5 Hz), 4.15 (t, 2H, J = 6.5 Hz), 3.94 (t, 4H, J = 6.5 Hz), 3.76 (t, 2H, J = 6.5 Hz), 2.79 (t, 2H, J = 6.5 Hz), 2.66-2.75 (m, 1H), 2.53-2.66 (m, 3H), 2.23-2.43 (m, 8H), 1.75-1.84 (m, 4H), 1.62-1.75 (m, 12H), 1.39-1.55 (m, 8H)

### Step 8: Synthesis of intermediate H

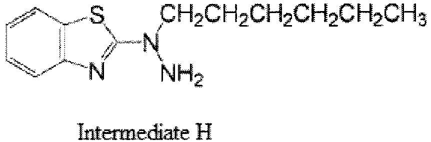

A four-necked reaction vessel equipped with a thermometer was charged with 2.00 g (12.1 mmol) of 2-hydrazinobenzothiazole and 20 mL of N,N-dimethylformamide in a stream of nitrogen, and a homogeneous solution was obtained. Next, 8.36 g (60.5 mmol) of potassium carbonate and 3.08 g (14.5 mmol) of 1-iodohexane were added to the solution, and the entire contents of the reaction vessel were stirred for 7 hours at 50°C. Once the reaction ended, the reaction liquid was cooled to 20°C and was added into 200 mL of water. Extraction was then performed with 300 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (hexane:ethyl acetate = 75:25) to yield 2.10 g (yield: 69.6%) of intermediate H in the form of a white solid.

The structure of the target was identified by ¹H-NMR.

¹H-NMR (500 MHz, CDCl₃, TMS, δ ppm): 7.60 (dd, 1H, J = 1.0 Hz, 8.0 Hz), 7.53 (dd, 1H, J = 1.0 Hz, 8.0 Hz), 7.27 (ddd, 1H, J = 1.0 Hz, 8.0 Hz, 8.0 Hz), 7.06 (ddd, 1H, J = 1.0 Hz, 8.0 Hz, 8.0 Hz), 4.22 (s, 2H), 3.74 (t, 2H, J = 7.5 Hz), 1.69-1.76 (m, 2H), 1.29-1.42 (m, 6H), 0.89 (t, 3H, J = 7.0 Hz)

### Step 9: Synthesis of compound 1

A three-necked reaction vessel equipped with a thermometer was charged with 2.38 g (2.44 mmol) of the intermediate G synthesized in step 7, 731 mg (2.93 mmol) of the intermediate H synthesized in step 8, 56.7 mg (0.244 mmol) of (±)-10-camphorsulfonic acid, 35 mL of THF, and 6 mL of ethanol in a stream of nitrogen, and the entire contents of the reaction vessel were stirred for 5 hours at 40°C. Once the reaction ended, the reaction liquid was added into 200 mL of water, and extraction was performed with 330 mL of ethyl acetate. The resultant ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (chloroform:THF = 99:1) to yield 2.02 g (yield: 68.7%) of compound 1 in the form of a pale yellow solid.

The structure of the target was identified by ¹H-NMR.

¹H-NMR (500 MHz, CDCl₃, TMS, δ ppm): 7.75 (d, 1H, J = 2.5 Hz), 7.64-7.72 (m, 3H), 7.34 (ddd, 1H, J = 1.0 Hz, 6.5 Hz, 7.5 Hz), 7.17 (ddd, 1H, J = 1.0 Hz, 7.5 Hz, 8.0 Hz), 7.07-7.14 (m, 2H), 6.99 (d, 2H, J = 9.0 Hz), 6.98 (d, 2H, J = 9.0 Hz), 6.88 (d, 4H, J = 9.0 Hz), 6.40 (dd, 1H, J = 1.5 Hz, 17.5 Hz), 6.13 (dd, 1H, J = 10.5 Hz, 17.5 Hz), 5.82 (dd, 1H, J = 1.5 Hz, 10.5 Hz), 4.30 (t, 2H, J = 7.5 Hz), 4.17 (t, 2H, J = 7.0 Hz), 4.15 (t, 2H, J = 6.5 Hz), 3.94 (t, 4H, J = 6.5 Hz), 3.76 (t, 2H, J = 7.0 Hz), 2.79 (t, 2H, J = 6.5 Hz), 2.53-2.75 (m, 4H), 2.25-2.42 (m, 8H), 1.64-1.85 (m, 18H), 1.29-1.56 (m, 14H), 0.90 (t, 3H, J = 7.0 Hz)

### (Synthesis Example 2) Synthesis of compound 2

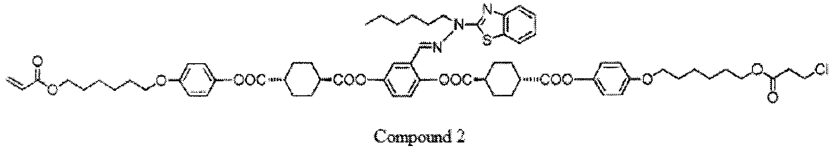

### Step 1: Synthesis of intermediate I

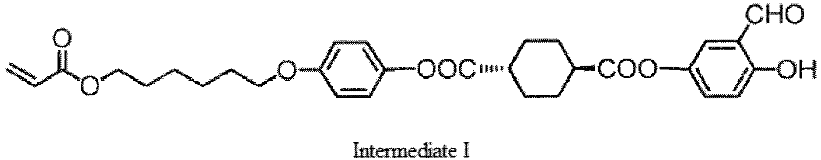

In a three-necked reaction vessel equipped with a thermometer, 1.50 g (3.58 mmol) of the intermediate B synthesized in step 1 of Synthesis Example 1 was dissolved in 30 mL of THF in a stream of nitrogen. Next, 431 mg (3.76 mmol) of methanesulfonyl chloride was added to the solution, and the reaction vessel was immersed in a water bath to attain a reaction liquid internal temperature of 15°C. In addition, 399 mg (3.94 mmol) of triethylamine was added dropwise over 5 minutes. After completion of the dropwise addition, further stirring was performed for 2 hours at 25°C. Next, 2.48 g (17.92 mmol) of 2,5-dihydroxybenzaldehyde and 40.0 mg (0.36 mmol) of 4-(dimethylamino)pyridine were added to the resultant reaction mixture. The reaction vessel was immersed in a water bath once again to attain a reaction liquid internal temperature of 15°C, and 440 mg (4.30 mmol) of triethylamine was added dropwise over 5 minutes. After completion of the dropwise addition, the entire contents of the reaction vessel were further stirred for 2 hours at 25°C.

Once the reaction ended, 300 mL of distilled water and 50 mL of saturated saline water were added to the reaction liquid, and extraction was performed twice with 100 mL of ethyl acetate. The organic layers were dried with anhydrous sodium sulfate, and then sodium sulfate was filtered off. The filtrate was concentrated using a rotary evaporator. Thereafter, 100 mL of toluene was added to the concentrate and insoluble solid was removed by filtration. The filtrate was concentrated using a rotary evaporator to obtain a concentrate that was subsequently purified by silica gel column chromatography (toluene:THF = 95:5) to yield 0.80 g (yield: 41.0%) of intermediate I in the form of a white solid.

The structure of the target was identified by ¹H-NMR.

¹H-NMR (500 MHz, CDCl₃, TMS, δ ppm): 10.91 (s, 1H), 9.86 (s, 1H), 7.32 (d, 1H, J = 3.0 Hz), 7.25 (dd, 1H, J = 3.0 Hz, 9.0 Hz), 7.01 (d, 1H, J = 9.0 Hz), 6.97 (d, 2H, J = 9.0 Hz), 6.87 (d, 2H, J = 9.0 Hz), 6.40 (dd, 1H, J = 1.5 Hz, 17.5 Hz), 6.12 (dd, 1H, J = 10.0 Hz, 17.5 Hz), 5.82 (dd, 1H, J = 1.5 Hz, 10.0 Hz), 4.17 (t, 2H, J = 6.5 Hz), 3.94 (t, 2H, J = 6.5 Hz), 2.53-2.65 (m, 2H), 2.23-2.35 (m, 4H), 1.75-1.84 (m, 2H), 1.62-1.75 (m, 6H), 1.41-1.55 (m, 4H)

### Step 2: Synthesis of intermediate J

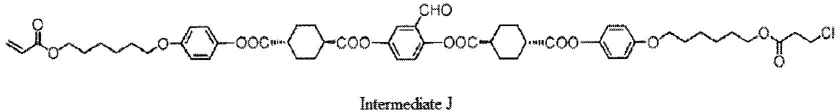

A three-necked reaction vessel equipped with a thermometer was charged with 2.50 g (5.59 mmol) of the intermediate E synthesized in step 5 of Synthesis Example 1 and 30 mL of THF in a stream of nitrogen, and a homogeneous solution was obtained. Next, 640 mg (5.59 mmol) of methanesulfonyl chloride was added to the solution, and the reaction vessel was subsequently immersed in an ice water bath to attain a reaction liquid internal temperature of 5°C. In addition, 646 mg (6.38 mmol) of triethylamine was added dropwise over 5 minutes while maintaining the reaction liquid internal temperature at 5°C to 10°C. After completion of the dropwise addition, the ice water bath was removed, and the entire contents of the reaction vessel were caused to react for 1 hour at 25°C. Thereafter, 48.7 mg (0.399 mmol) of N,N-dimethylaminopyridine and 2.15 g (3.99 mmol) of the intermediate I synthesized in step 1 were added, and the reaction vessel was immersed in an ice water bath once again to attain a reaction liquid internal temperature of 5°C. Next, 485 mg (4.79 mmol) of triethylamine was added dropwise over 5 minutes while maintaining the reaction liquid internal temperature at 5°C to 10°C. After completion of the dropwise addition, the ice water bath was removed, and the entire contents of the reaction vessel were further stirred for 2 hours at 25°C.

Once the reaction ended, 400 mL of distilled water and 40 mL of saturated saline water were added to the reaction liquid, and extraction was performed twice with 250 mL of chloroform. The organic layers were collected and were dried with anhydrous sodium sulfate, and then sodium sulfate was filtered off. The filtrate was concentrated using a rotary evaporator to obtain a solid that was then dissolved in 10 mL of THF. Crystals were caused to precipitate by adding 40 mL of methanol to the solution and were collected by filtration. The obtained crystals were washed with methanol and were subsequently vacuum dried to yield 3.40 g (yield: 89.0%) of intermediate J in the form of a white solid.

The structure of the target was identified by ¹H-NMR.

¹H-NMR (500 MHz, CDCl₃, TMS, δ ppm): 10.08 (s, 1H), 7.61 (d, 1H, J = 2.5 Hz), 7.37 (dd, 1H, J = 2.5 Hz, 9.0 Hz), 7.20 (d, 1H, J = 9.0 Hz), 6.98 (d, 2H, J = 9.0 Hz), 6.97 (d, 2H, J = 9.0 Hz), 6.88 (d, 4H, J = 9.0 Hz), 6.40 (dd, 1H, J = 1.5 Hz, 17.5 Hz), 6.12 (dd, 1H, J = 10.5 Hz, 17.5 Hz), 5.82 (dd, 1H, J = 1.5 Hz, 10.5 Hz), 4.17 (t, 2H, J = 6.5 Hz), 4.15 (t, 2H, J = 6.5 Hz), 3.94 (t, 4H, J = 6.5 Hz), 3.76 (t, 2H, J = 6.5 Hz), 2.79 (t, 2H, J = 6.5 Hz), 2.65-2.74 (m, 1H), 2.43-2.65 (m, 3H), 2.16-2.38 (m, 8H), 1.75-1.84 (m, 4H), 1.64-1.75 (m, 12H), 1.39-1.55 (m, 8H)

### Step 3: Synthesis of compound 2

A three-necked reaction vessel equipped with a thermometer was charged with 2.00 g (2.05 mmol) of the intermediate J synthesized in step 2, 613 mg (2.46 mmol) of the intermediate H synthesized in step 8 of Synthesis Example 1, 47.6 mg (0.205 mmol) of (±)-10-camphorsulfonic acid, 30 mL of THF, and 4 mL of ethanol in a stream of nitrogen, and the entire contents of the reaction vessel were stirred for 5 hours at 40°C. Once the reaction ended, the reaction liquid was added into 200 mL of water, and extraction was performed with 300 mL of ethyl acetate. The resultant ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (chloroform:THF = 99:1) to yield 1.58 g (yield: 63.9%) of compound 2 in the form of a pale yellow solid.

The structure of the target was identified by ¹H-NMR.

¹H-NMR (500 MHz, CDCl₃, TMS, δ ppm): 7.75 (d, 1H, J = 2.5 Hz), 7.64-7.73 (m, 3H), 7.34 (ddd, 1H, J = 1.0 Hz, 7.5 Hz, 8.0 Hz), 7.17 (ddd, 1H, J = 1.0 Hz, 7.5 Hz, 7.5 Hz), 7.07-7.14 (m, 2H), 6.99 (d, 2H, J = 9.0 Hz), 6.98 (d, 2H, J = 9.0 Hz), 6.88 (d, 4H, J = 9.0 Hz), 6.40 (dd, 1H, J = 1.5 Hz, 17.5 Hz), 6.13 (dd, 1H, J = 10.5 Hz, 17.5 Hz), 5.82 (dd, 1H, J = 1.5 Hz, 10.5 Hz), 4.30 (t, 2H, J = 8.0 Hz), 4.18 (t, 2H, J = 6.5 Hz), 4.15 (t, 2H, J = 6.5 Hz), 3.95 (t, 4H, J = 6.5 Hz), 3.76 (t, 2H, J = 6.5 Hz), 2.79 (t, 2H, J = 6.5 Hz), 2.52-2.74 (m, 4H), 2.24-2.41 (m, 8H), 1.64-1.88 (m, 18H), 1.25-1.56 (m, 14H), 0.90 (t, 3H, J = 7.0 Hz)

### (Synthesis Example 3) Synthesis of compound 3

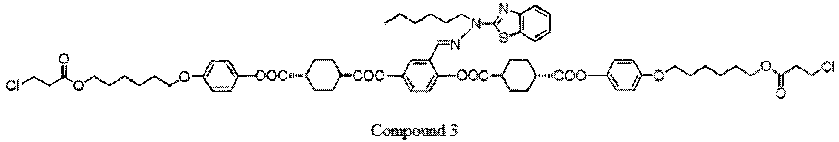

A three-necked reaction vessel equipped with a thermometer was charged with 1.00 g (2.21 mmol) of the intermediate E synthesized in step 5 of Synthesis Example 1, 132 mg (1.81 mmol) of N,N-dimethylformamide, and 15 mL of toluene in a stream of nitrogen, and a homogeneous solution was obtained. The reaction vessel was immersed in an ice water bath to attain a reaction liquid internal temperature of 5°C. In addition, 274 mg (2.30 mmol) of thionyl chloride was added dropwise over 5 minutes while maintaining the reaction liquid internal temperature at 5°C to 10°C. After completion of the dropwise addition, the entire contents of the reaction vessel were further stirred for 1 hour at 5°C to 10°C. Once the reaction ended, the reaction liquid was concentrated using a rotary evaporator, 15 mL of THF was added to the resultant concentrate, and a homogeneous solution was obtained. Next, 127 mg (0.919 mmol) of 2,5-dihydroxybenzaldehyde was added to the solution, the reaction vessel was immersed in an ice water bath to attain a reaction liquid internal temperature of 5°C, and 222 mg (2.20 mmol) of triethylamine was added dropwise over 5 minutes. After completion of the dropwise addition, the entire contents of the reaction vessel were further stirred for 2 hours at 5°C to 10°C. Next, 2.20 mL (2.20 mmol) of 1 N hydrochloric acid and 302 mg (1.21 mmol) of the intermediate H synthesized in step 8 of Synthesis Example 1 were added, and the entire contents of the reaction vessel were stirred for 3 hours at 40°C.

Once the reaction ended, the reaction liquid was added into 20 mL of water, and extraction was performed with 20 mL of ethyl acetate. The resultant ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (chloroform:THF = 99:1) to yield 431 mg (yield: 37.7%) of compound 3 in the form of a pale yellow solid.

The structure of the target was identified by ¹H-NMR.

¹H-NMR (500 MHz, CDCl₃, TMS, δ ppm): 7.75 (d, 1H, J = 2.5 Hz), 7.66-7.71 (m, 3H), 7.34 (ddd, 1H, J = 1.0 Hz, 6.5 Hz, 7.5 Hz), 7.17 (ddd, 1H, J = 1.0 Hz, 7.5 Hz, 7.5 Hz), 7.08-7.14 (m, 2H), 6.99 (d, 2H, J = 9.0 Hz), 6.98 (d, 2H, J = 9.0 Hz), 6.88 (d, 4H, J = 9.0 Hz), 4.30 (t, 2H, J = 7.5 Hz), 4.15 (t, 4H, J = 6.5 Hz), 3.95 (t, 4H, J = 6.5 Hz), 3.76 (t, 4H, J = 6.5 Hz), 2.79 (t, 4H, J = 6.5 Hz), 2.56-2.72 (m, 4H), 2.27-2.38 (m, 8H), 1.65-1.84 (m, 18H), 1.29-1.55 (m, 14H), 0.90 (t, 3H, J = 7.0 Hz)

### (Synthesis Example 4) Synthesis of compound 4

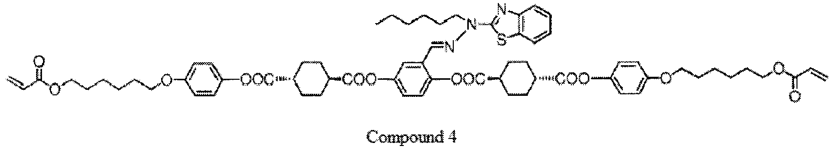

### Step 1: Synthesis of intermediate K

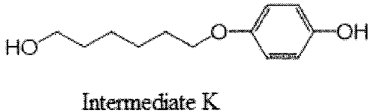

A three-necked reaction vessel equipped with a condenser and a thermometer was charged with 104.77 g (0.9515 mol) of hydroquinone, 100 g (0.7320 mol) of 6-chlorohexanol, 500 mL of distilled water, and 100 mL of o-xylene in a stream of nitrogen. The entire contents of the reaction vessel were stirred while gradually adding 35.15 g (0.8784 mol) of sodium hydroxide over 20 minutes such that the reaction liquid internal temperature did not exceed 40°C. After completion of addition of the sodium hydroxide, the contents were heated and a reaction was carried out for 12 hours under reflux conditions (96°C).

The reaction liquid internal temperature was lowered to 80°C after the reaction ended, and 200 mL of distilled water was added. Thereafter, the reaction liquid was cooled to 10°C to cause precipitation of crystals. Solid-liquid separation was carried out by filtration of the precipitated crystals. The resultant crystals were washed with 500 mL of distilled water and were vacuum dried to yield 123.3 g of brown crystals. The brown crystals were purified by silica gel column chromatography (chloroform:methanol = 90:10) to yield 20 g (yield: 13%) of intermediate K in the form of a white solid.

### Step 2: Synthesis of intermediate AA

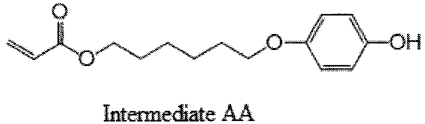

In a three-necked reaction vessel equipped with a condenser and a thermometer, 20 g (95.12 mmol) of the intermediate K synthesized in the preceding step 1 and 12.3 g (95.12 mmol) of N,N-diisopropylethylamine were dissolved in 500 mL of tetrahydrofuran in a stream of nitrogen. The solution was cooled using an ice bath and then 5.16 g (57.01 mmol) of acryloyl chloride was slowly added dropwise while controlling the temperature of the solution to 10°C or lower. After completion of the dropwise addition, a reaction was carried out for 2 hours in the ice bath. Once the reaction ended, the reaction liquid was added into 1 L of 0.1 N hydrochloric acid aqueous solution, and extraction was performed twice with 300 mL of ethyl acetate. The resultant ethyl acetate layers were washed with 300 mL of saturated saline water. Thereafter, the ethyl acetate layers were dried with anhydrous sodium sulfate, and then sodium sulfate was removed by filtration. Ethyl acetate was evaporated using a rotary evaporator to yield a pale yellow solid. The pale yellow solid was purified by silica gel column chromatography (toluene:ethyl acetate = 95:5) to yield 5.6 g (yield: 37%) of a white solid (crude intermediate AA) containing intermediate AA.

Through HPLC analysis of the obtained solid, it was confirmed that the solid contained the following intermediate AA' (halogenated compound of intermediate AA) in a proportion of 2.1 mass% among the total of the intermediate AA and the intermediate AA'.

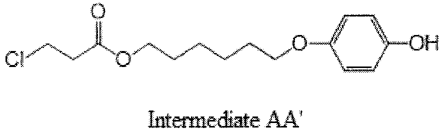

### Step 3: Synthesis of mixture L

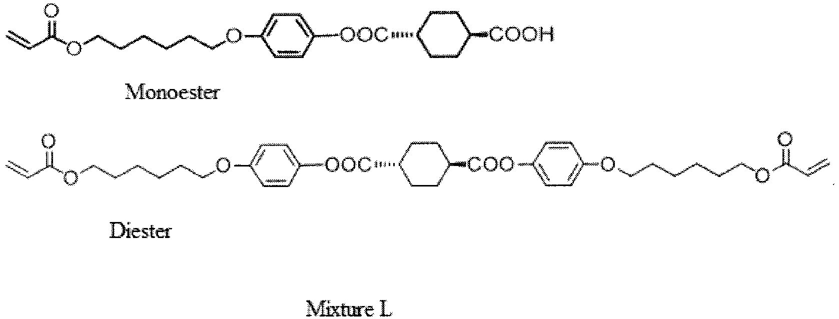

A three-necked reaction vessel equipped with a thermometer was charged with 10.0 g (47.83 mmol) of trans-1,4-cyclohexanedicarboxylic acid dichloride, 84 mL of cyclopentyl methyl ether (CPME), and 31 mL of THF in a stream of nitrogen. In addition, 12.04 g of the crude intermediate AA synthesized in step 2 was added into the reaction vessel and the reaction vessel was immersed in an ice bath to attain a reaction liquid internal temperature of 0°C. Next, 4.83 g (47.83 mmol) of triethylamine was slowly added dropwise over 5 minutes while maintaining the reaction liquid internal temperature at 10°C or lower. After completion of the dropwise addition, the entire contents of the reaction vessel were further stirred for 1 hour while maintaining the contents at 10°C or lower.

Thereafter, 30 mL of distilled water was added to the resultant reaction liquid. The reaction liquid was heated to 50°C, washing (hydrolysis) was subsequently performed for 2 hours, and then the aqueous layer was removed. Next, 30 mL of distilled water was added to the resultant organic layer, washing (hydrolysis) of the entire contents of the reaction vessel was performed for 2 hours at 50°C, and then the aqueous layer was removed. The resultant organic layer was cooled to 40°C and was washed five times with 50 mL of a buffer solution (pH 5.5) containing acetic acid in a concentration of 1 mol/L and sodium acetate. After this washing, the buffer solution was removed. The resultant organic layer was further washed with 30 mL of distilled water, and the aqueous layer was subsequently removed.

Next, 220 mL of n-hexane was added to the resultant organic layer and then the organic layer was cooled to 0°C to cause precipitation of crystals. The precipitated crystals were subsequently collected by filtration. The filtration residue was washed with n-hexane and was subsequently vacuum dried to yield 16.78 g of mixture L in the form of a white solid.

The obtained crystals were analyzed by HPLC to quantify the monoester and the diester using a calibration curve. It was confirmed that the crystals contained 11.49 g (27.45 mmol) of the monoester (target) and 5.29 g (7.96 mmol) of the diester. When the obtained crystals were analyzed by ¹³C-NMR (DMF-d₇) and the content of cyclohexanedicarboxylic acid was calculated, the content was below the limit of detection. Moreover, when the molar contents of the monoester and the diester were calculated from the compositional ratio thereof, the monoester content was 77.52 mol% and the diester content was 22.48 mol%.

### Step 4: Synthesis of compound 4

A three-necked reaction vessel equipped with a thermometer was charged with 16.78 g (entire amount) of the mixture L synthesized in step 3, 115 g of chloroform, and 4.0 g of DMF in a stream of nitrogen, and these materials were cooled to 10°C or lower. Next, 3.76 g (31.57 mmol) of thionyl chloride was added dropwise into the reaction vessel while controlling the reaction temperature to 10°C or lower. After completion of the dropwise addition, the reaction liquid was returned to 25°C and was stirred for 1 hour. Once the reaction ended, the reaction liquid was concentrated using an evaporator until the volume thereof had been reduced to 1/4 of the initial volume. Thereafter, 28.7 g of chloroform was added to the concentrated reaction liquid to obtain a chloroform solution.

Separately, in a three-necked reaction vessel equipped with a thermometer, 1.72 g (12.48 mmol) of 2,5-dihydroxybenzaldehyde and 7.58 g (74.88 mmol) of triethylamine were dissolved in 57 g of chloroform in a stream of nitrogen, and were cooled to 10°C or lower. The aforementioned chloroform solution was slowly added dropwise to this solution while maintaining the reaction liquid internal temperature at 10°C or lower. After completion of the dropwise addition, the reaction liquid was caused to further react for 1 hour while maintaining the reaction liquid at 10°C or lower.

Once the reaction ended, 4.05 g (16.22 mmol) of the intermediate H synthesized in step 8 of the preceding Synthesis Example 1 was added, and 45 g of 1.0 N hydrochloric acid aqueous solution was further added while the reaction liquid was still at 10°C or lower. The reaction liquid was subsequently heated to 40°C and a reaction was carried out for 3 hours. Once the reaction ended, the reaction liquid was cooled to 25°C and was subjected to a liquid separation operation.

Next, 0.57 g of Rokahelp #479 (produced by Mitsui Mining & Smelting Co., Ltd.) was added to the resultant organic layer, was stirred therewith for 30 minutes, and was then filtered off. Thereafter, approximately 80% of the total weight was removed from the resultant reaction liquid in an evaporator to perform concentration. Next, 23 g of THF was added to the solution and was stirred therewith for 1 hour. This was followed by dropwise addition of 92 g of n-hexane to the solution. The solution was subsequently cooled to 0°C to cause precipitation of crystals. Thereafter, the precipitated crystals were collected by filtration.

Next, 120 g of THF, 2.1 g of Rokahelp #479, and 110 mg of 2,6-di-t-butyl-4-methylphenol were added to the obtained crystals and were stirred therewith for 30 minutes. The Rokahelp #479 was subsequently filtered off. Next, 40 g of THF was evaporated from the resultant reaction liquid in an evaporator. Dropwise addition of 134 g of methanol to the solution was performed, and then the solution was cooled to 0°C to cause precipitation of crystals. The precipitated crystals were subsequently collected by filtration. The filtration residue was washed with methanol and was then vacuum dried to yield 12.02 g (yield: 82.3%) of a solid (crude compound 4).

Through analysis of the obtained solid by HPLC, it was confirmed that the solid contained the following compound 4' (halogenated compound of compound 4) in a proportion of 1.5 mass% among the total of the compound 4 and the compound 4'.

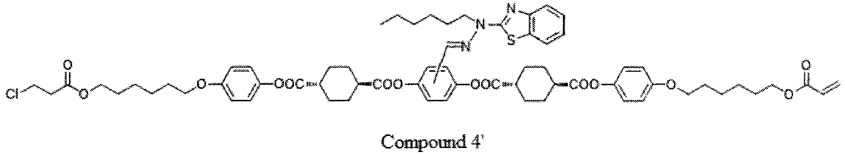

### (Synthesis Example 5) Synthesis of compound 5

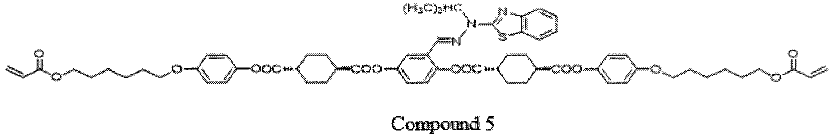

### Step 1: Synthesis of intermediate M

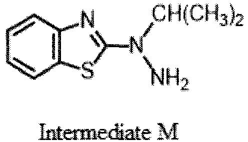

In a three-necked reaction vessel equipped with a thermometer, 30.0 g (181.6 mmol) of 2-hydrazinobenzothiazole was dissolved in 500 mL of DMF in a stream of nitrogen. Next, 118.3 g (363.2 mmol) of cesium carbonate was added to the solution. The solution was cooled to 0°C, and 33.3 g (272.3 mmol) of 2-bromopropane was added thereto. The entire contents of the reaction vessel were stirred for 1 hour at 0°C and were then further stirred for 20 hours at 25°C. Thereafter, 1,500 mL of distilled water was added to the reaction liquid, and extraction was performed twice with 1,000 mL of ethyl acetate. The organic layers were dried with anhydrous sodium sulfate, and then sodium sulfate was filtered off. The filtrate was concentrated using a rotary evaporator to obtain a concentrate that was subsequently purified by silica gel column chromatography (THF:toluene = 1:9) to yield 11.1 g (yield: 29%) of intermediate M in the form of a white solid.

The structure of the target was identified by ¹H-NMR.

¹H-NMR (500 MHz, DMSO-d₆, TMS, δ ppm): 7.65 (dd, 1H, J = 1.0 Hz, 8.0 Hz), 7.35 (dd, 1H, J = 1.0 Hz, 8.0 Hz), 7.20 (dt, 1H, J = 1.0 Hz, 7.5 Hz), 6.98 (dt, 1H, J = 1.0 Hz, 7.5 Hz), 5.10 (s, 2H), 4.61-4.72 (m, 1H), 1.17 (d, 6H, J = 6.5 Hz)

### Step 2: Synthesis of compound 5

Operations were carried out in the same manner as in Synthesis Example 4 with the exception that in step 4 of Synthesis Example 4, 4.05 g (16.22 mmol) of the intermediate H was changed to 3.36 g (16.22 mmol) of the intermediate M synthesized in step 1. As a result, 11.08 g (yield: 78.7%) of a solid (crude compound 5) was obtained.

Through analysis of the obtained solid by HPLC, it was confirmed that the solid contained the following compound 5' (halogenated compound of compound 5) in a proportion of 1.3 mass% among the total of the compound 5 and the compound 5'.

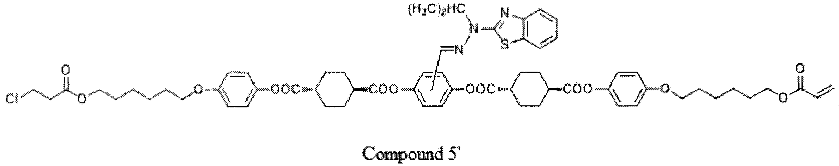

### (Synthesis Example 6) Synthesis of compound 6

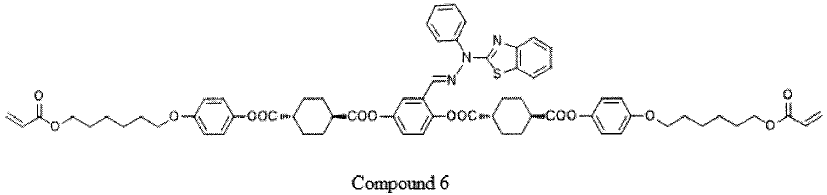

### Step 1: Synthesis of intermediate N

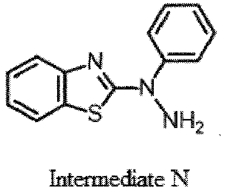

In a three-necked reaction vessel equipped with a thermometer, 15.0 g (88.45 mmol) of 2-chlorobenzothiazole and 38.25 g (353.7 mmol) of phenylhydrazine were dissolved in 150 mL of ethylene glycol in a stream of nitrogen. The solution was heated to 140°C and was stirred for 5 hours. Once the reaction ended, 1,000 mL of distilled water was added to the reaction liquid, and extraction was performed twice with 500 mL of ethyl acetate. The organic layers were dried with anhydrous sodium sulfate, and then sodium sulfate was filtered off. The filtrate was concentrated using a rotary evaporator to obtain a concentrate that was then dissolved through addition of 50 mL of THF. The solution was added into 1,000 mL of distilled water, and precipitated solid was collected by filtration. The filtration residue was washed with distilled water and was then vacuum dried to yield a yellow solid. The yellow solid was loaded into a flask, and 250 mL of toluene was added and stirred therewith for 30 minutes. Thereafter, solid content that did not dissolve in the toluene was removed by filtration. The filtrate was concentrated using a rotary evaporator to obtain a concentrate that was subsequently purified by silica gel column chromatography (THF:toluene = 2:50) to yield 4.70 g (yield: 22%) of intermediate N in the form of a yellow oil.

The structure of the target was identified by ¹H-NMR.

¹H-NMR (500 MHz, DMSO-d₆, TMS, δ ppm): 8.01 (dd, 2H, J = 1.0 Hz, 9.0 Hz), 7.78 (dd, 1H, J = 1.0 Hz, 8.0 Hz), 7.51 (dd, 1H, J = 1.0 Hz, 8.0 Hz), 7.43 (dd, 2H, J = 7.5 Hz, 8.5 Hz), 7.28 (dt, 1H, J = 1.0 Hz, 7.5 Hz), 7.08-7.16 (m, 2H), 6.26 (s, 2H)

### Step 2: Synthesis of compound 6

Operations were carried out in the same manner as in Synthesis Example 4 with the exception that in step 4 of Synthesis Example 4, 4.05 g (16.22 mmol) of the intermediate H was changed to 3.91 g (16.22 mmol) of the intermediate N synthesized in step 1. As a result, 10.65 g (yield: 73.4%) of a solid (crude compound 6) was obtained.

Through analysis of the obtained solid by HPLC, it was confirmed that the solid contained the following compound 6' (halogenated compound of compound 6) in a proportion of 0.6 mass% among the total of the compound 6 and the compound 6'.

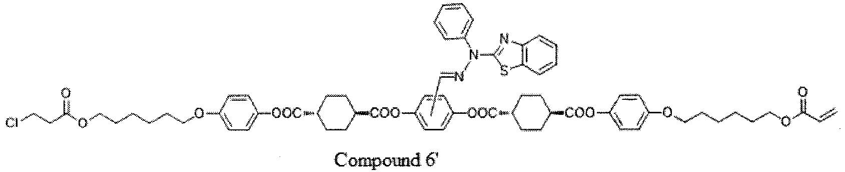

### (Synthesis Example 7) Synthesis of compound 7

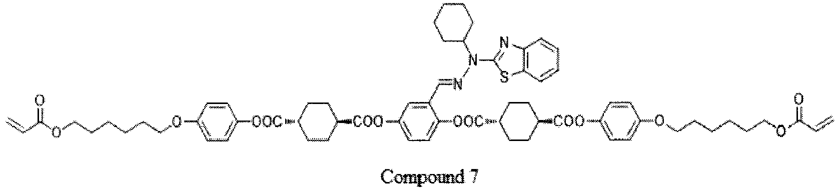

### Step 1: Synthesis of intermediate O

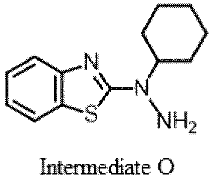

In a four-necked reaction vessel equipped with a thermometer, 12.5 g (83.0 mmol) of cyclohexylhydrazine hydrochloride was dissolved in 40 mL of triethylamine in a stream of nitrogen. In addition, 28.15 g (166.0 mmol) of 2-chlorobenzothiazole was added to the solution, and the entire contents of the reaction vessel were stirred for 5 hours at 80°C. Once the reaction ended, the reaction liquid was cooled to 20°C, was added into 500 mL of saturated sodium hydrogen carbonate aqueous solution, and extraction was performed with 1,000 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (hexane:ethyl acetate = 75:25) to yield 5.10 g (yield: 24.8%) of intermediate O in the form of a white solid.

The structure of the target was identified by ¹H-NMR.

¹H-NMR (400 MHz, CDCl₃, TMS, δ ppm): 7.58 (d, 1H, J = 7.8 Hz), 7.52 (d, 1H, J = 8.2 Hz), 7.26 (dd, 1H, J = 7.4 Hz, 8.2 Hz), 7.05 (dd, 1H, J = 7.4 Hz, 7.8 Hz), 4.25-4.32 (m, 1H), 4.04 (s, 2H), 1.84-1.88 (m, 4H), 1.68-1.73 (m, 1H), 1.43-1.59 (m, 4H), 1.08-1.19 (m, 1H)

### Step 2: Synthesis of compound 7

Operations were carried out in the same manner as in Synthesis Example 4 with the exception that in step 4 of Synthesis Example 4, 4.05 g (16.22 mmol) of the intermediate H was changed to 4.01 g (16.22 mmol) of the intermediate O synthesized in step 1. As a result, 11.11 g (yield: 76.2%) of a solid (crude compound 7) was obtained.

Through analysis of the obtained solid by HPLC, it was confirmed that the solid contained the following compound 7' (halogenated compound of compound 7) in a proportion of 0.4 mass% among the total of the compound 7 and the compound 7'.

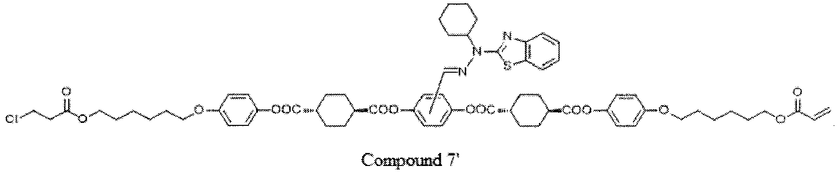

### (Synthesis Example 8) Synthesis of compound 8

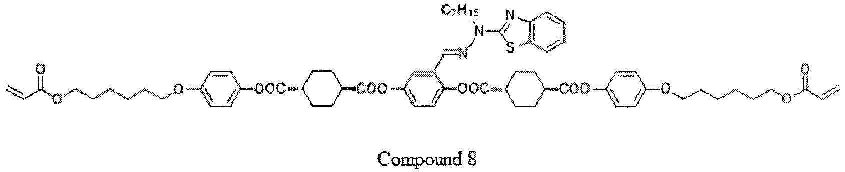

### Step 1: Synthesis of intermediate P

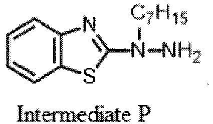

In a three-necked reaction vessel equipped with a thermometer, 10.0 g (60.5 mmol) of 2-hydrazinobenzothiazole was dissolved in 150 mL of DMF in a stream of nitrogen. Next, 39.4 g (121.0 mmol) of cesium carbonate was added to the solution. The solution was cooled to 0°C, and 16.4 g (72.5 mmol) of iodoheptane was added dropwise over 5 minutes. After completion of the dropwise addition, the entire contents of the reaction vessel were stirred for 3 hours at 25°C. Once the reaction ended, 1,000 mL of water was added to the reaction liquid, and extraction was performed twice with 500 mL of ethyl acetate. The organic layers were dried with anhydrous sodium sulfate, and then sodium sulfate was filtered off. The filtrate was concentrated using a rotary evaporator to obtain a concentrate that was subsequently purified by silica gel column chromatography (n-hexane:ethyl acetate = 85:15) to yield 9.05 g (yield: 56.9%) of intermediate P in the form of a white solid.

The structure of the target was identified by ¹H-NMR.

¹H-NMR (500 MHz, CDCl₃, TMS, δ ppm): 7.59 (dd, 1H, J = 1.5 Hz, 8.0 Hz), 7.53 (dd, 1H, J = 1.5 Hz, 8.0 Hz), 7.06-7.28 (m, 2H), 4.22 (s, 2H), 3.75 (t, 2H, J = 7.0 Hz), 1.29-1.38 (m, 10H), 0.88 (t, 3H, J = 7.0 Hz)

### Step 2: Synthesis of compound 8

Operations were carried out in the same manner as in Synthesis Example 4 with the exception that in step 4 of Synthesis Example 4, 4.05 g (16.22 mmol) of the intermediate H was changed to 4.27 g (16.22 mmol) of the intermediate P synthesized in step 1. As a result, 11.96 g (yield: 80.9%) of a solid (crude compound 8) was obtained.

Through analysis of the obtained solid by HPLC, it was confirmed that the solid contained the following compound 8' (halogenated compound of compound 8) in a proportion of 0.5 mass% among the total of the compound 8 and the compound 8'.

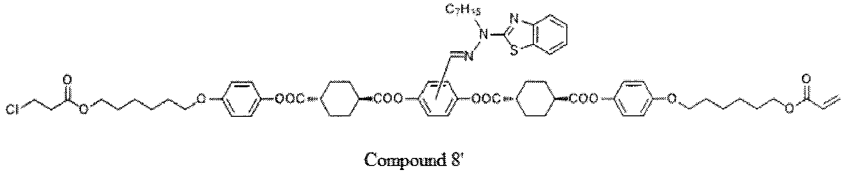

### (Synthesis Example 9) Synthesis of compound 9

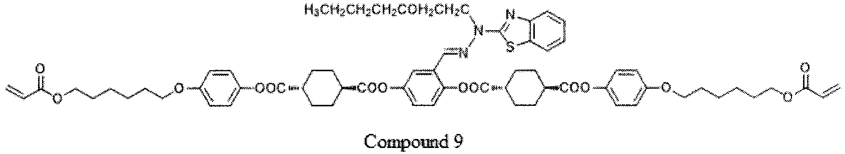

### Step 1: Synthesis of intermediate Q

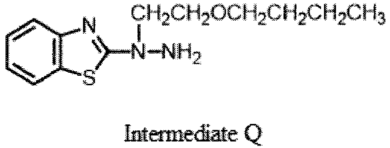

In a three-necked reaction vessel equipped with a thermometer, 10.0 g (60.5 mmol) of 2-hydrazinobenzothiazole was dissolved in 150 mL of DMF in a stream of nitrogen. Next, 39.4 g (121.0 mmol) of cesium carbonate was added to the solution. The solution was cooled to 0°C, and 9.90 g (72.5 mmol) of butyl 2-chloroethyl ether was added dropwise over 5 minutes. After completion of the dropwise addition, the entire contents of the reaction vessel were stirred for 3 hours at 25°C. Once the reaction ended, 1,000 mL of water was added to the reaction liquid, and extraction was performed twice with 500 mL of ethyl acetate. The organic layers were dried with anhydrous sodium sulfate, and then sodium sulfate was filtered off. The filtrate was concentrated using a rotary evaporator to obtain a concentrate that was subsequently purified by silica gel column chromatography (n-hexane:ethyl acetate = 75:25) to yield 8.50 g (yield: 53.0%) of intermediate Q in the form of a white solid.

The structure of the target was identified by ¹H-NMR.

¹H-NMR (500 MHz, CDCl₃, TMS, δ ppm): 7.61 (dd, 1H, J = 1.0 Hz, 8.0 Hz), 7.50 (dd, 1H, J = 1.0 Hz, 8.0 Hz), 7.27-7.29 (m, 1H), 7.04-7.08 (m, 1H), 4.70 (s, 2H), 4.01 (t, 2H, J = 5.0 Hz), 3.82 (t, 2H, J = 5.0 Hz), 3.44 (t, 2H, J = 7.0 Hz), 1.52-1.57 (m, 2H), 1.31-1.39 (m, 2H), 0.90 (t, 3H, J = 7.0 Hz)

### Step 2: Synthesis of compound 9

Operations were carried out in the same manner as in Synthesis Example 4 with the exception that in step 4 of Synthesis Example 4, 4.05 g (16.22 mmol) of the intermediate H was changed to 4.30 g (16.22 mmol) of the intermediate Q synthesized in step 1. As a result, 11.77 g (yield: 79.5%) of a solid (crude compound 9) was obtained.

Through analysis of the obtained solid by HPLC, it was confirmed that the solid contained the following compound 9' (halogenated compound of compound 9) in a proportion of 0.3 mass% among the total of the compound 9 and the compound 9'.

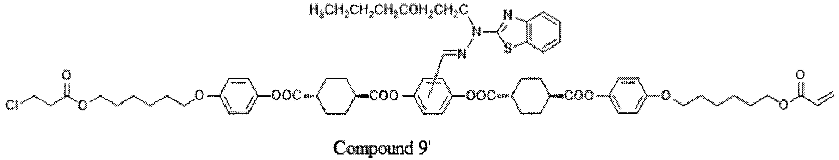

### (Synthesis Example 10) Synthesis of compound 10

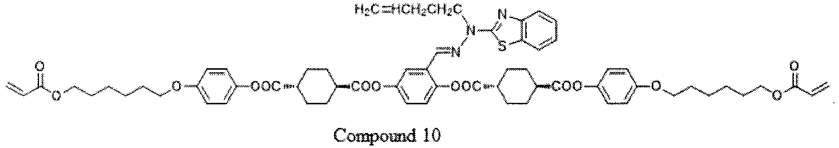

### Step 1: Synthesis of intermediate R

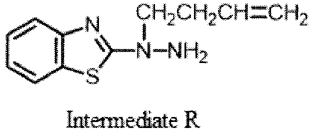

In a four-necked reaction vessel equipped with a thermometer, 5.04 g (30.5 mmol) of 2-hydrazinobenzothiazole was dissolved in 50 mL of DMF in a stream of nitrogen. Next, 14.9 g (45.8 mmol) of cesium carbonate and 4.94 g (36.6 mmol) of 4-bromo-1-butene were added to the solution, and the entire contents of the reaction vessel were stirred for 7 hours at 25°C. Once the reaction ended, the reaction liquid was added into 200 mL of water, and extraction was performed with 300 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (hexane:ethyl acetate = 70:30) to yield 4.40 g (yield: 65.8%) of intermediate R in the form of a white solid.

The structure of the target was identified by ¹H-NMR.

¹H-NMR (500 MHz, CDCl₃, TMS, δ ppm): 7.60 (dd, 1H, J = 1.0 Hz, 8.0 Hz), 7.54 (dd, 1H, J = 1.0 Hz, 8.0 Hz), 7.28 (ddd, 1H, J = 1.0 Hz, 7.5 Hz, 8.0 Hz), 7.06 (ddd, 1H, J = 1.0 Hz, 7.5 Hz, 8.0 Hz), 5.89 (ddt, 1H, J = 7.0 Hz, 10.5 Hz, 17.0 Hz), 5.17 (ddt, 1H, J = 1.5 Hz, 3.0 Hz, 17.0 Hz), 5.09 (ddt, 1H, J = 1.0 Hz, 3.0 Hz, 10.5 Hz), 4.26 (s, 2H), 3.85 (t, 2H, J = 7.0 Hz), 2.52 (dddt, 2H, J = 1.0 Hz, 1.5 Hz, 7.0 Hz, 7.0 Hz)

### Step 2: Synthesis of compound 10

Operations were carried out in the same manner as in Synthesis Example 4 with the exception that in step 4 of Synthesis Example 4, 4.05 g (16.22 mmol) of the intermediate H was changed to 3.56 g (16.22 mmol) of the intermediate R synthesized in step 1. As a result, 9.88 g (yield: 69.4%) of a solid (crude compound 10) was obtained.

Through analysis of the obtained solid by HPLC, it was confirmed that the solid contained the following compound 10' (halogenated compound of compound 10) in a proportion of 1.0 mass% among the total of the compound 10 and the compound 10'.

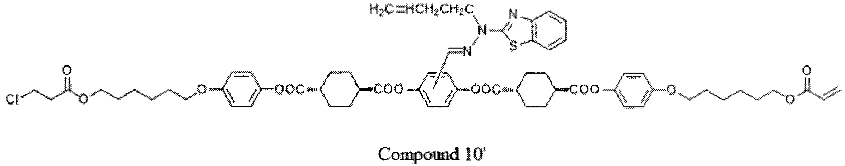

### (Synthesis Example 11) Synthesis of compound 11

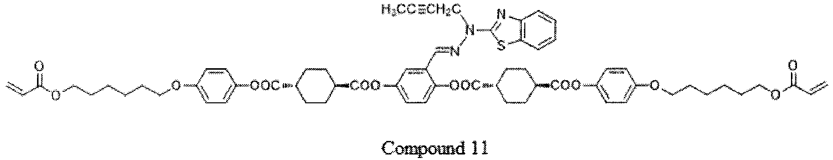

### Step 1: Synthesis of intermediate S

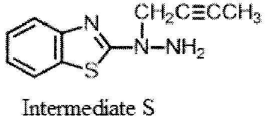

In a four-necked reaction vessel equipped with a thermometer, 10.0 g (60.5 mmol) of 2-hydrazinobenzothiazole was dissolved in 150 mL of DMF in a stream of nitrogen. Next, 39.4 g (121.0 mmol) of cesium carbonate and 9.65 g (72.5 mmol) of 1-bromo-2-butene were added to the solution, and the entire contents of the reaction vessel were stirred for 20 hours at 25°C. Once the reaction ended, the reaction liquid was added into 1,000 mL of water, and extraction was performed with 500 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a brown solid. The brown solid was purified by silica gel column chromatography (n-hexane:ethyl acetate = 85:15) to yield 6.25 g (yield: 47.5%) of intermediate S in the form of a white solid.

The structure of the target was identified by ¹H-NMR.

¹H-NMR (500 MHz, CDCl₃, TMS, δ ppm): 7.63 (dd, 1H, J = 1.3 Hz, 7.8 Hz), 7.58 (dd, 1H, J = 1.3 Hz, 7.8 Hz), 7.29 (ddd, 1H, J = 1.3 Hz, 7.8 Hz, 7.8 Hz), 7.10 (ddd, 1H, J = 1.3 Hz, 7.8 Hz, 7.8 Hz), 4.56 (q, 2H, J = 2.5 Hz), 4.36 (s, 2H), 1.84 (t, 3H, J = 2.5 Hz)

### Step 2: Synthesis of compound 11

Operations were carried out in the same manner as in Synthesis Example 4 with the exception that in step 4 of Synthesis Example 4, 4.05 g (16.22 mmol) of the intermediate H was changed to 3.52 g (16.22 mmol) of the intermediate S synthesized in step 1. As a result, 9.46 g (yield: 66.6%) of a solid (crude compound 11) was obtained.

Through analysis of the obtained solid by HPLC, it was confirmed that the solid contained the following compound 11' (halogenated compound of compound 11) in a proportion of 0.9 mass% among the total of the compound 11 and the compound 11'.

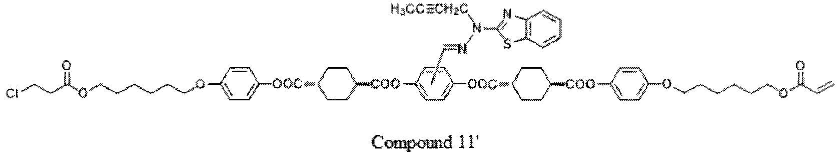

### (Synthesis Example 12) Synthesis of compound 12

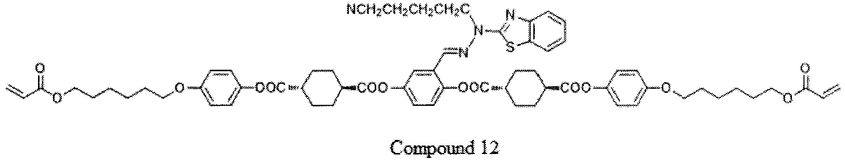

### Step 1: Synthesis of intermediate T

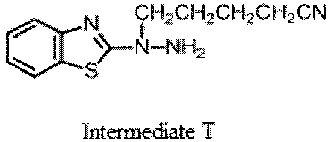

In a four-necked reaction vessel equipped with a thermometer, 10.0 g (60.5 mmol) of 2-hydrazinobenzothiazole was dissolved in 100 mL of DMF in a stream of nitrogen. Next, 41.8 g (304 mmol) of potassium carbonate and 10.34 g (60.06 mmol) of 5-bromovaleronitrile were added to the solution, and the entire contents of the reaction vessel were stirred for 8 hours at 60°C. Once the reaction ended, the reaction liquid was cooled to 20°C and was added into 1,000 mL of water, and then extraction was performed with 1,000 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (n-hexane:ethyl acetate = 60:40) to yield 6.82 g (yield: 45.7%) of intermediate T in the form of a white solid.

The structure of the target was identified by ¹H-NMR.

¹H-NMR (400 MHz, CDCl₃, TMS, δ ppm): 7.60 (d, 1H, J = 7.8 Hz), 7.51 (d, 1H, J = 8.1 Hz), 7.28 (dd, 1H, J = 7.3 Hz, 8.1 Hz), 7.07 (dd, 1H, J = 7.3 Hz, 7.8 Hz), 4.23 (s, 2H), 3.81 (t, 2H, J = 6.9 Hz), 2.46 (t, 2H, J = 7.1 Hz), 1.88-1.95 (m, 2H), 1.71-1.79 (m, 2H)

### Step 2: Synthesis of compound 12

Operations were carried out in the same manner as in Synthesis Example 4 with the exception that in step 4 of Synthesis Example 4, 4.05 g (16.22 mmol) of the intermediate H was changed to 4.00 g (16.22 mmol) of the intermediate T synthesized in step 1. As a result, 11.23 g (yield: 77.1%) of a solid (crude compound 12) was obtained.

Through analysis of the obtained solid by HPLC, it was confirmed that the solid contained the following compound 12' (halogenated compound of compound 12) in a proportion of 0.3 mass% among the total of the compound 12 and the compound 12'.

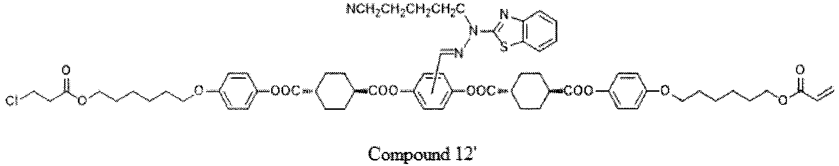

### (Synthesis Example 13) Synthesis of compound 13

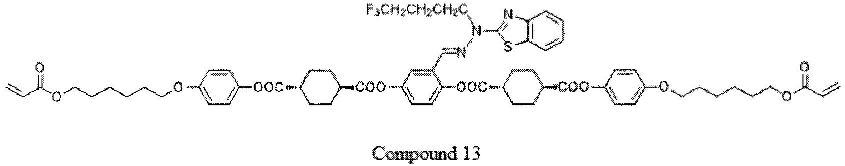

### Step 1: Synthesis of intermediate U

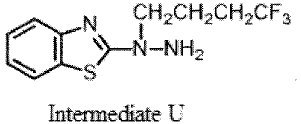

In a four-necked reaction vessel equipped with a thermometer, 14.5 g (87.5 mmol) of 2-hydrazinobenzothiazole was dissolved in 200 mL of DMF in a stream of nitrogen. Next, 36.3 g (263 mmol) of potassium carbonate and 25.0 g (105 mmol) of 1,1,1-trifluoro-4-iodobutane were added to the solution, and the entire contents of the reaction vessel were stirred for 8 hours at 80°C. Once the reaction ended, the reaction liquid was cooled to 20°C and was added into 1,000 mL of water, and then extraction was performed with 1,000 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (n-hexane:ethyl acetate = 85:15) to yield 9.61 g (yield: 39.9%) of intermediate U in the form of a white solid.

The structure of the target was identified by ¹H-NMR.

¹H-NMR (500 MHz, CDCl₃, TMS, δ ppm): 7.61 (d, 1H, J = 8.0 Hz), 7.54 (d, 1H, J = 7.8 Hz), 7.30 (dd, 1H, J = 7.8 Hz, 7.8 Hz), 7.09 (dd, 1H, J = 7.8 Hz, 8.0 Hz), 4.24 (s, 2H), 3.81 (t, 2H, J = 7.0 Hz), 2.16-2.26 (m, 2H), 1.99-2.05 (m, 2H)

### Step 2: Synthesis of compound 13

Operations were carried out in the same manner as in Synthesis Example 4 with the exception that in step 4 of Synthesis Example 4, 4.05 g (16.22 mmol) of the intermediate H was changed to 4.47 g (16.22 mmol) of the intermediate U synthesized in step 1. As a result, 11.81 g (yield: 79.1%) of a solid (crude compound 13) was obtained.

Through analysis of the obtained solid by HPLC, it was confirmed that the solid contained the following compound 13' (halogenated compound of compound 13) in a proportion of 1.1 mass% among the total of the compound 13 and the compound 13'.

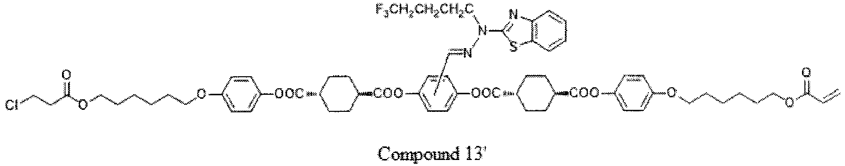

### (Synthesis Example 14) Synthesis of compound 14

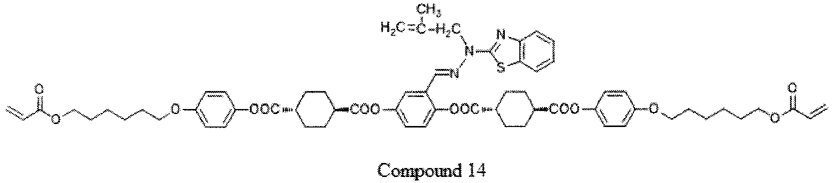

### Step 1: Synthesis of intermediate V

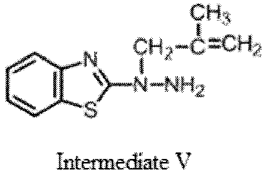

In a four-necked reaction vessel equipped with a thermometer, 40.0 g (241.6 mmol) of 2-hydrazinobenzothiazole was dissolved in 300 mL of DMF in a stream of nitrogen. Next, 118 g (363 mmol) of cesium carbonate and 39.2 g (291 mmol) of 3-bromo-2-methyl-1-propene were added to the solution, and the entire contents of the reaction vessel were stirred for 18 hours at 25°C. Once the reaction ended, the reaction liquid was added into 1,500 mL of water, and extraction was performed with 2,000 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a yellow solid. The yellow solid was purified by silica gel column chromatography (hexane:ethyl acetate = 80:20) to yield 5.88 g (yield: 11.1%) of intermediate V in the form of a white solid.

The structure of the target was identified by ¹H-NMR.

¹H-NMR (500 MHz, CDCl₃, TMS, δ ppm): 7.59 (dd, 1H, J = 1.0 Hz, 8.0 Hz), 7.52 (dd, 1H, J = 1.5 Hz, 8.0 Hz), 7.26 (ddd, 1H, J = 1.0 Hz, 7.5 Hz, 8.0 Hz), 7.05 (ddd, 1H, J = 1.5 Hz, 7.5 Hz, 8.0 Hz), 4.98 (s, 1H), 4.86 (s, 1H), 4.29 (s, 2H), 4.12 (s, 2H), 1.71 (s, 3H)

### Step 2: Synthesis of compound 14

Operations were carried out in the same manner as in Synthesis Example 4 with the exception that in step 4 of Synthesis Example 4, 4.05 g (16.22 mmol) of the intermediate H was changed to 3.56 g (16.22 mmol) of the intermediate V synthesized in step 1. As a result, 10.05 g (yield: 70.6%) of a solid (crude compound 14) was obtained.

Through analysis of the obtained solid by HPLC, it was confirmed that the solid contained the following compound 14' (halogenated compound of compound 14) in a proportion of 1.3 mass% among the total of the compound 14 and the compound 14'.

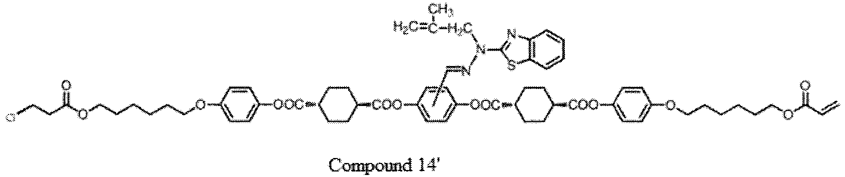

### (Synthesis Example 15) Synthesis of compound 15

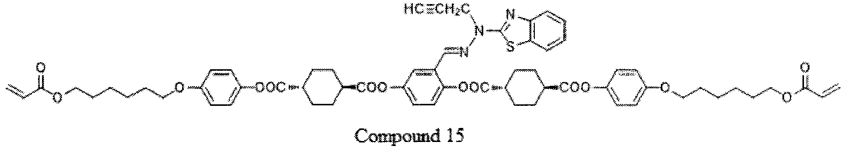

### Step 1: Synthesis of intermediate W

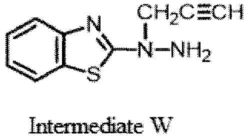

In a three-necked reaction vessel equipped with a thermometer, 20.0 g (121.1 mmol) of 2-hydrazinobenzothiazole was dissolved in 400 mL of DMF in a stream of nitrogen. Next, 78.9 g (242.1 mmol) of cesium carbonate and 17.3 g (145.3 mmol) of propargyl bromide were added to the solution, and the entire contents of the reaction vessel were stirred for 15 hours at 25°C. Once the reaction ended, 1,500 mL of distilled water was added to the reaction liquid, and extraction was performed twice with 1,000 mL of ethyl acetate. The organic layers were dried with anhydrous sodium sulfate, and then sodium sulfate was filtered off. The filtrate was concentrated using a rotary evaporator to obtain a concentrate that was subsequently purified by silica gel column chromatography (THF:toluene = 1:19) to yield 6.90 g (yield: 28%) of intermediate W in the form of a pale yellow solid.

The structure of the target was identified by ¹H-NMR.

¹H-NMR (500 MHz, DMSO-d₆, TMS, δ ppm): 7.73 (dd, 1H, J = 1.0 Hz, 8.0 Hz), 7.44 (dd, 1H, J = 1.0 Hz, 8.0 Hz), 7.26 (dt, 1H, J = 1.0 Hz, 7.5 Hz), 7.06 (dt, 1H, J = 1.0 Hz, 7.5 Hz), 5.31 (s, 2H), 4.52 (d, 2H, J = 2.5 Hz), 3.35 (t, 1H, J = 2.5 Hz)

### Step 2: Synthesis of compound 15

Operations were carried out in the same manner as in Synthesis Example 4 with the exception that in step 4 of Synthesis Example 4, 4.05 g (16.22 mmol) of the intermediate H was changed to 3.30 g (16.22 mmol) of the intermediate W synthesized in step 1. As a result, 10.12 g (yield: 72.1%) of a solid (crude compound 15) was obtained.

Through analysis of the obtained solid by HPLC, it was confirmed that the solid contained the following compound 15' (halogenated compound of compound 15) in a proportion of 1.8 mass% among the total of the compound 15 and the compound 15'.

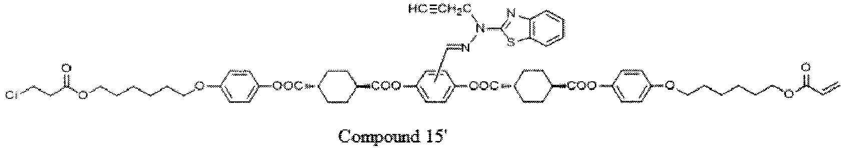

### (Synthesis Example 16) Synthesis of compound 16

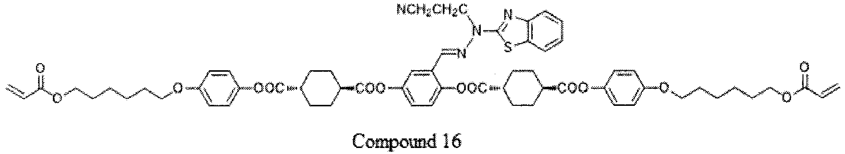

### Step 1: Synthesis of intermediate X

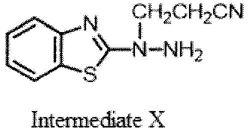

In a three-necked reaction vessel equipped with a thermometer, 20.0 g (121.1 mmol) of 2-hydrazinobenzothiazole was dissolved in 400 mL of DMF in a stream of nitrogen. Next, 78.9 g (242.1 mol) of cesium carbonate and 19.5 g (145.3 mmol) of 3-bromopropionitrile were added to the solution, and the entire contents of the reaction vessel were stirred for 15 hours at 25°C. Once the reaction ended, 1,500 mL of distilled water was added to the reaction liquid, and extraction was performed twice with 1,000 mL of ethyl acetate. The organic layers were dried with anhydrous sodium sulfate, and then sodium sulfate was filtered off. The filtrate was concentrated using a rotary evaporator, 200 mL of toluene was added to the concentrate, and then cooling was performed to 0°C. Precipitated crystals were collected by filtration and were vacuum dried to yield 11.2 g (yield: 42%) of intermediate X in the form of a white solid.

The structure of the target was identified by ¹H-NMR.

¹H-NMR (500 MHz, DMSO-d₆, TMS, δ ppm): 7.70 (dd, 1H, J = 1.0 Hz, 8.0 Hz), 7.42 (dd, 1H, J = 1.0 Hz, 8.0 Hz), 7.24 (dt, 1H, J = 1.0 Hz, 7.5 Hz), 7.03 (dt, 1H, J = 1.0 Hz, 7.5 Hz), 5.47 (s, 2H), 3.99 (t, 2H, J = 6.5 Hz), 2.97 (t, 2H, J = 6.5 Hz)

### Step 2: Synthesis of compound 16

Operations were carried out in the same manner as in Synthesis Example 4 with the exception that in step 4 of Synthesis Example 4, 4.05 g (16.22 mmol) of the intermediate H was changed to 3.54 g (16.22 mmol) of the intermediate X synthesized in step 1. As a result, 10.22 g (yield: 71.9%) of a solid (crude compound 16) was obtained.

Through analysis of the obtained solid by HPLC, it was confirmed that the solid contained the following compound 16' (halogenated compound of compound 16) in a proportion of 0.7 mass% among the total of the compound 16 and the compound 16'.

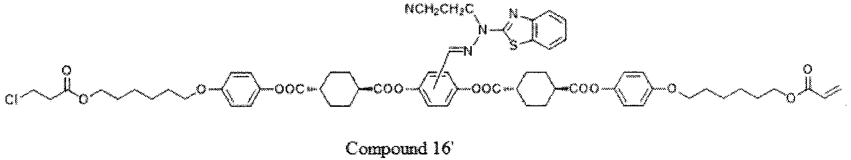

### (Synthesis Example 17) Synthesis of compound 17

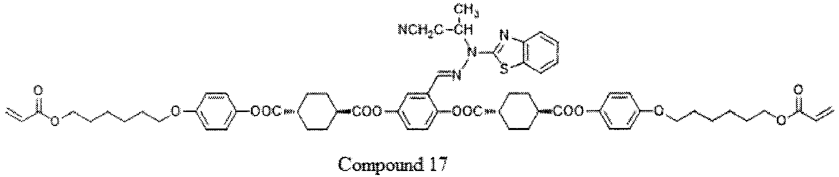

### Step 1: Synthesis of intermediate Y

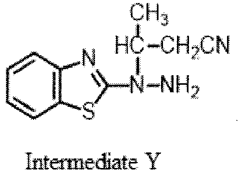

In a three-necked reaction vessel equipped with a thermometer, 10.0 g (60.5 mmol) of 2-hydrazinobenzothiazole was dissolved in 200 mL of DMF in a stream of nitrogen. Next, 39.5 g (121 mmol) of cesium carbonate and 10.8 g (72.7 mmol) of 3-bromobutyronitrile were added to the solution, and the entire contents of the reaction vessel were stirred for 15 hours at 25°C. Once the reaction ended, 1,000 mL of distilled water was added to the reaction liquid, and extraction was performed twice with 500 mL of ethyl acetate. The organic layers were dried with anhydrous sodium sulfate, and then sodium sulfate was filtered off. The filtrate was concentrated using a rotary evaporator to obtain a concentrate that was subsequently purified by silica gel column chromatography (THF:toluene = 1:9) to yield 10.2 g (yield: 72%) of intermediate Y in the form of a white solid.

The structure of the target was identified by ¹H-NMR.

¹H-NMR (500 MHz, DMSO-d₆, TMS, δ ppm): 7.70 (dd, 1H, J = 1.0 Hz, 8.0 Hz), 7.42 (dd, 1H, J = 1.0 Hz, 8.0 Hz), 7.24 (dt, 1H, J = 1.0 Hz, 7.5 Hz), 7.03 (dt, 1H, J = 1.0 Hz, 7.5 Hz), 5.47 (s, 2H), 3.99 (t, 2H, J = 6.5 Hz), 2.97 (t, 2H, J = 6.5 Hz)

### Step 2: Synthesis of compound 17

Operations were carried out in the same manner as in Synthesis Example 4 with the exception that in step 4 of Synthesis Example 4, 4.05 g (16.22 mmol) of the intermediate H was changed to 3.77 g (16.22 mmol) of the intermediate Y synthesized in step 1. As a result, 9.47 g (yield: 65.8%) of a solid (crude compound 17) was obtained.

Through analysis of the obtained solid by HPLC, it was confirmed that the solid contained the following compound 17' (halogenated compound of compound 17) in a proportion of 0.4 mass% among the total of the compound 17 and the compound 17'.

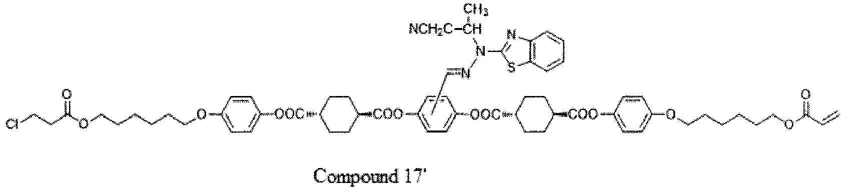

### (Synthesis Example 18) Synthesis of compound 18

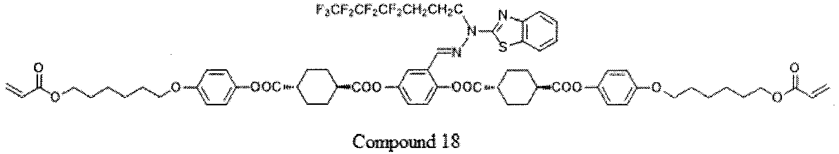

### Step 1: Synthesis of intermediate Z

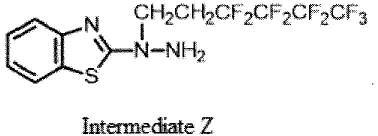

In a four-necked reaction vessel equipped with a thermometer, 20.0 g (121 mmol) of 2-hydrazinobenzothiazole was dissolved in 300 mL of DMF in a stream of nitrogen. Next, 78.9 g (242 mmol) of cesium carbonate and 50.0 g (145 mmol) of 2-(nonafluorobutyl)ethyl iodide were added to the solution, and the entire contents of the reaction vessel were stirred for 20 hours at 25°C. Once the reaction ended, the reaction liquid was added into 1,000 mL of water, and extraction was performed with 1,000 mL of ethyl acetate. The ethyl acetate layer was dried with anhydrous sodium sulfate, and then sodium sulfate was filtered off. Ethyl acetate was evaporated from the filtrate under reduced pressure in a rotary evaporator to yield a brown solid. The brown solid was purified by silica gel column chromatography (n-hexane:ethyl acetate = 9:1) to yield 11.5 g (yield: 22.9%) of intermediate Z in the form of a white solid.

The structure of the target was identified by ¹H-NMR.

¹H-NMR (500 MHz, CDCl₃, TMS, δ ppm): 7.63 (dd, 1H, J = 1.0 Hz, 7.5 Hz), 7.57 (dd, 1H, J = 1.0 Hz, 7.5 Hz), 7.32 (ddd, 1H, J = 1.0 Hz, 7.5 Hz, 7.5 Hz), 7.11 (ddd, 1H, J = 1.0 Hz, 7.5 Hz, 7.5 Hz), 4.35 (s, 2H), 4.08 (t, 2H, J = 7.5 Hz), 2.56-2.70 (m, 2H)

### Step 2: Synthesis of compound 18

Operations were carried out in the same manner as in Synthesis Example 4 with the exception that in step 4 of Synthesis Example 4, 4.05 g (16.22 mmol) of the intermediate H was changed to 6.67 g (16.22 mmol) of the intermediate Z synthesized in step 1. As a result, 10.34 g (yield: 62.2%) of a solid (crude compound 18) was obtained.

Through analysis of the obtained solid by HPLC, it was confirmed that the solid contained the following compound 18' (halogenated compound of compound 18) in a proportion of 0.2 mass% among the total of the compound 18 and the compound 18'.

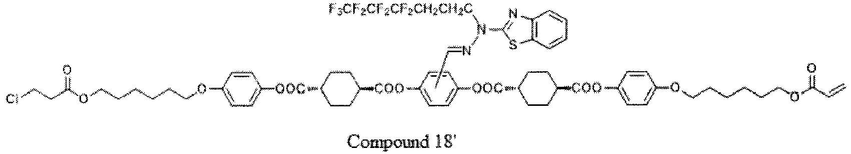

### (Synthesis Example 19) Synthesis of compound 19

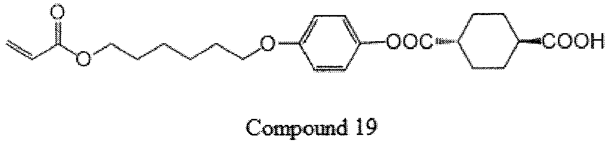

In a three-necked reaction vessel equipped with a condenser and a thermometer, 4.15 g (19.87 mmol) of trans-cyclohexanedicarboxylic acid dichloride was dissolved in 30 g of cyclopentyl methyl ether and 11.5 g of tetrahydrofuran in a stream of nitrogen. The solution was cooled in an ice bath, and then 5.0 g of the crude intermediate AA obtained in step 2 of the preceding Synthesis Example 4 was added to the solution and dissolved. Next, 2.01 g (19.87 mmol) of triethylamine was controlled to 10°C or lower and was slowly added dropwise to the solution in the ice bath. After completion of the dropwise addition, the entire contents of the reaction vessel were returned to 25°C and were further stirred for 1 hour. Next, 80 mL of distilled water was added to the obtained reaction liquid. Washing was performed for 4 hours at 50°C, and then the aqueous layer was removed. The organic layer was further washed five times with 150 mL of a buffer solution (pH 5.5) containing acetic acid in a concentration of 1.0 mol/L and sodium acetate, and then the buffer solution was removed. The organic layer was further washed with 100 mL of distilled water and was then subjected to liquid separation. Crystals were caused to precipitate by adding 400 mL of n-hexane to the resultant organic layer and were subsequently collected by filtration. The obtained crystals were purified by silica gel column chromatography (toluene:ethyl acetate = 70:30) to yield 3.56 g (yield: 45%) of a white solid (crude compound 19) containing compound 19.

Through analysis of the obtained solid by HPLC, it was confirmed that the solid contained the following compound 19' (halogenated compound of compound 19) in a proportion of 1.8 mass% among the total of the compound 19 and the compound 19'.

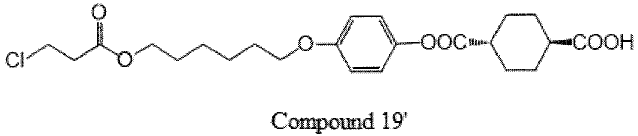

### (Synthesis Example 20) Synthesis of mixture 1

In a three-necked reaction vessel equipped with a condenser and a thermometer, 4.15 g (19.87 mmol) of trans-cyclohexanedicarboxylic acid dichloride was dissolved in 30 g of cyclopentyl methyl ether and 11.5 g of tetrahydrofuran in a stream of nitrogen. The solution was cooled in an ice bath, and then 5.0 g of the crude intermediate AA obtained in step 2 of the preceding Synthesis Example 4 was added to the solution and dissolved. Next, 2.01 g (19.87 mmol) of triethylamine was controlled to 10°C or lower and was slowly added dropwise to the solution in the ice bath. After completion of the dropwise addition, the entire contents of the reaction vessel were returned to 25°C and were further stirred for 1 hour. Next, 15 mL of distilled water was added to the obtained reaction liquid. Washing was performed for 4 hours at 50°C, and then the aqueous layer was removed. The organic layer was further washed five times with 25 g of a buffer solution (pH 5.5) containing acetic acid in a concentration of 1.0 mol/L and sodium acetate, and then the buffer solution was removed. The organic layer was further washed with 15 mL of distilled water and was then subjected to liquid separation. Crystals were caused to precipitate by adding 60 g of 60% hexane to the resultant organic layer. The resultant solution was cooled to 0°C and was stirred for 1 hour. Thereafter, the precipitated crystals were collected by filtration to yield 7.25 g of a mixture 1. The obtained solid was found to contain 5.5 g of the compound 19 and 1.74 g of a diester compound through quantitative analysis by HPLC. Moreover, through compositional analysis of the obtained solid by HPLC, it was confirmed that the solid contained the compound 19' (halogenated compound of compound 19) in a proportion of 1.5 mass% among the total of the compound 19 and the compound 19'.

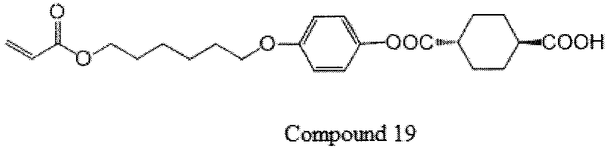

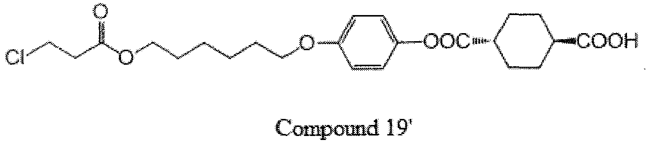

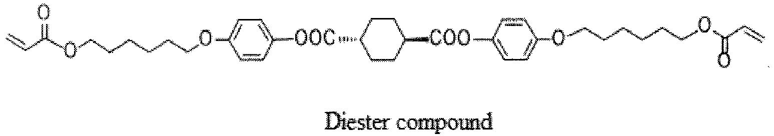

### (Example 1) Dehydrochlorination reaction of compound 1

In a four-necked reaction vessel equipped with a thermometer, 1.0 g (0.83 mmol) of the compound 1 and 126 mg (1.24 mmol) of triethylamine were dissolved in a mixed solvent of 30 mL of ethyl acetate and 15 mL of acetonitrile in a stream of nitrogen. Next, 1.5 mL of sodium carbonate aqueous solution having a concentration of 1 mol/L was added to the resultant solution, and then the solution was stirred for 4 hours at 50°C. Sodium carbonate aqueous solution was removed once the reaction ended, and the resultant organic layer was washed with 30 mL of water. Solid was caused to precipitate by adding 70 mL of methanol to the organic layer. The obtained solid was dried using a vacuum dryer to yield 0.95 g of a pale yellow solid.

Through analysis of the obtained solid by HPLC, it was confirmed that the compound 1 (halogenated compound) had been converted to the compound 4 since a peak attributed to the compound 1 had completely disappeared.

The structure of the target was identified by ¹H-NMR.

¹H-NMR (400 MHz, CDCl₃, TMS, δ ppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t, 4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz)

### (Example 2) Dehydrochlorination reaction of compound 2

Operations were carried out in the same manner as in Example 1 with the exception that in the operations of Example 1, 1.0 g (0.83 mmol) of the compound 1 was changed to 1.0 g (0.83 mmol) of the compound 2 synthesized in Synthesis Example 2. As a result, 0.94 g of a pale yellow solid was obtained.

Through analysis of the obtained solid by HPLC, it was confirmed that the compound 2 (halogenated compound) had been converted to the compound 4 since a peak attributed to the compound 2 had completely disappeared.

The structure of the target was identified by ¹H-NMR.

¹H-NMR (400 MHz, CDCl₃, TMS, δ ppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t, 4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz)

### (Example 3) Dehydrochlorination reaction of compound 3

Operations were carried out in the same manner as in Example 1 with the exception that in the operations of Example 1, 1.0 g (0.83 mmol) of the compound 1 was changed to 1.0 g (0.80 mmol) of the compound 3 synthesized in Synthesis Example 3, the amount of triethylamine that was used was changed from 126 mg (1.24 mmol) to 250 mg (2.47 mmol), and the amount of sodium carbonate aqueous solution of 1 mol/L in concentration that was used was changed from 1.5 mL to 3 mL. As a result, 0.92 g of a pale yellow solid was obtained.

Through analysis of the obtained solid by HPLC, it was confirmed that the compound 3 (halogenated compound) had been converted to the compound 4 since a peak attributed to the compound 3 had completely disappeared.

The structure of the target was identified by ¹H-NMR.

¹H-NMR (400 MHz, CDCl₃, TMS, δ ppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t, 4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz)

### (Example 4) Dehydrochlorination reaction of crude compound 4

Operations were carried out in the same manner as in Example 1 with the exception that in the operations of Example 1, 1.0 g (0.83 mmol) of the compound 1 was changed to 1.0 g of the crude compound 4 synthesized in Synthesis Example 4. As a result, 0.95 g of a pale yellow solid was obtained.

Through analysis of the obtained solid by HPLC, it was confirmed that the compound 4' (halogenated compound) had been converted to the compound 4 since a peak attributed to the compound 4' had completely disappeared.

### (Example 5) Dehydrochlorination reaction of crude compound 5

Operations were carried out in the same manner as in Example 1 with the exception that in the operations of Example 1, 1.0 g (0.83 mmol) of the compound 1 was changed to 1.0 g of the crude compound 5 synthesized in Synthesis Example 5. As a result, 0.94 g of a pale yellow solid was obtained. Through analysis of the obtained solid by HPLC, it was confirmed that the compound 5' (halogenated compound) had been converted to the compound 5 since a peak attributed to the compound 5' had completely disappeared.

### (Example 6) Dehydrochlorination reaction of crude compound 6

Operations were carried out in the same manner as in Example 1 with the exception that in the operations of Example 1, 1.0 g (0.83 mmol) of the compound 1 was changed to 1.0 g of the crude compound 6 synthesized in Synthesis Example 6. As a result, 0.94 g of a pale yellow solid was obtained. Through analysis of the obtained solid by HPLC, it was confirmed that the compound 6' (halogenated compound) had completely disappeared and had all been converted to the compound 6.

### (Example 7) Dehydrochlorination reaction of crude compound 7

Operations were carried out in the same manner as in Example 1 with the exception that in the operations of Example 1, 1.0 g (0.83 mmol) of the compound 1 was changed to 1.0 g of the crude compound 7 synthesized in Synthesis Example 7. As a result, 0.94 g of a pale yellow solid was obtained. Through analysis of the obtained solid by HPLC, it was confirmed that the compound 7' (halogenated compound) had completely disappeared and had all been converted to the compound 7.

### (Example 8) Dehydrochlorination reaction of crude compound 8

Operations were carried out in the same manner as in Example 1 with the exception that in the operations of Example 1, 1.0 g (0.83 mmol) of the compound 1 was changed to 1.0 g of the crude compound 8 synthesized in Synthesis Example 8. As a result, 0.94 g of a pale yellow solid was obtained. Through analysis of the obtained solid by HPLC, it was confirmed that the compound 8' (halogenated compound) had completely disappeared and had all been converted to the compound 8.

### (Example 9) Dehydrochlorination reaction of crude compound 9

Operations were carried out in the same manner as in Example 1 with the exception that in the operations of Example 1, 1.0 g (0.83 mmol) of the compound 1 was changed to 1.0 g of the crude compound 9 synthesized in Synthesis Example 9. As a result, 0.94 g of a pale yellow solid was obtained. Through analysis of the obtained solid by HPLC, it was confirmed that the compound 9' (halogenated compound) had completely disappeared and had all been converted to the compound 9.

### (Example 10) Dehydrochlorination reaction of crude compound 10

Operations were carried out in the same manner as in Example 1 with the exception that in the operations of Example 1, 1.0 g (0.83 mmol) of the compound 1 was changed to 1.0 g of the crude compound 10 synthesized in Synthesis Example 10. As a result, 0.94 g of a pale yellow solid was obtained. Through analysis of the obtained solid by HPLC, it was confirmed that the compound 10' (halogenated compound) had completely disappeared and had all been converted to the compound 10.

### (Example 11) Dehydrochlorination reaction of crude compound 11

Operations were carried out in the same manner as in Example 1 with the exception that in the operations of Example 1, 1.0 g (0.83 mmol) of the compound 1 was changed to 1.0 g of the crude compound 11 synthesized in Synthesis Example 11. As a result, 0.94 g of a pale yellow solid was obtained. Through analysis of the obtained solid by HPLC, it was confirmed that the compound 11' (halogenated compound) had completely disappeared and had all been converted to the compound 11.

### (Example 12) Dehydrochlorination reaction of crude compound 12

Operations were carried out in the same manner as in Example 1 with the exception that in the operations of Example 1, 1.0 g (0.83 mmol) of the compound 1 was changed to 1.0 g of the crude compound 12 synthesized in Synthesis Example 12. As a result, 0.94 g of a pale yellow solid was obtained. Through analysis of the obtained solid by HPLC, it was confirmed that the compound 12' (halogenated compound) had completely disappeared and had all been converted to the compound 12.

### (Example 13) Dehydrochlorination reaction of crude compound 13

Operations were carried out in the same manner as in Example 1 with the exception that in the operations of Example 1, 1.0 g (0.83 mmol) of the compound 1 was changed to 1.0 g of the crude compound 13 synthesized in Synthesis Example 13. As a result, 0.94 g of a pale yellow solid was obtained. Through analysis of the obtained solid by HPLC, it was confirmed that the compound 13' (halogenated compound) had completely disappeared and had all been converted to the compound 13.

### (Example 14) Dehydrochlorination reaction of crude compound 14

Operations were carried out in the same manner as in Example 1 with the exception that in the operations of Example 1, 1.0 g (0.83 mmol) of the compound 1 was changed to 1.0 g of the crude compound 14 synthesized in Synthesis Example 14. As a result, 0.94 g of a pale yellow solid was obtained. Through analysis of the obtained solid by HPLC, it was confirmed that the compound 14' (halogenated compound) had completely disappeared and had all been converted to the compound 14.

### (Example 15) Dehydrochlorination reaction of crude compound 15

Operations were carried out in the same manner as in Example 1 with the exception that in the operations of Example 1, 1.0 g (0.83 mmol) of the compound 1 was changed to 1.0 g of the crude compound 15 synthesized in Synthesis Example 15. As a result, 0.94 g of a pale yellow solid was obtained. Through analysis of the obtained solid by HPLC, it was confirmed that the compound 15' (halogenated compound) had completely disappeared and had all been converted to the compound 15.

### (Example 16) Dehydrochlorination reaction of crude compound 16

Operations were carried out in the same manner as in Example 1 with the exception that in the operations of Example 1, 1.0 g (0.83 mmol) of the compound 1 was changed to 1.0 g of the crude compound 16 synthesized in Synthesis Example 16. As a result, 0.94 g of a pale yellow solid was obtained. Through analysis of the obtained solid by HPLC, it was confirmed that the compound 16' (halogenated compound) had completely disappeared and had all been converted to the compound 16.

### (Example 17) Dehydrochlorination reaction of crude compound 17

Operations were carried out in the same manner as in Example 1 with the exception that in the operations of Example 1, 1.0 g (0.83 mmol) of the compound 1 was changed to 1.0 g of the crude compound 17 synthesized in Synthesis Example 17. As a result, 0.94 g of a pale yellow solid was obtained. Through analysis of the obtained solid by HPLC, it was confirmed that the compound 17' (halogenated compound) had completely disappeared and had all been converted to the compound 17.

### (Example 18) Dehydrochlorination reaction of crude compound 18

Operations were carried out in the same manner as in Example 1 with the exception that in the operations of Example 1, 1.0 g (0.83 mmol) of the compound 1 was changed to 1.0 g of the crude compound 18 synthesized in Synthesis Example 18. As a result, 0.94 g of a pale yellow solid was obtained. Through analysis of the obtained solid by HPLC, it was confirmed that the compound 18' (halogenated compound) had completely disappeared and had all been converted to the compound 18.

### (Example 19) Dehydrochlorination reaction of intermediate D

In a four-necked reaction vessel equipped with a thermometer, 1.0 g (3.32 mmol) of the intermediate D synthesized in step 3 of the preceding Synthesis Example 1 and 505 mg (4.99 mmol) of triethylamine were dissolved in a mixed solvent of 40 mL of ethyl acetate and 20 mL of acetonitrile in a stream of nitrogen. Next, 9.0 mL of sodium carbonate aqueous solution having a concentration of 1 mol/L was added to the resultant solution, and then the solution was stirred for 4 hours at 50°C. The sodium carbonate aqueous solution was removed once the reaction ended, and the resultant organic layer was further washed with 20 mL of 0.5 N hydrochloric acid aqueous solution. Next, washing was performed twice with 50 mL of distilled water. Solid was caused to precipitate by adding 200 mL of n-hexane to the resultant ethyl acetate layer. The solid was collected by filtration and was dried using a vacuum dryer to yield 0.77 g of a white solid. Through analysis of the obtained solid by HPLC, it was confirmed that the intermediate D (halogenated compound) had been converted to the intermediate AA since a peak attributed to the intermediate D had completely disappeared.

### (Example 20) Dehydrochlorination reaction of intermediate E

In a four-necked reaction vessel equipped with a thermometer, 1.0 g (2.20 mmol) of the intermediate E synthesized in step 4 of the preceding Synthesis Example 1 and 334 mg (3.30 mmol) of triethylamine were dissolved in a mixed solvent of 40 mL of ethyl acetate and 20 mL of acetonitrile in a stream of nitrogen. Next, 8.0 mL of sodium carbonate aqueous solution having a concentration of 1 mol/L was added to the resultant solution, and then the solution was stirred for 4 hours at 50°C. The sodium carbonate aqueous solution was removed once the reaction ended, and the resultant organic layer was further washed with 20 mL of 0.5 N hydrochloric acid aqueous solution. Next, washing was performed twice with 50 mL of distilled water. Solid was caused to precipitate by adding 200 mL of n-hexane to the resultant ethyl acetate layer. The solid was collected by filtration and was dried using a vacuum dryer to yield 0.82 g of a white solid. Through analysis of the obtained solid by HPLC, it was confirmed that the intermediate E (halogenated compound) had been converted to the compound 19 since a peak attributed to the intermediate E had completely disappeared.

### (Example 21) Dehydrochlorination reaction of crude intermediate AA

In a four-necked reaction vessel equipped with a thermometer, 1.0 g of the crude intermediate AA synthesized in step 2 of the preceding Synthesis Example 4 and 505 mg (4.99 mmol) of triethylamine were dissolved in a mixed solvent of 40 mL of ethyl acetate and 20 mL of acetonitrile in a stream of nitrogen. Next, 9.0 mL of sodium carbonate aqueous solution having a concentration of 1 mol/L was added to the resultant solution, and then the solution was stirred for 4 hours at 50°C. The sodium carbonate aqueous solution was removed once the reaction ended, and the resultant organic layer was further washed with 20 mL of 0.5 N hydrochloric acid aqueous solution. Next, washing was performed twice with 50 mL of distilled water. Solid was caused to precipitate by adding 200 mL of n-hexane to the resultant ethyl acetate layer. The solid was collected by filtration and was dried using a vacuum dryer to yield 0.92 g of a white solid. Through analysis of the obtained solid by HPLC, it was confirmed that the intermediate AA' (halogenated compound) had been converted to the intermediate AA since a peak attributed to the intermediate AA' had completely disappeared.

### (Example 22) Dehydrochlorination reaction of crude compound 19

In a four-necked reaction vessel equipped with a thermometer, 1.0 g (2.20 mmol) of the crude compound 19 synthesized in the preceding Synthesis Example 19 and 334 mg (3.30 mmol) of triethylamine were dissolved in a mixed solvent of 40 mL of ethyl acetate and 20 mL of acetonitrile in a stream of nitrogen. Next, 8.0 mL of sodium carbonate aqueous solution having a concentration of 1 mol/L was added to the resultant solution, and then the solution was stirred for 4 hours at 50°C. The sodium carbonate aqueous solution was removed once the reaction ended, and the resultant organic layer was further washed with 20 mL of 0.5 N hydrochloric acid aqueous solution. Next, washing was performed twice with 50 mL of distilled water. Solid was caused to precipitate by adding 200 mL of n-hexane to the resultant ethyl acetate layer. The solid was collected by filtration and was dried using a vacuum dryer to yield 0.89 g of a white solid. Through analysis of the obtained solid by HPLC, it was confirmed that the compound 19' (halogenated compound) had been converted to the compound 19 since a peak attributed to the compound 19' had completely disappeared.

### (Example 23) Dehydrochlorination reaction of mixture 1

In a four-necked reaction vessel equipped with a thermometer, 7.25 g of the mixture 1 synthesized in the preceding Synthesis Example 20 and 2.0 g (19.71 mmol) of triethylamine were dissolved in a mixed solvent of 200 mL of ethyl acetate and 100 mL of acetonitrile in a stream of nitrogen. Next, 50 mL of sodium carbonate aqueous solution having a concentration of 1 mol/L was added to the resultant solution, and then the solution was stirred for 4 hours at 50°C. The sodium carbonate aqueous solution was removed once the reaction ended, and the resultant organic layer was further washed with 110 mL of 0.5 N hydrochloric acid aqueous solution. Next, washing was performed twice with 100 mL of distilled water. The resultant ethyl acetate layer was concentrated to 100 mL in a rotary evaporator. Solid was caused to precipitate by adding 500 mL of n-hexane to the resultant ethyl acetate layer. The solid was collected by filtration and was dried using a vacuum dryer to yield 6.58 g of a white solid. Through analysis of the obtained solid by HPLC, it was confirmed that the compound 19' (halogenated compound) had been converted to the compound 19 since a peak attributed to the compound 19' had completely disappeared.

### INDUSTRIAL APPLICABILITY

According to the present disclosure, it is possible to provide a method of producing a high-purity polymerizable compound in an industrially advantageous manner.

Moreover, according to the present disclosure, it is possible to provide a halogenated compound and a mixture containing the halogenated compound that are useful in the method of producing a polymerizable compound.

## Claims

1. A method of producing a polymerizable compound indicated by formula (I), shown below, 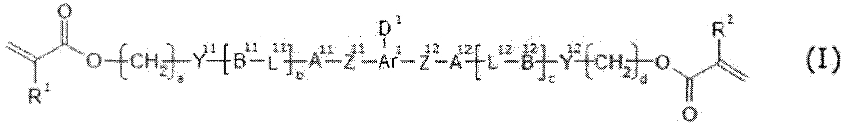 where, in formula (I):
Ar¹ represents a divalent aromatic hydrocarbon cyclic group having D¹ as a substituent or a divalent aromatic heterocyclic group having D¹ as a substituent;
D¹ represents an organic group having a carbon number of 1 to 20 and including at least one aromatic ring selected from the group consisting of an aromatic hydrocarbon ring and an aromatic heterocyclic ring;
Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
A¹¹, A¹², B¹¹, and B¹² each represent, independently of one another, an optionally substituted alicyclic group or an optionally substituted aromatic group;
Y¹¹, Y¹², L¹¹, and L¹² each represent, independently of one another, a single bond, -O-, -CO-, -CO-O-, -O-CO-, -NR²¹-CO-, -CO-NR²²-, -O-CO-O-, -NR²³-CO-O-, -O-CO-NR²⁴-, or -NR²⁵-CO-NR²⁶-, where R²¹ to R²⁶ each represent, independently of one another, a hydrogen atom or an alkyl group having a carbon number of 1 to 6;
R¹ and R² each represent, independently of one another, a hydrogen atom or a methyl group;
a and d each represent, independently of one another, an integer of 1 to 20; and
b and c are each, independently of one another, 0 or 1,
the method comprising subjecting a composition containing a halogenated compound indicated by formula (II), shown below, 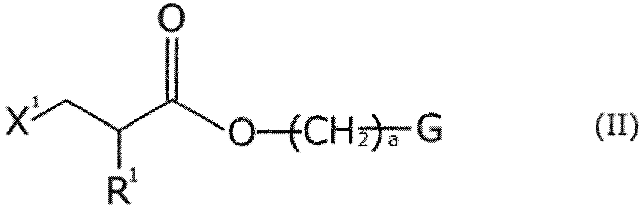
where X¹ represents a halogen atom, G represents an organic group, and R¹ and a have the same meaning as in formula (I), to a dehydrohalogenation reaction in an organic solvent in the presence of an aqueous layer containing a basic compound.

2. The method according to claim 1, wherein
the halogenated compound indicated by formula (II) is a halogenated compound indicated by formula (III), shown below, 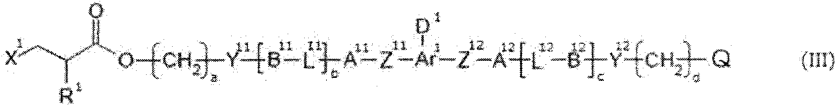 where, in formula (III):
Q indicates a group represented by formula (III-1), shown below, 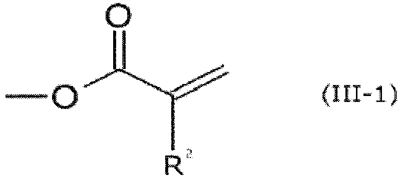 where R² has the same meaning as in formula (I), or represented by formula (III-2), shown below, 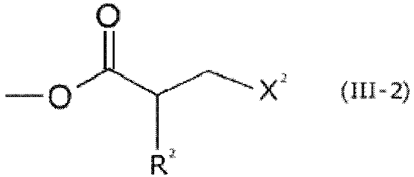 where X² represents a halogen atom and R² has the same meaning as in formula (I);
X¹ has the same meaning as in formula (II); and
Ar¹, D¹, Z¹¹, Z¹², A¹¹, A¹², B¹¹, B¹², Y¹¹, Y¹², L¹¹, L¹², R¹, a, b, c, and d have the same meaning as in formula (I).

3. The method according to claim 2, wherein
X¹ and X² are each a chlorine atom.

4. The method according to claim 1, wherein
the halogenated compound indicated by formula (II) is a halogenated compound indicated by formula (IV), shown below, 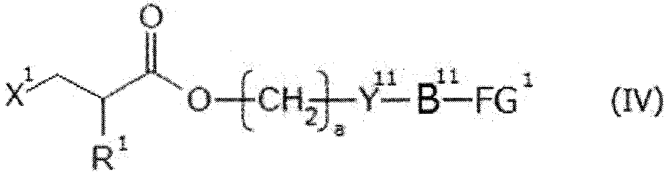 where, in formula (IV):
FG¹ represents a hydroxy group, a carboxyl group, or an amino group;
R¹, Y¹¹, B¹¹, and a have the same meaning as in formula (I); and
X¹ has the same meaning as in formula (II).

5. The method according to claim 4, wherein
X¹ is a chlorine atom.

6. The method according to claim 4 or 5, wherein
FG¹ is a hydroxy group.

7. The method according to any one of claims 4 to 6, wherein
the composition is a mixture containing the halogenated compound indicated by formula (IV) and a compound indicated by formula (V), shown below, 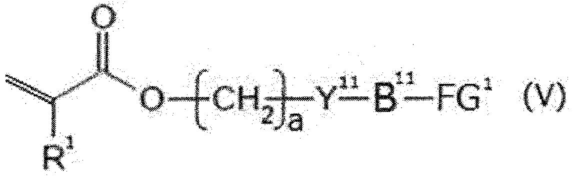 where R¹, Y¹¹, B¹¹, FG¹, and a have the same meaning as in formula (IV).

8. The method according to claim 7, wherein
the halogenated compound indicated by formula (IV) constitutes a proportion of at least 0.01 mass% and not more than 5 mass% among a total of the halogenated compound indicated by formula (IV) and the compound indicated by formula (V).

9. The method according to claim 1, wherein
the halogenated compound indicated by formula (II) is a halogenated compound indicated by formula (VI), shown below, 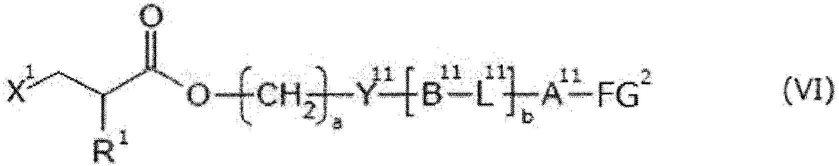 where, in formula (VI):
FG² represents a hydroxy group, a carboxyl group, or an amino group;
R¹, Y¹¹, B¹¹, L¹¹, A¹¹, a, and b have the same meaning as in formula (1); and
X¹ has the same meaning as in formula (II).

10. The method according to claim 9, wherein
X¹ is a chlorine atom.

11. The method according to claim 9 or 10, wherein
FG² is a carboxyl group, and
b is 1.

12. The method according to any one of claims 9 to 11, wherein
the composition is a mixture containing the halogenated compound indicated by formula (VI) and a compound indicated by formula (VII), shown below, 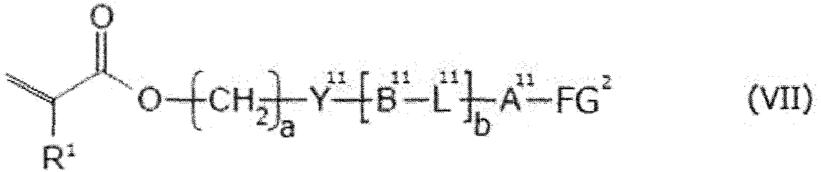 where R¹, Y¹¹, B¹¹, L¹¹, A¹¹, FG², a, and b have the same meaning as in formula (VI).

13. The method according to claim 12, wherein
the halogenated compound indicated by formula (VI) constitutes a proportion of at least 0.01 mass% and not more than 5 mass% among a total of the halogenated compound indicated by formula (VI) and the compound indicated by formula (VII).

14. The method according to any one of claims 1 to 13, wherein Ar¹-D¹ is a divalent group represented by formula (VIII), shown below, 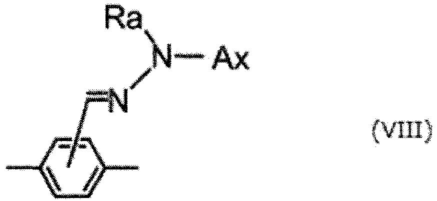 where, in formula (VIII):
Ax represents an organic group having a carbon number of 2 to 20 and including at least one aromatic ring selected from the group consisting of an aromatic hydrocarbon ring and an aromatic heterocyclic ring; and
Ra represents a hydrogen atom or an optionally substituted organic group having a carbon number of 1 to 20.

15. The method according to claim 14, wherein
Ax is a group represented by formula (IX), shown below, 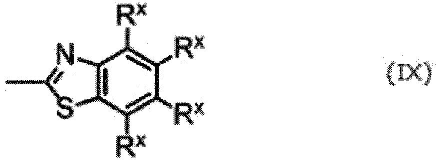 where R^{X} represents a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, or -C(=O)-O-R^{b}, where R^{b} represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 12, each R^{X} may be the same or different, and one or more ring constituent C-R^{X} may be replaced by a nitrogen atom.

16. A halogenated compound indicated by formula (IV), shown below, 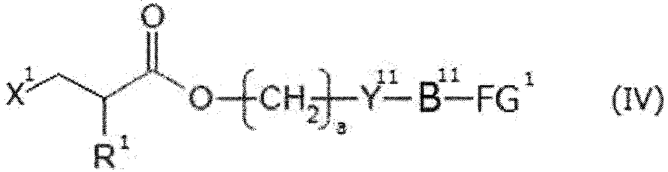 where, in formula (IV):
X¹ represents a halogen atom;
R¹ represents a hydrogen atom or a methyl group;
Y¹¹ represents a single bond, -O-, -CO-, -CO-O-, -O-CO-, -NR¹¹-CO-, -CO-NR¹²-, -O-CO-O-, -NR¹³-CO-O-, -O-CO-NR¹⁴-, or -NR¹⁵-CO-NR¹⁶-, where R¹¹ to R¹⁶ each represent, independently of one another, a hydrogen atom or an alkyl group having a carbon number of 1 to 6;
B¹¹ represents an optionally substituted alicyclic group or an optionally substituted aromatic group;
FG¹ represents a hydroxy group, a carboxyl group, or an amino group; and
a represents an integer of 1 to 20.

17. The halogenated compound according to claim 16, wherein
X¹ is a chlorine atom.

18. The halogenated compound according to claim 16 or 17, wherein
FG¹ is a hydroxy group.

19. A mixture comprising:
the halogenated compound according to any one of claims 16 to 18; and
a compound indicated by formula (V), shown below, 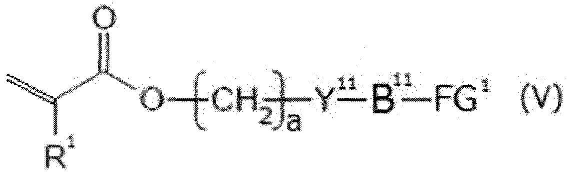 where R¹, Y¹¹, B¹¹, FG¹, and a have the same meaning as in formula (IV).

20. The mixture according to claim 19, wherein
the halogenated compound indicated by formula (IV) constitutes a proportion of at least 0.01 mass% and not more than 5 mass% among a total of the halogenated compound indicated by formula (IV) and the compound indicated by formula (V).

21. A halogenated compound indicated by formula (VI), shown below, 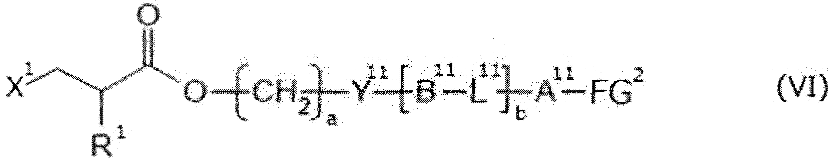 where, in formula (VI):
X¹ represents a halogen atom;
R¹ represents a hydrogen atom or a methyl group;
Y¹¹ and L¹¹ each represent, independently of one another, a single bond, -O-, -CO-, -CO-O-, -O-CO-, -NR¹¹-CO-, -CO-NR¹²-, -O-CO-O-, -NR¹³⁻CO-O-, -O-CO-NR¹⁴-, or -NR¹⁵-CO-NR¹⁶-, where R¹¹ to R¹⁶ each represent, independently of one another, a hydrogen atom or an alkyl group having a carbon number of 1 to 6;
A¹¹ and B¹¹ each represent, independently of one another, an optionally substituted alicyclic group or an optionally substituted aromatic group;
FG² represents a hydroxy group, a carboxyl group, or an amino group;
a represents an integer of 1 to 20; and
b represents 0 or 1.

22. The halogenated compound according to claim 21, wherein
X¹ is a chlorine atom.

23. The halogenated compound according to claim 21 or 22, wherein FG² is a carboxyl group, and
b is 1.

24. A mixture comprising:
the halogenated compound according to any one of claims 21 to 23; and
a compound indicated by formula (VII), shown below, 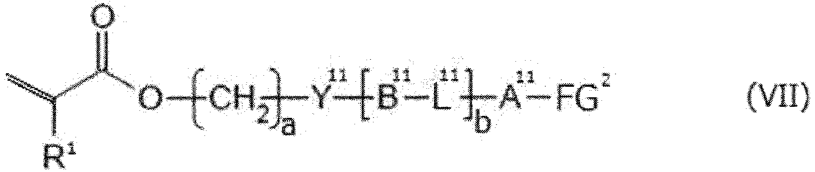 where R¹, Y¹¹, B¹¹, L¹¹, A¹¹, FG², a, and b have the same meaning as in formula (VI).

25. The mixture according to claim 24, wherein
the halogenated compound indicated by formula (VI) constitutes a proportion of at least 0.01 mass% and not more than 5 mass% among a total of the halogenated compound indicated by formula (VI) and the compound indicated by formula (VII).

26. A halogenated compound indicated by formula (III), shown below, 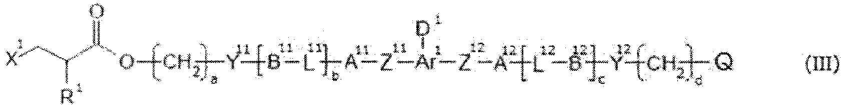 where, in formula (III):
Q indicates a group represented by formula (III-1), shown below, 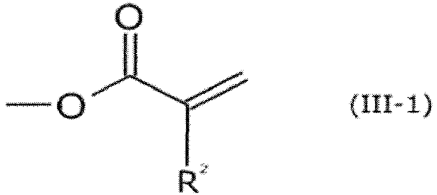 where R² represents a hydrogen atom or a methyl group, or represented by formula (III-2), shown below, 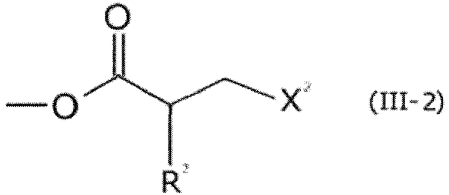 where X² represents a halogen atom and R² represents a hydrogen atom or a methyl group;
X¹ represents a halogen atom;
Ar¹ represents a divalent aromatic hydrocarbon cyclic group having D¹ as a substituent or a divalent aromatic heterocyclic group having D¹ as a substituent;
D¹ represents an organic group having a carbon number of 1 to 20 and including at least one aromatic ring selected from the group consisting of an aromatic hydrocarbon ring and an aromatic heterocyclic ring;
Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
A¹¹, A¹², B¹¹, and B¹² each represent, independently of one another, an optionally substituted alicyclic group or an optionally substituted aromatic group;
Y¹¹, Y¹², L¹¹, and L¹² each represent, independently of one another, a single bond, -O-, -CO-, -CO-O-, -O-CO-, -NR²¹-CO- -CO-NR²²-, -O-CO-O-, -NR²³-CO-O- -O-CO-NR²⁴-, or -NR²⁵-CO-NR²⁶-, where R²¹ to R²⁶ each represent, independently of one another, a hydrogen atom or an alkyl group having a carbon number of 1 to 6;
R¹ represents a hydrogen atom or a methyl group;
a and d each represent, independently of one another, an integer of 1 to 20; and
b and c are each, independently of one another, 0 or 1.

27. A mixture comprising:
the halogenated compound according to claim 26; and
a polymerizable compound indicated by formula (I), shown below, 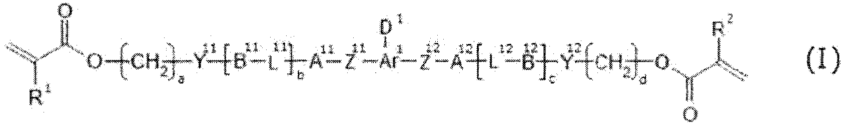 where, in formula (I):
Ar¹ represents a divalent aromatic hydrocarbon cyclic group having D¹ as a substituent or a divalent aromatic heterocyclic group having D¹ as a substituent;
D¹ represents an organic group having a carbon number of 1 to 20 and including at least one aromatic ring selected from the group consisting of an aromatic hydrocarbon ring and an aromatic heterocyclic ring;
Z¹¹ and Z¹² each represent, independently of one another, -CO-O-, -O-CO-, -NR¹¹-CO-, or -CO-NR¹²-, where R¹¹ and R¹² each represent, independently of one another, a hydrogen atom or an alkyl group having a carbon number of 1 to 6;
A¹¹, A¹², B¹¹, and B¹² each represent, independently of one another, an optionally substituted alicyclic group or an optionally substituted aromatic group;
Y¹¹, Y¹², L¹¹, and L¹² each represent, independently of one another, a single bond, -O-, -CO-, -CO-O-, -O-CO-, -NR²¹-CO-, -CO-NR²²-, -O-CO-O-, -NR²³-CO-O-, -O-CO-NR²⁴-, or -NR²⁵-CO-NR²⁶-, where R²¹ to R²⁶ each represent, independently of one another, a hydrogen atom or an alkyl group having a carbon number of 1 to 6;
R¹ and R² each represent, independently of one another, a hydrogen atom or a methyl group;
a and d each represent, independently of one another, an integer of 1 to 20; and
b and c are each, independently of one another, 0 or 1.
